# EUROPEAN PATENT APPLICATION

(11) **EP 2 060 563 A1**
(43) Date of publication of application: **20.05.2009**
(21) Application number: 07806967.1
(22) Date of filing: 07.09.2007
(51) Int. Cl.: C07D 207/14, A61K 31/4545, A61K 31/4725, A61K 31/496, A61K 31/5377, A61K 31/541, A61K 31/55, A61K 31/554, A61P 11/02, A61P 11/06, A61P 17/04, A61P 27/14, A61P 37/08, C07D 211/58, C07D 211/76, C07D 401/06, C07D 401/12, C07D 405/06, C07D 405/12, C07D 409/12, C07D 413/12

(54) **CYCLIC AMINOALKYLCARBOXAMIDE DERIVATIVE**

(30) Priority: 08.09.2006 JP 2006244302
(71) Applicant: Dainippon Sumitomo Pharma Co., Ltd., Osaka 541-8524 (JP)
(72) Inventor: OZAWA, Michinori, Osaka 564-0053 (JP); SHIRO, Tomoya, Osaka 564-0053 (JP); SUMIYOSHI, Takaaki, Osaka 564-0053 (JP); ITOH, Mari, Osaka 554-0022 (JP)
(74) Representative: Duckworth, Timothy John
(86) International application number: PCT/JP2007/067535
(87) International publication number: WO 2008/029924

(57) **Abstract**

The present invention relates to a compound represented by the following formula (I), which is useful as an antiallergic agent and/or an anti-inflammatory agent, or a physiologically acceptable salt thereof and the like: 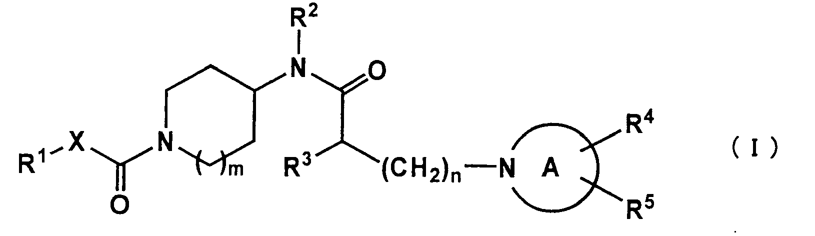 wherein R¹ and R² are the same or different and each is an optionally substituted aryl group and the like, R³ is a hydrogen atom, a C₁₋₆ alkyl group and the like, R⁴ and R⁵ are the same or different and each is a hydrogen atom, a halogen atom, a hydroxy group, a C₁₋₆ alkyl group and the like, X is a single bond or -C(R⁶)(R⁷)-, R⁶ and R⁷ are the same or different and each is a hydrogen atom, a halogen atom, an optionally substituted C₁₋₆ alkyl group and the like, or R⁶ and R⁷ optionally form, together with the carbon atom bonded thereto, an optionally substituted C₃₋₈ cycloalkyl group and the like, ring group A is an azetidin-1-yl group and the like, m is 0, 1 or 2, and n is 0, 1 or 2.

## Description

### Technical Field

The present invention relates to a novel cyclic aminoalkylcarboxamide derivative having a superior antiallergic action, a physiologically acceptable salt thereof and a pharmaceutical composition thereof.

### Background Art

In recent years, allergic diseases such as bronchial asthma, allergic rhinitis, urticaria, atopic dermatitis and contact dermatitis have been increasing due to air pollution, change of house structure (closed state, air conditioning, etc.) and the like. Currently, steroids are mainly used for the treatment of such allergic diseases; however, steroids often cause severe side effects. There are several kinds of pharmaceutical agents with an immunosuppressive action, which are also considered to be effective in recent years for the prophylaxis or treatment of allergic diseases. However, they are also feared to cause severe side effects and use thereof is limited. Although existing antihistamines and anti-inflammatory agents are also used for the treatment of allergic diseases for a palliative therapy, these pharmaceutical agents do not show a sufficient treatment effect particularly for atopic dermatitis. Therefore, a new non-steroidal antiallergic agent effective for the prophylaxis and/or treatment of allergic disease by oral administration is desired.

Patent document 1 discloses a protein inhibitor represented by the following formula. It describes that the compound is used for the treatment of osteoporosis, periodontal disease, osteoarthritis and the like. The compound has an NR¹R² group for Y, but a compound like the compound of the present invention, wherein R¹ and R² form, together with the nitrogen atom bonded thereto, a nitrogen atom-containing hetero ring, is not disclosed. Hence, the compound has a chemical structure clearly different from the compound of the present invention.

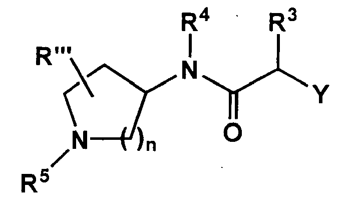

wherein Y is Ar or NR¹R²;
R¹ is R", R"C(O), R"C(S), R"SO₂, R"OC(O), R"R'NC(O) or
R"R'NC(S);
R² is H, C₁₋₆ alkyl, C₂₋₆ alkenyl, Ar-C₀₋₆ alkyl or Het-C₀₋₆ alkyl;
R³ is H, C₂₋₆ alkenyl, C₂₋₆ alkynyl, Het, Ar and the like;
R⁴ is Ar-C₀₋₆ alkyl, Het-C₀₋₆ alkyl and the like;
R⁵ is Ar-C(O)-, Het-C(O)- and the like;
each R' is independently C₁₋₆ alkyl, C₂₋₆ alkenyl, Ar-C₀₋₆ alkyl or Het-C₀₋₆ alkyl;
each R" is independently C₁₋₆ alkyl, C₃₋₆ cycloalkyl-C₀₋₆ alkyl, Ar-C₀₋₆ alkyl or Het-C₀₋₆ alkyl;
R'" is H, C₁₋₆ alkyl and the like; and
n is 1, 2 or 3.

Patent document 2 discloses a PGD2 receptor antagonist represented by the following formula. It describes that the compound is used for the treatment of inflammatory diseases (allergic rhinitis, allergic asthma, atopic dermatitis, chronic obliterative pulmonary diseases, rheumatoid arthritis, osteoarthritis, inflammatory bowel disease or skin disorder). The compound has a piperidine ring. However, the piperidine ring is always fused with other monocyclic aromatic ring to form a fused bicyclic heterocycle. Thus, the compound is clearly different from the compound of the present invention in the chemical structure.

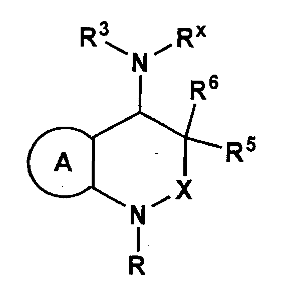

wherein ring A is an optionally substituted monocyclic aromatic ring;
R is -X₁-R¹;
R^{x} is -X₂-R⁴;
R³ is an optionally substituted cyclic aliphatic group, an aromatic group and the like;
X is -C(R²)₂- and the like;
X₁ and X₂ are each independently a bond, C(O) and the like;
R¹ is H or a cyclic aliphatic group, an aromatic group or a nonaromatic heterocyclic group as necessary;
R² is independently H, -X₄-R⁸ and the like;
R⁴ is H, -X₆-R¹⁰ and the like;
X₄ and X₆ are each independently a straight or branched hydrocarbyl group, and the hydrocarbyl group is optionally substituted by one or more groups selected from the group consisting of halo, -OH, =O and the like;
R⁵ and R⁶ are each independently H or C₁-C₃ alkyl; and
R⁸ and R¹⁰ are each independently H, a cyclic aliphatic group, an aromatic group, a nonaromatic heterocyclic group etc.; and the like.

In addition, patent document 3 discloses a PGD2 receptor antagonist represented by the following formula. It describes that the compound is used for the treatment of inflammatory diseases (allergic rhinitis, rheumatoid arthritis, chronic obliterative pulmonary diseases, atopic dermatitis or allergic asthma). The compound has a piperidine ring. However, the piperidine ring is always fused with other monocyclic aromatic ring to form a fused bicyclic heterocycle. Thus, the compound is clearly different from the compound of the present invention in the chemical structure.

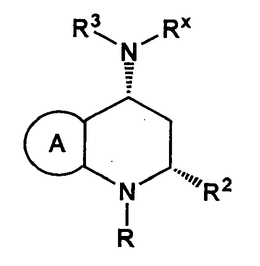

wherein ring A is an optionally substituted monocyclic aromatic ring;
R is -X₁-R¹;
R^{x} is -X₂-R⁴;
X₁ and X₂ are each independently C(O) and the like;
R¹ is a substituted 5- or 6-membered aromatic or heteroaromatic ring group;
R² is a C₁₋₃ alkyl group;
R³ is an optionally substituted monocyclic or bicyclic group, selected from an aromatic ring, a heteroaromatic ring, a nonaromatic carbon ring and a non-aromatic heterocycle; and
R⁴ is an optionally substituted C₁₋₆ alkyl group, etc.; and the like.

Patent document 4 discloses a serotonin and noradrenaline reuptake inhibitor represented by the following formula. It describes that the compound is used for the treatment of a disease relating to the inhibition of both serotonin and noradrenaline (incontinence), and a disease that inhibits reuptake of one of serotonin and noradrenaline (pain, depression etc.). Such uses are completely different from the use of the present invention. Furthermore, although various substituents are recited as the substituents R¹ and R³ of the compound, the document discloses only a compound wherein R¹ is a hydrogen atom in its Examples, and does not disclose a compound wherein R³ is a Het-C₁₋₄ alkyl group, as in the present invention. Thus, the compound is clearly different from the compound of the present invention in the chemical structure.

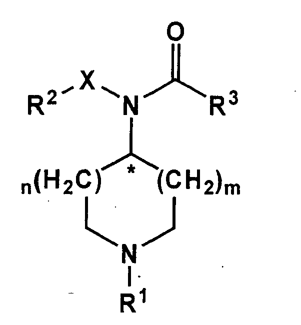

wherein R¹ is -H, -C(A)Y, -C₃₋₈ cycloalkyl, -aryl and the like, where cycloalkyl, aryl and het are substituted by at least any one independent substituent selected from B,
A is S or O,
Y is -aryl, -het and the like,
B is -C₁₋₈ alkyl, -C₁₋₈ alkoxy, -OH, -halo, -CF₃, -OCF₃, C₁₋₄ alkoxy-C₁₋₆ alkyl- and the like,
R² is aryl¹ or het¹, each of which is optionally substituted by at least one independent substituent selected from D, aryl¹ is independently selected from phenyl, naphthyl and the like,
het¹ is a 5- to 10-membered aromatic ring containing at least one N, O or S hetero atom, which optionally contains an aryl group,
D is -C₁₋₈ alkyl, -C₁₋₈ alkoxy, -OH, -halo, -CF₃, -OCF₃, C₁₋₄ alkoxy-C₁₋₆ alkyl-, C₃₋₈ cycloalkyl and the like,
R³ is -het³, het³-C₁₋₄ alkyl- and the like, wherein het³ and the like are optionally substituted by at least one independent substituent selected from G,
het³ is a 4-, 5- or 6-membered nonaromatic hetero ring containing at least one hetero atom selected from N, O and S, which is optionally fused with a 5- or 6-membered carbon ring or another 4-, 5- or 6-membered hetero ring containing at least one hetero atom selected from N, O and S, G is -C₁₋₈ alkyl, -C₁₋₆ alkoxy, -OH, -halo, -CF₃ and the like, wherein the alkyl group may have a substituent, X is a single bond, -C₁₋₈ alkyl and the like, and
when n is 1, m is 0 or 1, and the like.

Non-patent document 1 discloses a compound represented by the following formula. It describes that the compound is useful as analgesic or topical anesthetic. When X is N(CH₃), the compound has a substituent on the piperidine ring and the substituent of the nitrogen atom is a methyl group alone. Thus, the compound is clearly different from the compound of the present invention in the chemical structure.

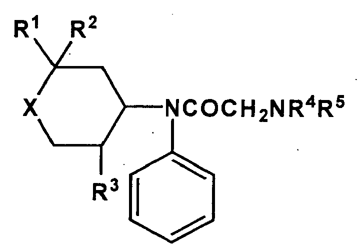

wherein R¹ is a methyl group, R² and R³ are each a hydrogen atom or a methyl group,
NR⁴R⁵ is N(CH₂)₂, N(CH₂CH₃)₂, a pyrrolidinyl group, a piperidinyl group or a morpholinyl group, and
X is an oxygen atom, a sulfur atom or N(CH₃).
patent document 1: JP-A-2001-525809
patent document 2: JP-A-2006-508077
patent document 3: WO2005/100321
patent document 4: WO2006/064351
non-patent document 1: Chemical Abstract 95:24753 [Khimiko-Farmatsevticheskii Zhurnal (1981), 15(3), 43-49]

### Disclosure of the Invention

### Problems to be Solved by the Invention

Since existing antiallergic agents and antihistamine agents show almost no suppressive effect on inflammation in human atopic dermatitis, a novel therapeutic agent for atopic dermatitis other than steroids and immunosuppressive agents causing strong side effects is desired.

### Means of Solving the Problems

The present inventors have conducted intensive studies in an attempt to solve the above-mentioned problems and found a new compound having a non-steroidal chemical structure and a superior antiallergic action, which resulted in the completion of the present invention. The present invention provides a compound represented by the following formula (I) or a physiologically acceptable salt thereof (hereinafter sometimes to be referred to as "the compound of the present invention") and a pharmaceutical composition comprising the compound of the present invention.

(1) A compound represented by the following formula (I):

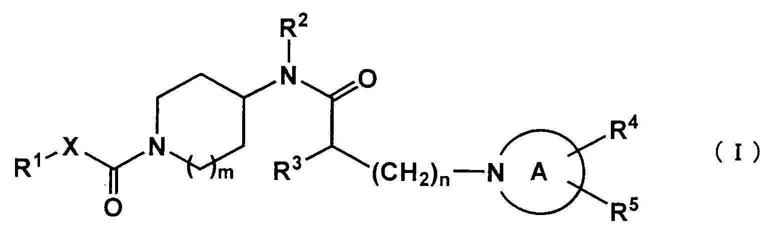

wherein R¹ and R² are the same or different and each is an optionally substituted aryl group, an optionally substituted C₃₋₈ cycloalkyl group, an optionally substituted heterocyclic group or an optionally substituted partially hydrogenated aryl group,
R³ is a hydrogen atom, an optionally substituted C₁₋₆ alkyl group, an optionally substituted aryl group or an optionally substituted benzyl group,
R⁴ and R⁵ are the same or different and each is a hydrogen atom, a halogen atom, a hydroxy group, a carboxyl group, an optionally substituted C₁₋₆ alkyl group, an optionally substituted C₃₋₈ cycloalkyl group, an optionally substituted C₁₋₆ alkoxy group, a C₁₋₆ alkoxycarbonyl group, an optionally substituted amino group, a substituted carbamoyl group, an optionally substituted aryl group, an optionally substituted benzyl group, an optionally substituted benzoyl group, an optionally substituted heterocyclic group or an optionally substituted heterocarbonyl group, or R⁴ and R⁵ in combination may form an oxo group,
X is a single bond or -C(R⁶)(R⁷)-,
wherein R⁶ and R⁷ are the same or different and each is a hydrogen atom, a halogen atom, a hydroxy group, an optionally substituted C₁₋₆ alkyl group or an optionally substituted aryl group, or R⁶ and R⁷ may form, together with the carbon atom bonded thereto, an optionally substituted C₃₋₈ cycloalkane ring, a substituted piperidine ring or a tetrahydro-2H-pyran ring, ring group A is an azetidin-1-yl group, a pyrrolidin-1-yl group, a piperidino group, a piperazin-1-yl group, a morpholino group, a hexahydroazepin-1-yl group, a hexahydrodiazepin-1-yl group, an aziridin-1-yl group, a perhydroisoquinolin-2-yl group, a perhydroquinolin-1-yl group, a 1,2,3,4-tetrahydroisoquinolin-2-yl group, a 1,2,3,4-tetrahydroquinolin-1-yl group, a 2,3-dihydroindol-1-yl group, a perhydroindol-1-yl group, a 1,3-dihydroisoindol-2-yl group, a perhydroisoindol-2-yl group, a pyrazolidin-1-yl group, a tetrahydroimidazol-1-yl group, a hexahydro-1,4-oxaazepin-4-yl-group, an oxazolidin-3-yl group, a thiazolidin-3-yl group, a thiomorpholin-4-yl group, a hexahydropyridazin-1-yl group, a hexahydropyrimidin-1-yl group, a tetrazolyl group, a 2-pyrrolin-1-yl group or a 3-pyrrolin-1-yl group,
m is 0, 1 or 2, and
n is 0, 1 or 2
or a physiologically acceptable salt thereof.
(2) The compound of the above-mentioned (1), wherein the ring group A is an azetidin-1-yl group, a pyrrolidin-1-yl group, a piperidino group, a morpholino group, a piperazin-1-yl group or a perhydroazepin-1-yl group, or a physiologically acceptable salt thereof.
(3) The compound of the above-mentioned (1) or (2), wherein R⁴ and R⁵ are the same or different and each is a hydrogen atom, a halogen atom, a hydroxy group, a C₁₋₆ alkyl group, a C₁₋₆ alkoxy group, a C₁₋₆ dialkylamino group, a C₁₋₆ dialkylcarbamoyl group, a pyrrolidinylcarbonyl group or a C₁₋₆ alkyloxadiazolyl group, or a physiologically acceptable salt thereof.
(4) The compound of any one of the above-mentioned (1) to (3), wherein m is 1, or a physiologically acceptable salt.
(5) The compound of any one of the above-mentioned (1) to (4), wherein R³ is a hydrogen atom or a C₁₋₆ alkyl group, and n is 0 or 1, or a physiologically acceptable salt thereof.
(6) The compound of any one of the above-mentioned (1) to (4), wherein R³ is a hydrogen atom, and n is 0 or 1, or a physiologically acceptable salt thereof.
(7) The compound of any one of the above-mentioned (1) to (6), wherein R¹ and R² are the same or different and each is an aryl group optionally substituted at substitutable position(s) by 1 to 4 atoms or groups selected from the group consisting of a halogen atom, a C₁₋₆ alkyl group, a C₁₋₆ alkoxy group, a trifluoromethyl group, a trifluoromethoxy group, a cyano group, a nitro group, a phenyl group, a carboxyl group, a C₃₋₈ cycloalkyl group, a C₁₋₃ alkoxy-C₁₋₃ alkyl group, a C₁₋₆ alkoxycarbonyl group, a C₁₋₆ alkylcarbonyl group, a trifluoromethylcarbonyl group, a C₁₋₃ alkylthio group, a C₁₋₃ alkylsulfonyl group, a hydroxy group and an amino group (the amino group is optionally substituted by one or two, the same or different, groups selected from the group consisting of a C₁₋₃ alkyl group, a C₃₋₈ cycloalkyl group and a tetrahydro-2H-pyranyl group); a C₃₋₈ cycloalkyl group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group; a hetero ring group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group; or a partially hydrogenated aryl group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group, or a physiologically acceptable salt thereof.
(8) The compound of any one of the above-mentioned (1) to (6), wherein R¹ and R² are the same or different and each is a phenyl group optionally substituted at substitutable position(s) by 1 to 4 atoms or groups selected from the group consisting of a halogen atom, a C₁₋₆ alkyl group, a C₁₋₆ alkoxy group, a trifluoromethyl group, a trifluoromethoxy group, a cyano group, a nitro group, a carboxyl group, a hydroxy group and an amino group (the amino group is optionally substituted by one or the same or different two C₁₋₃ alkyl groups); a cyclohexyl group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group; a pyridyl group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group; or a benzofuranyl group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group, or a physiologically acceptable salt thereof.
(9) The compound of any one of the above-mentioned (1) to (8), wherein X is a single bond or -C(R⁶)(R⁷)- wherein R⁶ and R⁷ are the same substituent and is a C₁₋₆ alkyl group, or R⁶ and R⁷ form, together with the carbon atom bonded thereto, a C₃₋₈ cycloalkane ring; a piperidine ring substituted by a C₁₋₆ alkyl group, a phenyl group (the phenyl group is optionally substituted by 1 to 4 atoms or groups selected from the group consisting of a halogen atom, a C₁₋₆ alkyl group, a C₁₋₆ alkoxy group, a trifluoromethyl group and a hydroxy group), a benzyl group (the benzyl group is optionally substituted by 1 to 4 atoms or groups selected from the group consisting of a halogen atom, a C₁₋₆ alkyl group, a C₁₋₆ alkoxy group, a trifluoromethyl group and a hydroxy group) or a C₁₋₃ alkylcarbonyl group; or a tetrahydro-2H-pyran ring, or a physiologically acceptable salt thereof.
(10) The compound of any one of the above-mentioned (1) to (8), wherein X is a single bond, or a physiologically acceptable salt thereof.
(11) The compound of the above-mentioned (1), which is selected from the group consisting of
   N-[1-(4-chloro-2,5-difluorobenzoyl)piperidin-4-yl]-N-phenyl-2-(piperidin-1-yl)acetamide (compound of Example 8),
   N-phenyl-2-(piperidin-1-yl)-N-[1-(4-trifluoromethylbenzoyl)piperidin-4-yl]acetamide (compound of Example 9),
   N-[1-(3-fluoro-4-trifluoromethylbenzoyl)piperidin-4-yl]-N-phenyl-2-(piperidin-1-yl)acetamide (compound of Example 12),
   N-phenyl-2-(piperidin-1-yl)-N-[1-(4-trifluoromethoxybenzoyl)piperidin-4-yl]acetamide (the compound of Example 13),
   2-(morpholin-4-yl)-N-phenyl-N-[1-(4-trifluoromethylbenzoyl)piperidin-4-yl]acetamide (compound of Example 29),
   N-[1-(3-fluoro-4-trifluoromethylbenzoyl)piperidin-4-yl]-2-(morpholin-4-yl)-N-phenylacetamide (compound of Example 32),
   N-[1-(2,3-difluoro-4-trifluoromethylbenzoyl)piperidin-4-yl]-2-(morpholin-4-yl)-N-phenylacetamide (compound of Example 34),
   N-(4-methoxyphenyl)-2-(piperidin-1-yl)-N-[1-(4-trifluoromethylbenzoyl)piperidin-4-yl]acetamide (compound of Example 55),
   N-[1-(4-chlorobenzoyl)piperidin-4-yl]-N-cyclohexyl-2-(morpholin-4-yl)acetamide (compound of Example 65),
   N-[1-(trans-4-trifluoromethylcyclohexylcarbonyl)piperidin-4-yl]-2-(morpholin-4-yl)-N-phenylacetamide (compound of Example 86),
   N-phenyl-2-(pyrrolidin-1-yl)-N-[1-(4-trifluoromethylbenzoyl)piperidin-4-yl]acetamide (compound of Example 103),
   N-[1-(3,4-dichlorobenzoyl)piperidin-4-yl]-N-phenyl-2-(pyrrolidin-1-yl)acetamide (compound of Example 104),
   3-(morpholin-4-yl)-N-phenyl-N-[1-(4-trifluoromethylbenzoyl)piperidin-4-yl]propionamide (compound of Example 154),
   N-[1-(4-chlorobenzoyl)piperidin-4-yl]-N-phenyl-3-(pyrrolidin-1-yl)propionamide (compound of Example 158),
   N-[1-[2-(4-chlorophenyl)-2-methylpropionyl]piperidin-4-yl]-N-phenyl-2-(piperidin-1-yl)acetamide (compound of Example 190), N-[1-[1-(4-chlorophenyl)cyclopentane-1-carbonyl]piperidin-4-yl]-N-phenyl-2-(piperidin-1-yl)acetamide (compound of Example 198),
   N-phenyl-2-(piperidin-1-yl)-N-[1-[1-(3-trifluoromethylphenyl)cyclopentane-1-carbonyl]piperidin-4-yl]acetamide (compound of Example 199),
   N-[1-[1-(2-fluorophenyl)cyclopentane-1-carbonyl]piperidin-4-yl]-N-phenyl-2-(piperidin-1-yl)acetamide (compound of Example 202),
   2-(3-dimethylaminopyrrolidin-1-yl)-N-phenyl-N-[1-(4-trifluoromethylbenzoyl)piperidin-4-yl]acetamide (compound of Example 131),
   N-[1-(2,4-ditrifluoromethylbenzoyl)piperidin-4-yl]-2-(morpholin-4-yl)-N-phenylacetamide (compound of Example 50), N-cyclohexyl-2-(pyrrolidin-1-yl)-N-[1-(4-trifluoromethylbenzoyl)piperidin-4-yl]acetamide (compound of Example 77),
   2-(3-hydroxypyrrolidin-1-yl)-N-phenyl-N-[1-(4-trifluoromethylbenzoyl)piperidin-4-yl]acetamide (compound of Example 135),
   2-[4-(5-methyloxodiazol-3-yl)]piperidin-1-yl]-N-phenyl-N-[1-(4-trifluoromethylbenzoyl)piperidin-4-yl]acetamide (compound of Example 138),
   3-(4-methylpiperazin-1-yl)-N-phenyl-N-[1-(4-trifluoromethylbenzoyl)piperidin-4-yl]propionamide (compound of Example 159),
   3-(4-dimethylaminopiperidine-1-yl)-N-phenyl-N-[1-(4-trifluoromethylbenzoyl)piperidin-4-yl]propionamide (compound of Example 161),
   3-(4-methylpiperazin-1-yl)-N-phenyl-N-[1-[(benzofuran-2-yl)carbonyl]piperidin-4-yl]propionamide (compound of Example 174),
   N-(3-carboxylphenyl)-2-(pyrrolidin-1-yl)-N-[1-(4-trifluoromethylbenzoyl)piperidin-4-yl]acetamide (compound of Example 227), and
   N-[1-(3-fluoro-4-trifluoromethylbenzoyl)piperidin-4-yl]-3-(4-methylpiperazin-1-yl)-N-phenylpropionamide (compound of Example 176), or a physiologically acceptable salt thereof.
(12) A pharmaceutical composition comprising the compound of any one of the above-mentioned (1) to (11) or a physiologically acceptable salt thereof as an active ingredient.
(13) The pharmaceutical composition of the above-mentioned
(12), which is used for the prophylaxis and/or treatment of an allergic disease.
(14) The pharmaceutical composition of the above-mentioned (13), wherein the allergic disease is atopic dermatitis or contact dermatitis.
(15) Use of the compound of any one of the above-mentioned (1) to (11) or a physiologically acceptable salt thereof for the production of a pharmaceutical composition to be used for the prophylaxis and/or treatment of an allergic disease.
(16) The use of the above-mentioned (15), wherein the allergic disease is atopic dermatitis or contact dermatitis.
(17) A method of preventing and/or treating an allergic disease in a mammal, comprising administering an effective amount of the compound of any one of the above-mentioned (1) to (11) or a physiologically acceptable salt thereof to the mammal.
(18) The method of the above-mentioned (17), wherein the allergic disease is atopic dermatitis or contact dermatitis.

The physiologically acceptable salt of the compound represented by the formula (I) means a physiologically acceptable acid addition salt. Specific examples thereof include inorganic acid salts such as hydrochloride, hydrobromide, hydroiodide, sulfate, phosphate and the like, organic acid salts such as oxalate, maleate, fumarate, malonate, lactate, malate, citrate, tartrate, benzoate, methanesulfonate, p-toluenesulfonate, gluconate and the like.

The compound of the present invention may be a crystal or non-crystal, and may be present in the form of a hydrate and/or a solvate. Therefore, such hydrates and/or solvates are also encompassed in the compound of the present invention. Stoichiometric amount of hydrate and compounds containing various amounts of water, which are obtained by a method such as freeze-drying, are also within the scope of the present invention.

In addition, some of the compounds represented by the formula (I) contain an asymmetric carbon atom or show a geometric isomerism. Such stereoisomers, a mixture thereof and a racemate thereof are also encompassed in the present invention. A compound of the formula (I) which is substituted by an isotope is also encompassed in the compound of the present invention.

### Effect of the Invention

Since the compound of the present invention shows a suppressive action equal to or greater than that of steroids on skin inflammatory response in atopic dermatitis animal models, it is useful as a novel therapeutic agent for non-steroidal atopic dermatitis.

### Best Mode for Carrying out the Invention

The terms used in the present specification are explained in the following.

The "C₁₋₆ alkyl group" means a linear or branched alkyl group having 1 to 6 carbon atoms. Specific examples thereof include a methyl group, an ethyl group, a propyl group, an isopropyl group, a butyl group, an isobutyl group, a sec-butyl group, a tert-butyl group, a pentyl group, an isopentyl group, a hexyl group and the like. The "C₁₋₃" means that the number of carbon atoms is 1 to 3.

The "optionally substituted C₁₋₆ alkyl group" means, a C₁₋₆ alkyl group optionally substituted at each substitutable position by 1 to 4 atoms or groups selected from the group consisting of a halogen atom, a hydroxy group, a C₁₋₃ alkoxy group and a trifluoromethyl group. Specific preferable examples of the substituted C₁₋₆ alkyl group include a fluoromethyl group, a trifluoromethyl group, a methoxymethyl group, a 1-chloro-2-trifluoroethyl group, a 2-fluoroethyl group, a 2-chloroethyl group, a trifluoroethyl group, a 2-hydroxyethyl group, a hydroxymethyl group, a 1-hydroxy-1-methylethyl group, a 2-hydroxy-1-methylethyl group, a 3-hydroxypropyl group, a methoxyethyl group, a methoxybutyl group and the like.

The "C₁₋₆ alkoxy group" may be linear or branched. Specific examples thereof include methoxy, ethoxy, propoxy, isopropoxy, butoxy, isobutoxy, sec-butoxy, tert-butoxy and the like.

The "optionally substituted C₁₋₆ alkoxy group" means a C₁₋₆ alkoxy group optionally substituted at each substitutable position by 1 to 4 atoms or groups selected from the group consisting of a halogen atom, a hydroxy group and a trifluoromethyl group. Specific preferable examples of the substituted C₁₋₆ alkoxy group include a 2-fluoroethoxy group, a 2-chloroethoxy group, a trifluoromethoxy group, a trifluoroethoxy group, a 3-hydroxypropoxy group and the like.

The "C₁₋₆ alkoxycarbonyl group" may be linear or branched. Specific examples thereof include a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group and the like.

The "optionally substituted amino group" means an amino group optionally substituted by one or the same or different two C₁₋₆ alkyl groups or C₁₋₆ alkoxy groups. Specific examples of the substituted amino group include an ethylamino group, a dimethylamino group, a diethylamino group, an N-methyl-N-ethylamino group, an N-methyl-N-methoxyamino group, a diisobutylamino group, a dihexylamino group, a bis(methoxyethyl)amino group and the like.

The "substituted carbamoyl group" means a carbamoyl group substituted by one or the same or different two C₁₋₆ alkyl groups. Specific examples thereof include a methylaminocarbonyl group, an ethylaminocarbonyl group, a t-butylaminocarbonyl group, a dimethylaminocarbonyl group, a diethylaminocarbonyl group, an ethylmethylaminocarbonyl group and the like.

The "C₃₋₈ cycloalkyl group" is a monovalent cycloalkane group having 3 to 8 carbon atoms. Specific examples thereof include a cyclopropyl group, a cyclobutyl group, a cyclopentyl group, a cyclohexyl group, a cycloheptyl group and the like.

The "optionally substituted C₃₋₈ cycloalkyl group" means a C₃₋₈ cycloalkyl group optionally substituted at each substitutable position by 1 to 4 atoms or groups selected from the group consisting of a halogen atom; a hydroxy group; a C₁₋₆ alkyl group; a C₁₋₆ alkoxy group; a trifluoromethyl group; a trifluoromethoxy group; and an aryl group [the aryl group may be substituted by 1 to 4 atoms or groups selected from the group consisting of a halogen atom, a C₁₋₃ alkyl group, a C₁₋₃ alkoxy group, a trifluoromethyl group, a trifluoromethoxy group, a cyano group, a nitro group, a C₁₋₃ alkoxy-C₁₋₃ alkyl group, a C₁₋₃ alkoxycarbonyl group, a C₁₋₃ alkylcarbonyl group, a trifluoromethylcarbonyl group, a C₁₋₃ alkylthio group, a C₁₋₃ alkylsulfonyl group, a hydroxy group and an amino group (the amino group may be substituted by one or the same or different two C₁₋₃ alkyl groups)]. Specific preferable examples of the substituted C₃₋₈ cycloalkyl group include a 4-methylcyclohexyl group, a 4-tert-butylcyclohexyl group, a 4,4-dimethylcyclohexyl group, a 3,3,5,5-tetramethylcyclohexyl group, a 4-fluorocyclohexyl group, a 4-hydroxycyclohexyl group, a 4-trifluoromethylcyclohexyl group, a 4-methoxycyclohexyl group, a 4-phenylcyclohexyl group, a 4-(4-chlorophenyl)cyclohexyl group and the like.

The "C₃₋₈ cycloalkane ring" means a cycloalkane ring having 3 to 8 carbon atoms. Specific examples thereof include a cyclopropane ring, a cyclobutane ring, a cyclopentane ring, a cyclohexane ring, a cycloheptane ring and the like.

The "optionally substituted C₃₋₈ cycloalkane ring" means a C₃₋₈ cycloalkyl group optionally substituted at each substitutable position by 1 to 4 atoms or groups selected from the group consisting of a halogen atom; a hydroxy group; a C₁₋₆ alkyl group; a C₁₋₆ alkoxy group; a trifluoromethyl group; a trifluoromethoxy group; and an aryl group [the aryl group may be substituted by 1 to 4 atoms or groups selected from the group consisting of a halogen atom, a C₁₋₃ alkyl group, a C₁₋₃ alkoxy group, a trifluoromethyl group, a trifluoromethoxy group, a cyano group, a nitro group, a C₁₋₃ alkoxy-C₁₋₃ alkyl group, a C₁₋₃ alkoxycarbonyl group, a C₁₋₃ alkylcarbonyl group, a trifluoromethylcarbonyl group, a C₁₋₃ alkylthio group, a C₁₋₃ alkylsulfonyl group, a hydroxy group and an amino group (the amino group may be substituted by one or the same or different two C₁₋₃ alkyl groups)]. Specific preferable examples of the substituted C₃₋₈ cycloalkane ring include a 4-methylcyclohexane ring, a 4-tert-butylcyclohexane ring, a 4,4-dimethylcyclohexane ring, a 3,3,5,5-tetramethylcyclohexane ring, a 4-fluorocyclohexane ring, a 4-hydroxycyclohexane ring, a 4-trifluoromethylcyclohexane ring, a 4-methoxycyclohexane ring, a 4-phenylcyclohexane ring, a 4-(4-chlorophenyl)cyclohexane ring and the like.

The "aryl group" means a fused polycyclic aromatic hydrocarbon group comprising a phenyl group and a benzene ring having a carbon number of 6 to 14. Specific examples thereof include a phenyl group, a naphthyl group, an indenyl group, an indanyl group, a fluorenyl group, an anthracenyl group, a phenanthrenyl group and the like. Of these, preferred are a phenyl group and a naphthyl group.

The "optionally substituted aryl group" means an aryl group optionally substituted at each substitutable position by 1 to 4 atoms or groups selected from the group consisting of a halogen atom; a C₁₋₆ alkyl group (the C₁₋₆ alkyl group may be substituted by 1 to 4 atoms or groups selected from the group consisting of a halogen atom, a hydroxy group, a C₁₋₃ alkoxy group and a trifluoromethyl group); a C₁₋₆ alkoxy group; a trifluoromethyl group; a trifluoromethoxy group; a cyano group; a nitro group; a carboxyl group; a phenyl group [the phenyl group may be substituted by 1 to 4 atoms or groups selected from the group consisting of a halogen atom, a C₁₋₆ alkyl group, a C₁₋₃ alkoxy group, a trifluoromethyl group, a trifluoromethoxy group, a cyano group, a nitro group, a C₁₋₃ alkoxy-C₁₋₃ alkyl group, a C₁₋₃ alkoxycarbonyl group, a C₁₋₃ alkylcarbonyl group, a C₁₋₃ alkylthio group, a C₁₋₃ alkylsulfonyl group, a hydroxy group and an amino group (the amino group may be substituted by one or the same or different two C₁₋₃ alkyl groups)]; a C₃₋₈ cycloalkyl group (the C₃₋₈ cycloalkyl group may be substituted by 1 to 4 atoms or groups selected from the group consisting of a halogen atom, a hydroxy group, a C₁₋₃ alkyl group, a C₁₋₃ alkoxy group, a trifluoromethyl group and a trifluoromethoxy group); a C₁₋₆ alkoxycarbonyl group; a C₁₋₆ alkylcarbonyl group; a trifluoromethylcarbonyl group; a C₁₋₃ alkylthio group; a C₁₋₃ alkylsulfonyl group; a hydroxy group; and an amino group [the amino group may be substituted by one or two, the same or different, groups selected from the group consisting of a C₁₋₃ alkyl group, a C₃₋₈ cycloalkyl group (the C₃₋₈ cycloalkyl group may be substituted by 1 to 4 atoms or substituents selected from the group consisting of a halogen atom, a hydroxy group, a C₁₋₃ alkyl group, a C₁₋₃ alkoxy group, a trifluoromethyl group and a trifluoromethoxy group) and a tetrahydro-2H-pyranyl group]. Specific preferable examples of the substituted aryl group include a 3-fluorophenyl group, a 4-chlorophenyl group, a 4-bromophenyl group, a 2-methylphenyl group, a 4-(1,1,1,3,3,3-hexafluoro-2-hydroxypropyl)phenyl group, a 2-methoxyphenyl group, a 4-trifluoromethylphenyl group, a 4-trifluoromethoxyphenyl group, a 4-cyanophenyl group, a 4-chloro-3-nitrophenyl group, a 4-phenylphenyl group, a 4-(4-chlorophenyl)phenyl group, a 4-cyclohexylphenyl group, a 3-carboxylphenyl group, a 4-methoxycarbonylphenyl group, a 2-acetylphenyl group, a 4-(2,2,2-trifluoroacetyl)phenyl group, a 4-methylthiophenyl group, a 4-methylsulfonylphenyl group, a 2-hydroxyphenyl group, a 4-dimethylaminophenyl group, a 4-(3,3,5,5-tetramethylcyclohexylamino)phenyl group, a 2-(tetrahydro-2H-pyran-4-ylamino)phenyl group, a 3,4-dichlorophenyl group, a 2,4,6-trichlorophenyl group, a 2-methoxy-4-chlorophenyl group, a 2,5-difluoro-4-chlorophenyl group, a 2-fluoro-4-trifluoromethylphenyl group, a 4-fluorophenyl group, a 3-chloro-4-fluorophenyl group, a 4-methylphenyl group, 2-trifluoromethylphenyl group, a 2-chlorophenyl group, a 3-fluoro-4-chlorophenyl group, a 3-fluoro-4-trifluoromethylphenyl group, a 2,3-difluoro-4-trifluoromethylphenyl group, a 4-methylphenyl group, a 4-methoxymethylphenyl group, a 4-fluoromethylphenyl group, a 4-(1-chloro-2,2,2-trichloroethyl)phenyl group, a 2,4-ditrifluoromethylphenyl group, a 3-chlorophenyl group, a 4-methoxyphenyl group, a 3-dimethylaminophenyl group, a 3-methoxyphenyl group, a 3-trifluoromethylphenyl group, a 2-fluorophenyl group and the like.

The "optionally substituted phenyl group" means a phenyl group optionally substituted at each substitutable position by 1 to 4 atoms or groups selected from the group consisting of a halogen atom; a C₁₋₆ alkyl group (the C₁₋₆ alkyl group may be substituted by 1 to 4 atoms or groups selected from the group consisting of a halogen atom, a hydroxy group, a C₁₋₃ alkoxy group and a trifluoromethyl group); a C₁₋₆ alkoxy group; a trifluoromethyl group; a trifluoromethoxy group; a cyano group; a nitro group; a carboxyl group; a phenyl group [the phenyl group may be substituted by 1 to 4 atoms or groups selected from the group consisting of a halogen atom, a C₁₋₆ alkyl group, a C₁₋₃ alkoxy group, a trifluoromethyl group, a trifluoromethoxy group, a cyano group, a nitro group, a C₁₋₃ alkoxy-C₁₋₃ alkyl group, a C₁₋₃ alkoxycarbonyl group, a C₁₋₃ alkylcarbonyl group, a C₁₋₃ alkylthio group, a C₁₋₃ alkylsulfonyl group, a hydroxy group and an amino group (the amino group may be substituted by one or the same or different two C₁₋₃ alkyl groups)]; a C₃₋₈ cycloalkyl group (the C₃₋₈ cycloalkyl group may be substituted by 1 to 4 atoms or groups selected from the group consisting of a halogen atom, a hydroxy group, a C₁₋₃ alkyl group, a C₁₋₃ alkoxy group, a trifluoromethyl group and a trifluoromethoxy group); a C₁₋₆ alkoxycarbonyl group; a C₁₋₆ alkylcarbonyl group; a trifluoromethylcarbonyl group; a C₁₋₃ alkylthio group; a C₁₋₃ alkylsulfonyl group; a hydroxy group; and an amino group [the amino group may be substituted by one or two, the same or different, groups selected from the group consisting of a C₁₋₃ alkyl group, a C₃₋₈ cycloalkyl group (the C₃₋₈ cycloalkyl group may be substituted by 1 to 4 atoms or groups selected from the group consisting of a halogen atom, a hydroxy group, a C₁₋₃ alkyl group, a C₁₋₃ alkoxy group, a trifluoromethyl group and a trifluoromethoxy group) and a tetrahydro-2H-pyranyl group]. Specific preferable examples of the substituted phenyl group include a 3-fluorophenyl group, a 4-chlorophenyl group, a 4-bromophenyl group, a 2-methylphenyl group, a 4-(1,1,1,3,3,3-hexafluoro-2-hydroxypropyl)phenyl group, a 2-methoxyphenyl group, a 4-trifluoromethylphenyl group, a 4-trifluoromethoxyphenyl group, a 4-cyanophenyl group, a 4-chloro-3-nitrophenyl group, a 4-phenylphenyl group, a 4-(4-chlorophenyl)phenyl group, a 4-cyclohexylphenyl group, a 3-carboxylphenyl group, a 4-methoxycarbonylphenyl group, a 2-acetylphenyl group, a 4-(2,2,2-trifluoroacetyl)phenyl group, a 4-methylthiophenyl group, a 4-methylsulfonylphenyl group, a 2-hydroxyphenyl group, a 4-dimethylaminophenyl group, a 4-(3,3,5,5-tetramethylcyclohexylamino)phenyl group, a 2-(tetrahydro-2H-pyran-4-ylamino)phenyl group, a 3,4-dichlorophenyl group, a 2,4,6-trichlorophenyl group, a 2-methoxy-4-chlorophenyl group, a 2,5-difluoro-4-chlorophenyl group, a 2-fluoro-4-trifluoromethylphenyl group, a 4-fluorophenyl group, a 3-chloro-4-fluorophenyl group, a 4-methylphenyl group, a 2-trifluoromethylphenyl group, a 2-chlorophenyl group, a 3-fluoro-4-chlorophenyl group, a 3-fluoro-4-trifluoromethylphenyl group, a 2,3-difluoro-4-trifluoromethylphenyl group, a 4-methylphenyl group, a 4-methoxymethylphenyl group, a 4-fluoromethylphenyl group, a 4-(1-chloro-2,2,2-trichloroethyl)phenyl group, a 2,4-ditrifluoromethylphenyl group, a 3-chlorophenyl group, a 4-methoxyphenyl group, a 3-dimethylaminophenyl group, a 3-methoxyphenyl group, a 3-trifluoromethylphenyl group, a 2-fluorophenyl group and the like.

The "partially hydrogenated aryl group" means an aryl group wherein a part of the noncumulative double bond possessed by the aryl group is hydrogenated. Specific examples thereof include a cyclohexenyl group, a cyclohexadienyl group, a 1,2,3,4-tetrahydronaphthyl group, a 2,3-dihydroindenyl group and the like.

The "optionally substituted partially hydrogenated aryl group" means a partially hydrogenated aryl group optionally substituted at each substitutable position by 1 to 4 atoms or groups selected from the group consisting of a halogen atom, a C₁₋₆ alkyl group, a C₁₋₆ alkoxy group, a trifluoromethyl group, a trifluoromethoxy group, a cyano group, a nitro group, a carboxyl group, a C₁₋₃ alkoxy-C₁₋₃ alkyl group, a C₁₋₃ alkoxycarbonyl group, a C₁₋₃ alkylcarbonyl group, a C₁₋₃ alkylthio group, a C₁₋₃ alkylsulfonyl group, a hydroxy group and an amino group (the amino group may be substituted by one or the same or different two C₁₋₃ alkyl groups). Specific examples of the substituted partially hydrogenated aryl group include a 5-fluoro-tetrahydronaphthyl group, a 7-chloro-tetrahydronaphthyl group, a 4-methyl-tetrahydronaphthyl group, a 6-hydroxy-tetrahydronaphthyl group, a 5-methoxy-dihydroindenyl group and the like.

The "heterocyclic group" means a 3- to 14-membered monocycle, bicyclic or tricyclic aromatic or nonaromatic heterocyclic group optionally containing, besides carbon atom, 1 to 3 hetero atoms selected from a nitrogen atom, a sulfur atom and an oxygen atom. Specific examples thereof include a thienyl group, a furyl group, a xanthenyl group, an indolyl group, an isoindolinyl group, a 2,3-dihydro-1H-indolinyl group, a pyridinyl group, a [1,3]benzothiazolyl group, a benzofuranyl group, an oxazolyl group, an isoxazolyl group, a thiazolyl group, an isothiazolyl group, a pyridyl group, a pyrazinyl group, a pyrimidinyl group, a pyridazinyl group, a [1,2,4]oxadiazolyl group, a tetrazolyl group, a [1,2,4]triazinyl group, a benzooxazolyl group, a benzoisoxazolyl group, a pyrrolidinyl group, a piperazinyl group, a piperidinyl group, a morpholinyl group, a perhydroisoquinolinyl group, a 1,2,3,4-tetrahydroisoquinolinyl group, an azetidinyl group, a perhydroazepinyl group, a hexahydrodiazepinyl group, a tetrahydro-2H-pyranyl group, a tetrahydrofuranylimidazoyl group, a benzoimidazoyl group, a benzoisothiazolyl group, a quinolyl group, an isoquinolyl group, a quinoxalinyl group, a quinazolinyl group, a phthalazinyl group, a cinnolinyl group, a naphthyridinyl group and the like. Specific preferable examples from among these include a thienyl group, a furyl group, a xanthenyl group, an indolyl group, an isoindolinyl group, a 2,3-dihydro-1H-indolinyl group, a pyridinyl group, a [1,3]benzothiazolyl group, a benzofuranyl group, a pyridyl group, a piperidinyl group, a morpholinyl group, a perhydroisoquinolinyl group, a 1,2,3,4-tetrahydroisoquinolinyl group, a [1,2,4]oxadiazolyl group, a tetrazolyl group, a [1,2,4]triazinyl group, a tetrahydro-2H-pyranyl group and the like.

The "optionally substituted heterocyclic group " means a heterocyclic group optionally substituted at each substitutable position by 1 to 4 atoms or groups selected from the group consisting of a halogen atom, a C₁₋₆ alkyl group, a C₁₋₆ alkoxy group, a trifluoromethyl group, a trifluoromethoxy group, a cyano group, a phenyl group, a C₃₋₈ cycloalkyl group, a C₁₋₃ alkoxy-C₁₋₃ alkyl group, a C₁₋₆ alkoxycarbonyl group, a C₁₋₃ alkylthio group, a C₁₋₃ alkylsulfonyl group, a hydroxy group, a nitro group, an oxo group and an amino group (the amino group may be substituted by one or the same or different two C₁₋₃ alkyl groups or C₃₋₈ cycloalkyl groups). Specific preferable examples of the substituted heterocyclic group include a 2-methylfuryl group, a 3-methylfuryl group, a 3-phenylfuryl group, a 2-trifluoromethylpyridyl group, a 2-chloropyridyl group, a 2-hydroxypyridyl group, a 2-methoxypyridyl group, a N-methylpiperidinyl group, a 2-phenylpiperidinyl group, a 2-(3,3,5,5-tetramethylcyclohexylamino)pyridyl group, a 2-(tetrahydro-2H-pyran-4-ylamino)pyridyl group, a 4-methoxy-2-quinolyl group, a 5-chloroindolyl group, a 5-nitrobenzofuranyl group, a 2-methylbenzoimidazolyl group, a 5-methyl[1,2,4]oxadiazolyl group, a 3-methyl[1,2,4]oxadiazolyl group, a 1-methylpyrrolyl group, a 1,5-dimethylpyrazolyl group, a 2-methylthiopyridyl group, a phthalimidyl group, a 2,6-dichloropyridyl group, a 2,3-dichloropyridyl group, a 2-(cycloheptylamino)pyridyl group and the like.

The "optionally substituted benzyl group" means a benzyl group wherein the benzene ring portion is optionally substituted at each substitutable position by 1 to 4 atoms or groups selected from the group consisting of a halogen atom, a C₁₋₆ alkyl group, a C₁₋₃ alkoxy group, a trifluoromethyl group, a trifluoromethoxy group, a cyano group, a nitro group, a C₁₋₃ alkoxy-C₁₋₃ alkyl group, a C₁₋₃ alkoxycarbonyl group, a C₁₋₃ alkylcarbonyl group, a C₁₋₃ alkylthio group, a C₁₋₃ alkylsulfonyl group, a hydroxy group, a carboxyl group and an amino group (the amino group may be substituted by one or the same or different two C₁₋₃ alkyl groups). Specific examples of the substituted benzyl group include a 3-fluorobenzyl group, a 4-chlorobenzyl group, a 2-methylbenzyl group, a 2-methoxybenzyl group, a 4-trifluoromethylbenzyl group, a 4-trifluoromethoxybenzyl group, a 4-cyanobenzyl group, a 3-nitrobenzyl group, a 4-methoxycarbonylbenzyl group, a 2-acetylbenzyl group, a 4-methylthiobenzyl group, a 4-methylsulfonylbenzyl group, a 2-hydroxybenzyl group, a 2-carboxybenzyl group, a 4-dimethylaminobenzyl group and the like.

The "optionally substituted benzoyl group" means a benzoyl group, wherein the benzene ring portion is optionally substituted at each substitutable position by 1 to 4 atoms or groups selected from the group consisting of a halogen atom, a C₁₋₆ alkyl group, a C₁₋₃ alkoxy group, a trifluoromethyl group, a trifluoromethoxy group, a cyano group, a nitro group, a C₁₋₃ alkoxy-C₁₋₃ alkyl group, a C₁₋₃ alkoxycarbonyl group, a C₁₋₃ alkylcarbonyl group, a C₁₋₃ alkylthio group, a C₁₋₃ alkylsulfonyl group, a hydroxy group, a carboxyl group and an amino group (the amino group may be substituted by one or the same or different two C₁₋₃ alkyl groups). Specific examples of the substituted benzoyl group include a 3-fluorobenzoyl group, a 4-chlorobenzoyl group, a 2-methylbenzoyl group, a 2-methoxybenzoyl group, a 4-trifluoromethylbenzoyl group, a 4-trifluoromethoxybenzoyl group, a 4-cyanobenzoyl group, a 3-nitrobenzoyl group, a 4-methoxycarbonylbenzoyl group, a 2-acetylbenzoyl group, a 4-methylthiobenzoyl group, a 4-methylsulfonylbenzoyl group, a 2-hydroxybenzoyl group, a 2-carboxybenzoyl group, a 4-dimethylaminobenzoyl group and the like.

The "optionally substituted heterocarbonyl group" means a carbonyl group having a 3- to 11-membered monocyclic or bicyclic aromatic or nonaromatic heterocyclic group optionally containing, besides carbon atom, 1 to 3 hetero atoms selected from a nitrogen atom, a sulfur atom and an oxygen atom, and means a heterocarbonyl group optionally substituted at each substitutable position by 1 to 4 atoms or groups selected from the group consisting of a halogen atom, a C₁₋₆ alkyl group, a C₁₋₃ alkoxy group, a trifluoromethyl group and an amino group (the amino group may be substituted by one or the same or different two C₁₋₃ alkyl groups). Specific examples of the substituted heterocarbonyl group include a pyrrolidinylcarbonyl group, a piperidylcarbonyl group, a morpholinocarbonyl group, a 4-methylpiperazinylcarbonyl group, an indolinylcarbonyl group, a 2-methoxypyridylcarbonyl group, a 4-fluoropyridylcarbonyl group, a 2-trifluoromethylpyridylcarbonyl group, a dimethylaminopiperidylcarbonyl group, a 2-furylcarbonyl group and the like.

The "substituted piperidine ring" means a piperidine ring substituted by a group selected from the group consisting of a C₁₋₆ alkyl group; a phenyl group [the phenyl group may be substituted by 1 to 4 atoms or groups selected from the group consisting of a halogen atom, a C₁₋₆ alkyl group, a C₁₋₆ alkoxy group, a trifluoromethyl group, a trifluoromethoxy group, a nitro group, a C₁₋₃ alkylcarbonyl group, a hydroxy group and an amino group (the amino group may be substituted by one or the same or different two C₁₋₃ alkyl groups)]; a benzyl group [the benzyl group may be substituted by 1 to 4 atoms or groups selected from the group consisting of a halogen atom, a C₁₋₆ alkyl group, a C₁₋₆ alkoxy group, a trifluoromethyl group, a trifluoromethoxy group, a nitro group, a C₁₋₃ alkylcarbonyl group, a hydroxy group and an amino group (the amino group may be substituted by one or the same or different two C₁₋₃ alkyl groups)]; and a C₁₋₃ alkylcarbonyl group. The substituent is preferably present at the nitrogen atom in the piperidine ring. Specific preferable examples include an N-methylpiperidine ring, an N-ethylpiperidine ring, an N-isopropylpiperidine ring, an N-phenylpiperidine ring, an N-benzylpiperidine ring, an N-acetylpiperidine ring and the like.

The "halogen atom" means fluorine, chlorine, bromine or iodine.

Specific examples of the compound of the present invention include the compounds recited in the below-mentioned Examples (Examples 1 to 230).

Preferable examples of the compound of the present invention include the following compounds and physiologically acceptable salts thereof:
N-[1-(4-chloro-2,5-difluorobenzoyl)piperidin-4-yl]-N-phenyl-2-(piperidin-1-yl)acetamide (compound of Example 8),
N-phenyl-2-(piperidin-1-yl)-N-[1-(4-trifluoromethylbenzoyl)piperidin-4-yl]acetamide (compound of Example 9 ),
N-[1-(3-fluoro-4-trifluoromethylbenzoyl)piperidin-4-yl]-N-phenyl-2-(piperidin-1-yl)acetamide (compound of Example 12)
N-phenyl-2-(piperidin-1-yl)-N-[1-(4-trifluoromethoxybenzoyl)piperidin-4-yl]acetamide (compound of Example 13),
2-(morpholin-4-yl)-N-phenyl-N-[1-(4-trifluoromethylbenzoyl)piperidin-4-yl]acetamide (compound of Example 29),
N-[1-(3-fluoro-4-trifluoromethylbenzoyl)piperidin-4-yl]-2-(morpholin-4-yl)-N-phenylacetamide (compound of Example 32),
N-[1-(2,3-difluoro-4-trifluoromethylbenzoyl)piperidin-4-yl]-2-(morpholin-4-yl)-N-phenylacetamide (compound of Example 34),
N-(4-methoxyphenyl)-2-(piperidin-1-yl)-N-[1-(4-trifluoromethylbenzoyl)piperidin-4-yl]acetamide (compound of Example 55),
N-[l-(4-chlorobenzoyl)piperidin-4-yl]-N-cyclohexyl-2-(morpholin-4-yl)acetamide (compound of Example 65),
N-[1-(trans-4-trifluoromethylcyclohexylcarbonyl)piperidin-4-yl]-2-(morpholin-4-yl)-N-phenylacetamide (compound of Example 86),
N-phenyl-2-(pyrrolidin-1-yl)-N-[1-(4-trifluoromethylbenzoyl)piperidin-4-yl]acetamide (compound of Example 103),
N-[1-(3,4-dichlorobenzoyl)piperidin-4-yl]-N-phenyl-2-(pyrrolidin-1-yl)acetamide (compound of Example 104), 3-(morpholin-4-yl)-N-phenyl-N-[1-(4-trifluoromethylbenzoyl)piperidin-4-yl]propionamide (compound of Example 154),
N-[1-(4-chlorobenzoyl)piperidin-4-yl]-N-phenyl-3-(pyrrolidin-1-yl)propionamide (compound of Example 158),
N-[1-[2-(4-chlorophenyl)-2-methylpropionyl]piperidin-4-yl]-N-phenyl-2-(piperidin-1-yl)acetamide (compound of Example 190),
N-[1-[1-(4-chlorophenyl)cyclopentane-1-carbonyl]piperidin-4-yl]-N-phenyl-2-(piperidin-1-yl)acetamide (compound of Example 198),
N-phenyl-2-(piperidin-1-yl)-N-[1-[1-(3-trifluoromethylphenyl)cyclopentane-1-carbonyl]piperidin-4-yl]acetamide (compound of Example 199),
N-[1-[1-(2-fluorophenyl)cyclopentane-1-carbonyl]piperidin-4-yl]-N-phenyl-2-(piperidin-1-yl)acetamide (compound of Example 202),
2-(3-dimethylaminopyrrolidin-1-yl)-N-phenyl-N-[1-(4-trifluoromethylbenzoyl)piperidin-4-yl]acetamide (compound of Example 131),
N-[1-(2,4-ditrifluoromethylbenzoyl)piperidin-4-yl]-2-(morpholin-4-yl)-N-phenylacetamide (compound of Example 50),
N-cyclohexyl-2-(pyrrolidin-1-yl)-N-[1-(4-trifluoromethylbenzoyl)piperidin-4-yl]acetamide (compound of Example 77),
2-(3-hydroxypyrrolidin-1-yl)-N-phenyl-N-[1-(4-trifluoromethylbenzoyl)piperidin-4-yl]acetamide (compound of Example 135),
2-[4-(5-methyloxodiazol-3-yl)]piperidin-1-yl]-N-phenyl-N-[1-(4-trifluoromethylbenzoyl)piperidin-4-yl]acetamide (compound of Example 138),
3-(4-methylpiperazin-1-yl)-N-phenyl-N-[1-(4-trifluoromethylbenzoyl)piperidin-4-yl]propionamide (compound of Example 159),
3-(4-dimethylaminopiperidine-1-yl)-N-phenyl-N-[1-(4-trifluoromethylbenzoyl)piperidin-4-yl]propionamide (compound of Example 161),
3-(4-methylpiperazin-1-yl)-N-phenyl-N-[1-[(benzofuran-2-yl)carbonyl]piperidin-4-yl]propionamide (compound of Example 174),
N-(3-carboxylphenyl)-2-(pyrrolidin-1-yl)-N-[1-(4-trifluoromethylbenzoyl)piperidin-4-yl]acetamide (compound of Example 227), and
N-[1-(3-fluoro-4-trifluoromethylbenzoyl)piperidin-4-yl]-3-(4-methylpiperazin-1-yl)-N-phenylpropionamide (compound of Example 176).

The production methods of the compound of the present invention are now explained in the following.
The compound represented by the formula (I) of the present invention can be produced by production method A or production method B shown below.

[Production method A]: A method of producing a compound represented by the above-mentioned formula (I), comprising reacting a compound represented by the following formula (II)

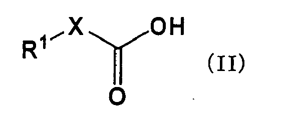

wherein R¹ and X are as defined in the above-mentioned (1), or a reactive derivative thereof, with a compound represented by the following formula (III)

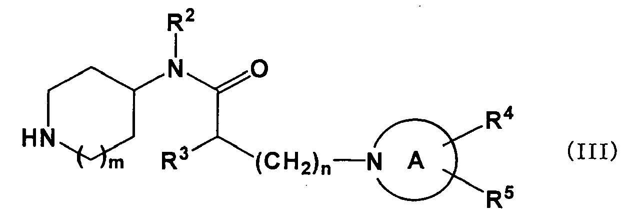

wherein R², R³, R⁴, R⁵, m, n and a group: ring A are as defined in the above-mentioned (1).

Examples of the reactive derivative of the compound of the formula (II) include active ester, acid anhydride, acid halide, lower alkyl ester and the like, which are induced from a compound of the formula (II). Specific examples of the active ester include p-nitrophenyl ester, 2,4,5-trichlorophenyl ester, N-hydroxysuccinate imide ester, N-hydroxyphthalimido ester, 1-hydroxybenzotriazole ester, N-hydroxypiperidine ester, 2-pyridylthiol ester, N-methylimidazole ester and the like. As the acid anhydride, a symmetric acid anhydride or a mixed acid anhydride can be used. Specific examples of the mixed acid anhydride include mixed acid anhydride with ethyl chlorcarbonate, isobutyl chlorocarbonate or phenyl chlorcarbonate, a mixed acid anhydride with alkane such as isovaleric acid, pivalic acid, and the like. As the acid halide, acid chloride is preferable. As the lower alkyl ester, methyl ester is preferable.

The reaction between the compound of the formula (II) and the compound of the formula (III) can be performed in the presence of a condensing agent such as dicyclohexylcarbodiimide, 1-ethyl-3-(3-dimethylaminopropyl)carbodiimide hydrochloride, N,N-carbonyldiimidazole, dimethylaminosulfonyl chloride, and 1-ethoxycarbonyl-2-ethoxy-1,2-dihydroquinoline. When dicyclohexylcarbodiimide or 1-ethyl-3-(3-dimethylaminopropyl)carbodiimide hydrochloride is used as a condensing agent, N-hydroxysuccinimide, N-hydroxyphthalimide, 1-hydroxybenzotriazol, 3-hydroxy-4-oxo-3,4-dihydro-1,2,3-benzotriazine and the like may be used in combination.

The reaction between the compound of the formula (II) or a reactive derivative thereof and the compound of the formula (III) is performed without solvent or in a suitable solvent. Examples of the solvent to be used include toluene, xylene, diethyl ether, tetrahydrofuran, dioxane, methylene chloride, chloroform, ethyl acetate, acetonitrile, dimethylformamide, dimethyl sulfoxide and the like, and these solvents are used alone or in a mixture of two or more kinds thereof. This reaction is performed in the presence of a base as necessary. Specific examples of the base include inorganic bases such as sodium hydrogen carbonate, potassium hydrogen carbonate, sodium bicarbonate, potassium bicarbonate and sodium hydroxide, and organic bases such as triethylamine, diisopropylethylamine, N-methylmorpholine and dimethylaminopyridine. When the compound of the formula (III) is added in excess to the reaction, the compound functions as a base. The reaction temperature is generally -40°C to 200°C, preferably -20°C to 70°C.

[Production method B]: A method of producing a compound represented by the above-mentioned formula (I), comprising reacting a compound represented by the following formula (IV)

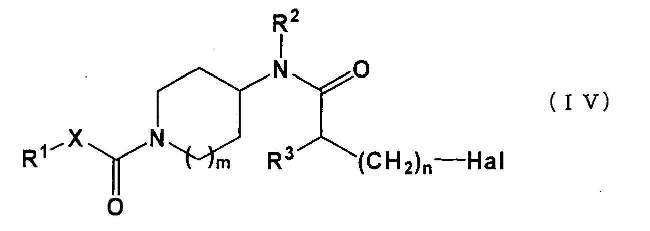

wherein Hal is Br or Cl, and R¹, R², R³, m, n and X are as defined in the above-mentioned (1), or a reactive derivative thereof, with a compound represented by the following formula (V)

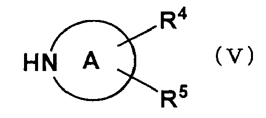

wherein R⁴, R⁵ and a group: ring A are as defined in the above-mentioned (1).

The reaction between the compound of the formula (IV) and the compound of the formula (V) is performed without solvent or in a suitable solvent. Examples of the solvent to be used include toluene, xylene, tetrahydrofuran, dioxane, chloroform, acetone, ethyl acetate, acetonitrile, dimethylformamide, dimethyl sulfoxide and the like, and these solvents are used alone or in a mixture of two or more kinds thereof. This reaction is performed in the presence of a base as necessary. Specific examples of the base include inorganic bases such as sodium hydrogen carbonate, potassium hydrogen carbonate, sodium bicarbonate and potassium bicarbonate, and organic bases such as triethylamine, diisopropylethylamine, N-methylmorpholine, dimethylaminopyridine and pyridine. When the compound of the formula (V) is added in excess to the reaction, the compound functions as a base. The reaction temperature is generally -20°C to 150°C, preferably 0°C to 120°C.

The production methods of compounds represented by the formulas (II) - (V) are now explained in the following.

The compound of the formula (II) may be, for example, a commercially available compound, or can be produced by a known method such as the method described in a reference, Chemistry Letters, No. 3, 192-193, 2001. or a method shown in the following scheme 1.

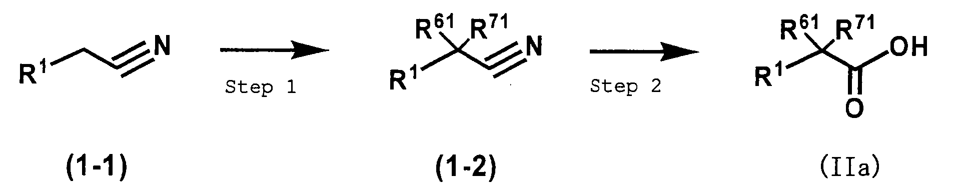

wherein R⁶¹ and R⁷¹ are the same or different and each is a hydrogen atom or a C₁₋₆ alkyl group, or R⁶¹ and R⁷¹ form, together with the carbon atom bonded thereto, a C₃₋₈ cycloalkane ring, a substituted piperidine ring or a tetrahydro-2H-pyran ring, and R¹ is as defined in the above-mentioned (1).

Compound (1-2) can be obtained by reacting compound (1-1) with an organic base such as potassium hexamethyldisilasane, and reacting the resulting compound with the corresponding brominated form or chlorinated form in a suitable solvent. Examples of the solvent to be used include toluene, xylene, tetrahydrofuran, dioxane, methylene chloride, chloroform, acetonitrile, dimethylformamide and the like, and these solvents are used alone or in a mixture of two or more kinds thereof. The reaction temperature is generally -20°C to 150°C, preferably 0°C to 120°C.

Compound (IIa) can be obtained by reacting compound (1-2) with an inorganic base, such as potassium hydroxide and sodium hydroxide, in a suitable solvent. Examples of the solvent to be used include toluene, xylene, tetrahydrofuran, dioxane, methylene chloride, chloroform, acetonitrile, dimethylformamide, water, ethanol, methanol and the like, and these solvents are used alone or in a mixture of two or more kinds thereof. The reaction temperature is generally -20°C to 200°C, preferably 0°C to 150°C

A compound of the formula (III) can be produced by a method shown in the following scheme 2.

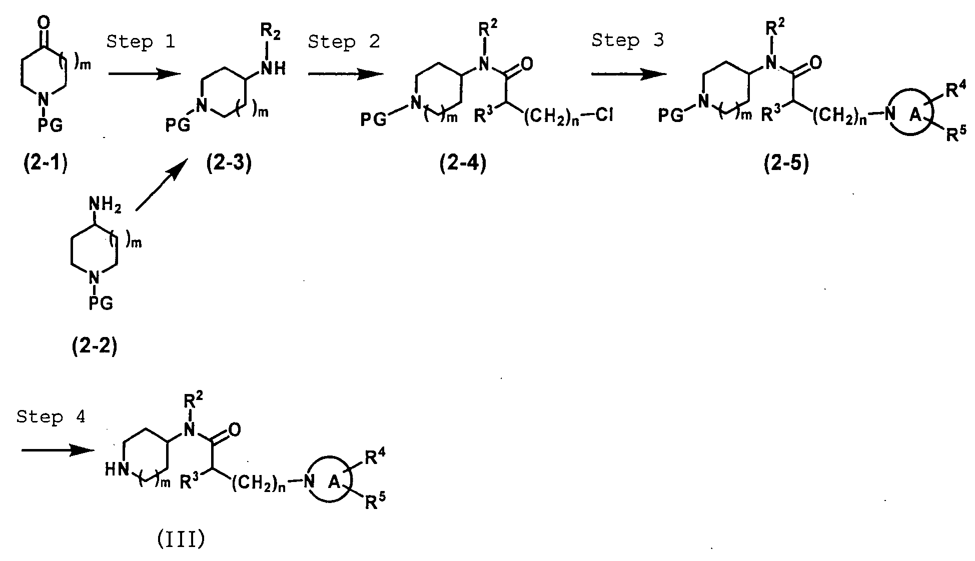

wherein PG is a protecting group, and R², R³, R⁴, R⁵, m, n and a ring group A are as defined in the above-mentioned (1).

Compound (2-3) can be produced by a reductive alkylation using compound (2-1) and an available corresponding primary monoamine in a suitable solvent. The reaction is performed by the use of a reducing agent such as sodium borohydride, sodium triacetoxyborate, sodium cyanoborohydride, picoline-borane and the like, or by a catalytic reduction using a metal catalyst such as palladium and the like under a hydrogen atmosphere. Examples of the solvent to be used include toluene, xylene, tetrahydrofuran, dioxane, methylene chloride, chloroform, acetonitrile, methanol, ethanol and the like, and these solvents are used alone or in a mixture of two or more kinds thereof. This reaction more preferably proceeds when an acid such as acetic acid, hydrochloric acid and the like is added as a reaction catalyst. The reaction temperature is generally -20°C to 150°C, preferably 0°C to 100°C.

Compound (2-3) can also be produced by reacting the corresponding bromine-substituted aryl or chlorine-substituted heteroaryl with compound (2-2) in the presence of a palladium catalyst [e.g., tris(dibenzylideneacetone)dipalladium], a phosphine ligand (e.g., 2',6'-dimethoxy-2-dicyclohexylphosphinobiphenyl) and an organic base (e.g., t-butoxy sodium). Examples of the solvent to be used include toluene, xylene, tetrahydrofuran, dioxane, methylene chloride, chloroform, acetonitrile, dimethylformamide and the like, and these solvents are used alone or in a mixture of two or more kinds thereof. The reaction temperature is generally 0°C to 150°C, preferably 25°C to 120°C.

Compound (2-4) can be produced by reacting compound (2-3) with the corresponding acid halide (e.g., chloroacetyl chloride, bromoacetyl bromide, chloropropionyl chloride and the like) in a suitable in a solvent. Examples of the solvent to be used include toluene, xylene, tetrahydrofuran, dioxane, chloroform, acetone, methyl ethyl ketone, ethyl acetate, acetonitrile, dimethylformamide, dimethyl sulfoxide and the like, and these solvents are used alone or in a mixture of two or more kinds thereof. This reaction is performed in the presence of a base as necessary. Specific examples of the base include inorganic bases such as sodium hydrogen carbonate, potassium hydrogen carbonate, sodium bicarbonate and potassium bicarbonate, and organic bases such as triethylamine, diisopropylethylamine, N-methylmorpholine, dimethylaminopyridine and pyridine. The reaction temperature is generally -20°C to 150°C, preferably 0°C to 120°C.

Compound (2-5) can be produced by reacting compound (2-4) with the corresponding saturated nitrogen atom-containing hetero ring compound without solvent or in a suitable solvent. Examples of the solvent to be used include toluene, xylene, tetrahydrofuran, dioxane, methylene chloride, chloroform, acetonitrile, dimethylformamide and the like, and these solvents are used alone or in a mixture of two or more kinds thereof. This reaction is performed in the presence of a base as necessary. Specific examples of the base include inorganic bases such as sodium hydrogen carbonate, potassium hydrogen carbonate, sodium bicarbonate and potassium bicarbonate, and organic bases such as triethylamine, diisopropylethylamine, N-methylmorpholine, dimethylaminopyridine and pyridine. Moreover, when the corresponding saturated nitrogen atom-containing hetero ring compound is added in excess to the reaction solution, the compound functions as a base. The reaction temperature is generally -20°C to 150°C, preferably 0°C to 120°C.

Compound (III) can be obtained by removing a protecting group of compound (2-5) according to a conventional method. Specific examples of the protecting group include a group forming carbamates such as a t-butoxycarbonyl group, a benzyloxycarbonyl group and the like, a group forming benzylamines such as a benzyl group, a trityl group and the like.

A compound of the formula (IV) can be produced by a method shown in the following scheme 3.

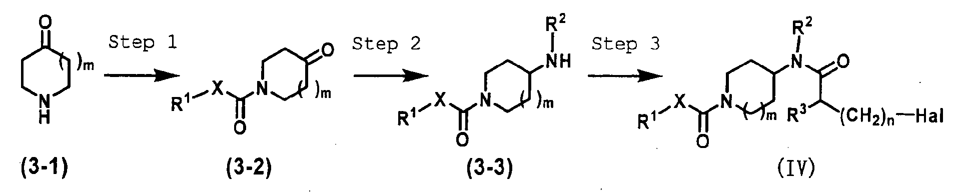

wherein Hal is Br or Cl, and R¹, R², R³, m, n and X are as defined in the above-mentioned (1).

Compound (3-2) can be produced by subjecting compound (3-1) and the corresponding carboxylic acid or a reactive derivative thereof to an operation similar to the reaction between the compound of the formula (II) and the compound of the formula (III) shown in the aforementioned Production method A.

Compound (3-3) can be produced by a reductive alkylation using compound (3-2) and the corresponding primary monoamine. This reaction can be performed in the same manner as in the production method of compound (2-3) by reductive alkylation.

The formula (IV) can be produced by reacting compound (3-3) with the corresponding acid halide (e.g., chloroacetyl chloride, bromoacetyl bromide, chloropropionyl chloride and the like) in a suitable solvent. Examples of the solvent to be used include toluene, xylene, tetrahydrofuran, dioxane, chloroform, acetone, methyl ethyl ketone, ethyl acetate, acetonitrile, dimethylformamide, dimethyl sulfoxide and the like, and these solvents are used alone or in a mixture of two or more kinds thereof. This reaction is performed in the presence of a base as necessary. Specific examples of the base include inorganic bases such as sodium hydrogen carbonate, potassium hydrogen carbonate, sodium bicarbonate and potassium bicarbonate, and organic bases such as triethylamine, diisopropylethylamine, N-methylmorpholine, dimethylaminopyridine and pyridine. The reaction temperature is generally -20°C to 150°C, preferably 0°C to 120°C.

The compound of the formula (V) may be a commercially available compound, or can be produced by a known method.

The compounds of the formula (I) obtained by the production methods shown above can be isolated and purified according to a conventional method such as extraction, silica gel column chromatography, recrystallization and reprecipitation. As the extraction solvent, diethyl ether, ethyl acetate, chloroform, dichloromethane, toluene and the like are used. Purification by silica gel column chromatography can be performed using acidic, basic or variously chemically-treated silica gel, alumina and the like. As an eluent, for example, hexane/ethyl acetate, hexane/chloroform, ethyl acetate/methanol, chloroform/methanol, acetonitrile/water, methanol/water and the like can be used.

When the compound of the formula (I) is a racemate, it can be separated and purified into each enantiomer according to a conventional method such as chromatography using an optically active column, optical resolution using an optically active acid or synthetic chiral resolving reagent and the like, preferential crystallization, diastereomer method and the like. In addition, an optically active compound of the formula (I) can also be produced by reacting an optically active compound of the formula (III) with a compound of the formula (II). For example, separation into an enantiomer using an optically active acid can be performed by forming a diastereomer salt according to a conventional method, separating the salt into two kinds of diastereomer salts, then converting them into free bases. Examples of the optically active acid to be used as an optical resolution reagent include (+)- or (-)-camphoric acid, (1S)-(+)- or (1R)-(-)-camphor-10-sulfonic acid, L-(+)-or D-(-)-tartaric acid, (+)- or (-)-mandelic acid, (S)-(-)- or (R)-(+)-malic acid, L-pyroglutamic acid, (S)-(+)- or (R)-(-)-1,1'-binaphthyl-2,2'-diyl, (+)-dibenzoyl-D-tartaric acid or (-)-dibenzoyl-L-tartaric acid and the like.

The compound of the formula (I) having a hydroxy group can be synthesized by performing protection and deprotection of hydroxy group according to a conventional method.

While the compound of the formula (I) is obtained in the form of a free base or an acid addition salt depending on the kind of the functional group present in the structural formula, selection of a starting material compound and reaction treatment conditions, it can be converted to the compound of the formula (I) according to a conventional method. In addition, the compound of the formula (I) can be led to an acid addition salt by treatments with various acids according to a conventional method.

Since the compound of the present invention has a superior antiallergic action as a non-steroidal pharmaceutical agent as mentioned below, it is expected to be usable for human and mammals as a safe antiallergic agent with a few side effects. The compound of the present invention is useful as a prophylactic and/or therapeutic drug for allergic diseases such as allergic dermatitis, allergic rhinitis, bronchial asthma, atopic dermatitis, contact dermatitis, urticaria, eczema, allergic ophthalmia and pollinosis.

The administration route of the compound of the present invention may be any of oral administration, parenteral administration and intrarectal administration, and the daily dose thereof varies depending on the kind of the compound, administration method, and symptom, age and the like of patients. In the case of oral administration, for example, the dose is generally about 0.01-100 mg, more preferably about 0.1-10 mg, per 1 kg body weight of a human or mammal, which can be administered in one to several portions a day. In the case of parenteral administration such as intravenous injection and the like, for example, generally about 1 µg-10 mg, more preferably about 10 µg-1 mg, per 1 kg body weight of a human or mammal can be administered.

When the compound of the present invention is used for a pharmaceutical application as mentioned above, it is generally administered in the form of a preparation formulated by mixing with a carrier for preparation. As the carrier for preparation, a nontoxic substance conventionally used in the pharmaceutical field, which does not react with the compound of the present invention, is used. Specific examples thereof include citric acid, glutamic acid, glycine, lactose, inositol, glucose, mannitol, dextran, sorbitol, cyclodextrin, starch, partially pregelatinized magnesium, synthesis aluminum silicate, crystalline cellulose, sodium carboxymethylcellulose, hydroxypropylstarch, calcium carboxymethylcellulose, ion exchange resin, methylcellulose, gelatin, gum arabic, pullulan, hydroxypropylcellulose, low-substituted hydroxypropylcellulose, hydroxypropylmethylcellulose, polyvinylpyrrolidone, polyvinyl alcohol, alginic acid, sodium alginate, light anhydrous silicic acid, magnesium stearate, talc, tragacanth, bentonite, veegum, carboxyvinyl polymer, titanium oxide, sorbitan fatty acid ester, sodium lauryl sulfate, glycerol, fatty acid glycerol ester, purified lanolin, glycerol gelatin, polysorbate, macrogol, vegetable oil, wax, propylene glycol, ethanol, benzyl alcohol, sodium chloride, sodium hydroxide, hydrochloric acid, water and the like.

Examples of the dosage form include tablet, capsule, granule, powder, liquid, syrup, suspension, injection, suppository, eye drop, ointment, coating agent, adhesive preparation, inhalant and the like. These preparations can be formulated according to a conventional method. The liquid preparation may be in a form to be dissolved or suspended in water or other suitable medium when in use. In addition, tablet and granule may be coated by a well-known method. When a parenteral preparation, for example, injection, is to be produced, an aqueous solvent (e.g., distilled water, saline, Ringer's solution etc.), an isotonicity agent (e.g., glucose, D-sorbitol, D-mannitol, sodium chloride etc.), a stabilizer (e.g., human serum albumin etc.), a preservative (e.g., benzyl alcohol, chlorobutanol, methyl p-hydroxybenzoate, propyl p-hydroxybenzoate, phenol etc.), a buffer (e.g., phosphate buffer, sodium acetate buffer etc.) and a soothing agent (e.g., benzalkonium chloride, procaine hydrochloride etc.) can be appropriately added. Furthermore, these preparations may contain other components valuable for the treatment.

### Examples

The present invention is explained in more detail in the following by referring to Reference Examples, Examples and Experimental Examples, which are not to be construed as limitative. The compounds were identified by elemental analysis, hydrogen nuclear magnetic resonance absorption spectrum (¹H-NMR), LS-MS, melting point, optical rotation and the like. In the Reference Examples and Examples, CHROMATOREX NH manufactured by Fuji Silysia Chemical Ltd. was used for basic silica gel column chromatography.

To simplify the description in the specification, the following abbreviations may be used in the Reference Examples, Examples and Tables.
The abbreviations used for substituents are Me: methyl group, Ph: phenyl group, and cHex: cyclohexyl group.

The abbreviations used for NMR are s: singlet, d: doublet, dd: double doublet, t: triplet, td: triple doublet, q: quartet, m: multiplet, br: broad, brs: broad singlet, brd: broad doublet, brt: broad triplet and J: binding constant.

### Reference Example 1 (1): 4-(4-chlorophenyl)tetrahydro-2H-pyran-4-carboxylic acid

### [step 1]

To a solution of 4-chlorophenylacetonitrile (3.0 g) in dry tetrahydrofuran (50 ml) was added dropwise under an argon atmosphere and under ice-cooling a solution of 0.7 mol/L potassium hexamethyldisilazane in toluene (85 ml, 3.0 eq), and the mixture was stirred for 30 min. To this reaction mixture was added di(2-bromoethyl)ether (5.5 g, 1.2 eq), and the mixture was stirred for 12 hr. To this reaction mixture was added saturated aqueous ammonium chloride solution (50 ml), and the mixture was extracted with ethyl acetate (100 ml×2). The organic layer was washed with saturated brine (50 ml), dried over anhydrous magnesium sulfate and concentrated under reduced pressure. The obtained residue was crystallized from ethyl acetate/diisopropyl ether, and the crystals were collected by filtration to give 4-(4-chlorophenyl)tetrahydro-2H-pyran-4-carbonitrile (2.6 g, yield 59%).

### [step 2]

To a suspension of 4-(4-chlorophenyl)tetrahydro-2H-pyran-4-carbonitrile (2.6 g) obtained in the above-mentioned step 1 in water (50 ml) was added potassium hydroxide (3.3 g, 5.0 eq), and the mixture was heated under reflux for 5 hr. The reaction mixture was extracted with chloroform (100 ml×2). The organic layer was washed with saturated brine (50 ml), dried over anhydrous magnesium sulfate, and concentrated under reduced pressure. The obtained residue was crystallized from diisopropyl ether/hexane, and the crystals were collected by filtration to give the object product (2.0 g, yield 85%).

Using the corresponding starting material compounds and by the reaction and treatment in the same manner as in Reference Example 1 (1), the compounds of the following Reference Examples 1 (2)-(4) were obtained.
Reference Example 1 (2): 4-(4-trifluoromethylphenyl)tetrahydro-2H-pyran-4-carboxylic acid
Reference Example 1 (3): 1-(3-trifluoromethylphenyl) cyclopentane-1-carboxylic acid
Reference Example 1 (4): 1-(3-chlorophenyl)cyclopentane-1-carboxylic acid

### Reference Example 1 (5): 4-fluoromethylbenzoic acid

A solution of methyl 4-bromomethylbenzoate (2.0 g), potassium fluoride (1.0 g) and 18-crown ether-6 (230.7 mg) in acetonitrile (40 ml) was stirred under reflux for 48 hr. To the reaction mixture was added saturated aqueous ammonium chloride solution (20 ml) and the mixture was extracted with ethyl acetate (20 ml×3). The organic layer was washed with saturated brine (20 ml), dried over anhydrous magnesium sulfate, and concentrated under reduced pressure to give methyl 4-fluoromethylbenzoate. To this solution of methyl 4-fluoromethylbenzoate (300.0 mg) in tetrahydrofuran (5 ml) was added 1 mol/L aqueous sodium hydroxide solution (5.4 ml) and the mixture was stirred under reflux for 5 hr. The reaction mixture was allowed to cool and neutralized with hydrochloric acid. Tetrahydrofuran was evaporated under reduced pressure. The crystals precipitated in the aqueous solution were collected by filtration to give the object product (109.0 mg).

### Reference Example 1 (6): 4-(1-chloro-2,2,2-trifluoroethyl)benzoic acid

To a solution of 4-(2,2,2-trifluoroacetyl)benzoic acid (4.0 g) in methanol (100 ml) was added palladium hydroxide (200 mg) and the mixture was vigorously stirred under a hydrogen atmosphere (about 4 atm) and at room temperature for 42 hr. The reaction mixture was filtered, and the filtrate was concentrated under reduced pressure to give 4-(2,2,2-trifluoro-1-hydroxyethyl)benzoic acid (4.0 g). To a solution of 4-(2,2,2-trifluoro-1-hydroxyethyl)benzoic acid (1.0 g) in carbon tetrachloride (50 ml) was added triphenylphosphine (1.8 g) and the mixture was stirred under reflux for 5 hr. The reaction mixture was allowed to cool, and concentrated under reduced pressure. The obtained residue was purified by silica gel column chromatography (elution solvent: chloroform/methanol) to give the object product (343 mg).

### Reference Example 2 (1): N-phenyl-N-(piperidin-4-yl)-2-(piperidin-1-yl)acetamide

### [step 1]

To a solution of 1-t-butyloxycarbonylpiperidin-4-one (50.0 g) and aniline (19.5 g) in dichloromethane (250 ml) was added acetic acid (24.2 ml) and the mixture was stirred at room temperature for 30 min. To this reaction mixture was added, under ice-cooling, sodium triacetoxyborohydride (40.9 g) and the mixture was stirred at room temperature for 12 hr. To the reaction mixture was added saturated aqueous ammonium chloride solution (100 ml) and the mixture was extracted with chloroform (100 ml×3). The organic layer was washed with saturated brine (100 ml), dried over anhydrous magnesium sulfate, and concentrated under reduced pressure. The obtained residue was crystallized from ethyl acetate/diisopropyl ether and the crystals were collected by filtration to give t-butyl 4-phenylaminopiperidine-1-carboxylate (48.7 g, yield 84%).

### [step 2]

To a solution of t-butyl 4-phenylaminopiperidine-1-carboxylate (15.0 g) obtained in the above-mentioned step 1 and triethylamine (18.9 ml) in dichloromethane (200 ml) was added dropwise chloroacetyl chloride (9.2 ml) under a nitrogen atmosphere and ice-cooling. After the completion of the dropwise addition, the reaction mixture was warmed to room temperature and stirred for 12 hr. To the reaction mixture was added saturated aqueous sodium hydrogen carbonate solution (50 ml) and the mixture was extracted with chloroform (50 ml×3). The organic layer was washed with saturated brine (50 ml), dried over anhydrous magnesium sulfate, and concentrated under reduced pressure to give t-butyl 4-[N-(2-chloroacetyl)-N-phenylamino]piperidine-1-carboxylate (crude oil).

### [step 3]

To a mixture of t-butyl 4-[N-(2-chloroacetyl)-N-phenylamino]piperidine-1-carboxylate and potassium carbonate (26.3 g) obtained in the above-mentioned step 2 in acetonitrile (300 ml) was added piperidine (16.1 ml) and the mixture was stirred under reflux for 12 hr. Water (50 ml) was added to the reaction mixture, and the mixture was extracted with ethyl acetate (50 ml×3). The organic layer was washed with saturated brine (50 ml), dried over anhydrous magnesium sulfate, and concentrated under reduced pressure. The residue was purified by basic silica gel column chromatography (elution solvent: hexane/ethyl acetate) to give t-butyl 4-[N-phenyl-N-(2-piperidin-1-ylacetyl)amino]piperidine-1-carboxylate (16.9 g, yield of two steps of step 2 and step 3, 77%).

### [step 4]

To a solution of t-butyl 4-[N-phenyl-N-(2-piperidin-1-ylacetyl)amino]piperidine-1-carboxylate (16.9 g) obtained in the above-mentioned step 3 in dichloromethane (100 ml) was added trifluoroacetic acid (15.6 ml) and the mixture was stirred at room temperature for 12 hr. Under ice-cooling, the reaction mixture was neutralized with 1 mol/L aqueous sodium hydroxide solution (50 ml), and extracted with chloroform (50 ml×3). The organic layer was washed with saturated brine (50 ml), dried over anhydrous magnesium sulfate, and concentrated under reduced pressure to give the object product (15.7 g, yield 100%).

Using the corresponding acid chloride and corresponding cyclic amine and by the reaction and treatment in the same manner as in Reference Example 2 (1), step 2, the compounds of the following Reference Examples 2 (2) - (25) were obtained.
Reference Example 2 (2): 2-(morpholin-4-yl)-N-phenyl-N-(piperidin-4-yl)acetamide
Reference Example 2 (3): 2-(4-methylpiperazin-1-yl)-N-phenyl-N-(piperidin-4-yl)acetamide
Reference Example 2 (4): 3-(morpholin-4-yl)-N-phenyl-N-(piperidin-4-yl)propionamide
Reference Example 2 (5): N-phenyl-N-(piperidin-4-yl)-3-(piperidin-1-yl)propionamide
Reference Example 2 (6): N-phenyl-N-(piperidin-4-yl)-3-(pyrrolidin-1-yl)propionamide
Reference Example 2 (7): N-phenyl-N-(piperidin-4-yl)-2-(pyrrolidin-1-yl)acetamide
Reference Example 2 (8):2-(azepan-1-yl)-N-phenyl-N-(piperidin-4-yl) acetamide
Reference Example 2 (9): N-phenyl-N-(piperidin-4-yl)-2-(piperidin-2-one-1-yl)acetamide
Reference Example 2 (10): N-(4-fluorophenyl)-N-(piperidin-4-yl)-2-(piperidin-1-yl)acetamide
Reference Example 2 (11): N-(3-chlorophenyl)-N-(piperidin-4-yl)-2-(piperidin-1-yl)acetamide
Reference Example 2 (12): N-(4-methoxyphenyl)-N-(piperidin-4-yl)-2-(piperidin-1-yl)acetamide
Reference Example 2 (13): N-(2-chlorophenyl)-N-(piperidin-4-yl)-2-(piperidin-1-yl)acetamide
Reference Example 2 (14): N-(4-chlorophenyl)-2-(morpholin-4-yl)-N-(piperidin-4-yl)acetamide
Reference Example 2 (15): N-(3-chlorophenyl)-2-(morpholin-4-yl)-N-(piperidin-4-yl)acetamide
Reference Example 2 (16): N-(4-methylphenyl)-2-(morpholin-4-yl)-N-(piperidin-4-yl)acetamide
Reference Example 2 (17): 2-(morpholin-4-yl)-N-(piperidin-4-yl)-N-(4-trifluoromethylphenyl)acetamide
Reference Example 2 (18): N-cyclohexyl-2-(morpholin-4-yl)-N-(piperidin-4-yl)acetamide
Reference Example 2 (19): 2-(morpholin-4-yl)-N-phenyl-N-(pyrrolidin-3-yl)acetamide
Reference Example 2 (20): N-cyclohexyl-N-(piperidin-4-yl)-2-(pyrrolidin-1-yl)acetamide
Reference Example 2 (21): 3-(4-methylpiperazin-1-yl)-N-phenyl-N-(piperidin-4-yl)propionamide
Reference Example 2 (22): 3-(3-dimethylaminopyrrolidin-1-yl)-N-phenyl-N-(piperidin-4-yl)propionamide
Reference Example 2 (23): 3-[4-(1-methylpiperidin-4-yl)piperazin-1-yl]-N-phenyl-N-(piperidin-4-yl)propionamide
Reference Example 2 (24): 3-(4-dimethylaminopiperidin-1-yl)-N-phenyl-N-(piperidin-4-yl)propionamide
Reference Example 2 (25): 2-(3-hydroxypyrrolidin-1-yl)-3-methyl-N-phenyl-N-(piperidin-4-yl)butylamide

### Reference Example 2 (26): 2-(morpholin-4-yl)-N-(piperidin-4-yl)-N-(thiophen-3-yl)acetamide

### [step 1]

A solution of t-butyl 4-aminopiperidine-1-carboxylate (1.47 g), 3-bromothiophene (1.0 g), tris(dibenzylideneacetone)dipalladium (112.3 mg), 2',6'-dimethoxy-2-dicyclohexylphosphinobiphenyl (201.3 mg) and t-butoxy sodium (824.7 mg) in toluene (15 ml) was stirred under a nitrogen atmosphere and at 100°C for 16 hr. The reaction mixture was filtered through celite, water (10 ml) was added and the mixture was extracted with ethyl acetate (10 ml×3). The organic layer was washed with saturated brine (10 ml), dried over anhydrous magnesium sulfate, and concentrated under reduced pressure. The residue was purified by basic silica gel column chromatography (elution solvent: hexane/ethyl acetate) to give t-butyl 4-(thiophen-3-ylamino)piperidine-1-carboxylate (269 mg, yield 16%).

### [step 2]

By the reaction and treatment in the same manner as in Reference Example 2 (1), steps 3 and 4, of t-butyl 4-(thiophen-3-ylamino)piperidine-1-carboxylate (269 mg) obtained in the above-mentioned step 1, the object product (161 mg, yield 55%) was obtained.

### Reference Example 3 (1): 2-chloro-N-phenyl-N-[1-(4-trifluoromethylbenzoyl)piperidin-4-yl]acetamide

### [step 1]

To a solution of piperidin-4-one hydrochloride monohydrate (5.0 g) and triethylamine (13.6 ml) in dichloromethane (50 ml) was added dropwise 4-trifluoromethylbenzoylchloride (5.8 ml), and the mixture was stirred overnight at room temperature. To the reaction mixture was added saturated aqueous sodium hydrogen carbonate solution (30 ml) and the mixture was extracted with chloroform (20 ml×3). The organic layer was washed with 10% aqueous hydrochloric acid (30 ml), water (30 ml) and then saturated brine (30 ml), dried over anhydrous magnesium sulfate, and concentrated under reduced pressure to give 1-(4-trifluoromethylbenzoyl)piperidin-4-one (10 g) as a yellow oil.

### [step 2]

To a solution of 1-(4-trifluoromethylbenzoyl)piperidin-4-one (10 g) obtained in the above-mentioned step 1 and aniline (3.0 g) in dichloromethane (100 ml) was added acetic acid (3.9 g) and the mixture was stirred at room temperature for 30 min. Under ice-cooling, sodium triacetoxyborohydride (9.6 g) was added to the reaction mixture, and the mixture was stirred at room temperature for 12 hr. Saturated aqueous sodium hydrogen carbonate solution (30 ml) was added to the reaction mixture and the mixture was extracted with chloroform (20 ml×3). The organic layer was washed with saturated brine (30 ml), dried over anhydrous magnesium sulfate, and concentrated under reduced pressure. The obtained residue was crystallized from acetic acid/water (1:10) (55 ml), and the crystals were collected by filtration and washed with diisopropyl ether to give (4-phenylaminopiperidin-1-yl)-(4-trifluoromethylphenyl)methanone (10.4 g, yield of two steps of step 1 and step 2, 92%).

### [step 3]

To a solution of (4-phenylaminopiperidin-1-yl)-(4-trifluoromethylphenyl)methanone (17.4 g) obtained in the above-mentioned step 2 and dimethylaminopyridine (0.31 g) in dichloromethane (200 ml) was added dropwise chloroacetyl chloride (4.8 ml) under a nitrogen atmosphere and under ice-cooling. After the completion of the dropwise addition, the reaction mixture was stirred by heating under reflux overnight. Saturated aqueous sodium hydrogen carbonate solution (200 ml) was added to the reaction mixture, and the mixture was extracted with chloroform (100 ml×2). The organic layer was washed with 10% aqueous hydrochloric acid (200 ml), water (200 ml) and then saturated brine (200 ml), dried over anhydrous magnesium sulfate, and concentrated under reduced pressure. The obtained residue was crystallized from hexane/diisopropyl ether/ethyl acetate (4:1:1) (120 ml) and the crystals were collected by filtration and washed with hexane (60 ml) to give the object product (16.7 g, yield 79%).

Using the corresponding aniline or substituted primary amine and in the same manner as in Reference Example 3 (1), step 2, which is followed by subsequent reaction and treatment in the same manner, the compounds of the following Reference Examples 3 (2) - (17) were obtained.
Reference Example 3 (2): 2-chloro-N-naphthalen-1-yl-N-[1-(4-trifluoromethylbenzoyl)piperidin-4-yl]acetamide
Reference Example 3 (3): N-(benzothiazole-6-yl)-2-chloro-N-[1-(4-trifluoromethylbenzoyl)piperidin-4-yl]acetamide
Reference Example 3 (4): 2-chloro-N-(tetrahydro-2H-pyran-4-yl)-N-[1-(4-trifluoromethylbenzoyl)piperidin-4-yl]acetamide
Reference Example 3 (5): 2-chloro-N-(4,4-dimethylcyclohexan-1-yl)-N-[1-(4-trifluoromethylbenzoyl)piperidin-4-yl]acetamide
Reference Example 3 (6): 2-chloro-N-(1,2,3,4-tetrahydronaphthalen-1-yl)-N-[1-(4-trifluoromethylbenzoyl)piperidin-4-yl]acetamide
Reference Example 3 (7): 2-chloro-2-methyl-N-phenyl-N-[1-(4-trifluoromethylbenzoyl)piperidin-4-yl]acetamide
Reference Example 3 (8): 2-chloro-2-phenyl-N-phenyl-N-[1-(4-trifluoromethylbenzoyl)piperidin-4-yl]acetamide
Reference Example 3 (9): 2-chloro-N-(3-dimethylaminobenzen-1-yl)-N-[1-(4-trifluoromethylbenzoyl)piperidin-4-yl]acetamide
Reference Example 3 (10): 2-chloro-N-(3-methoxybenzen-1-yl)-N-[1-(4-trifluoromethylbenzoyl)piperidin-4-yl]acetamide
Reference Example 3 (11): 3-chloro-N-phenyl-N-[1-(4-trifluoromethylbenzoyl)piperidin-4-yl]propionamide
Reference Example 3 (12): 3-chloro-N-(3-methoxybenzen-1-yl)-N-[1-(4-trifluoromethylbenzoyl)piperidin-4-yl]propionamide
Reference Example 3 (13): 3-chloro-N-(3-fluorobenzen-1-yl)-N-[1-(4-trifluoromethylbenzoyl)piperidin-4-yl]propionamide
Reference Example 3 (14): 3-chloro-N-(3-chlorobenzen-1-yl)-N-[1-(4-trifluoromethylbenzoyl)piperidin-4-yl]propionamide
Reference Example 3 (15): 4-chloro-N-phenyl-N-[1-(4-trifluoromethylbenzoyl)piperidin-4-yl]butylamide
Reference Example 3 (16): 2-chloro-N-(3-chlorobenzen-1-yl)-N-[1-(4-trifluoromethylbenzoyl)piperidin-4-yl]acetamide
Reference Example 3 (17): 2-chloro-N-(3-ethoxycarbonylbenzen-1-yl)-N-[1-(4-trifluoromethylbenzoyl)piperidin-4-yl]acetamide

### Reference Example 4: 5-methyl-3-(piperidin-4-yl)oxadiazole

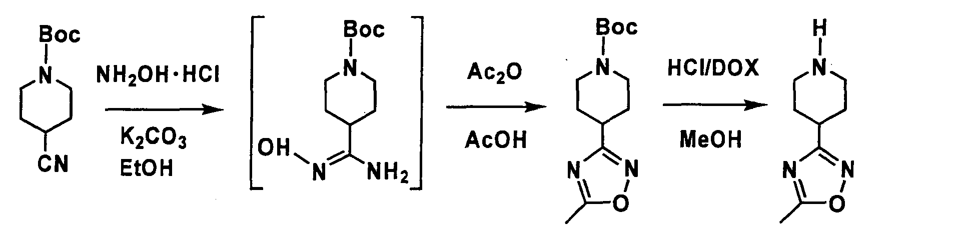

To a solution of N-t-butoxycarbonyl-4-cyanopiperidine (1.5 g) in ethanol (30 ml) were added potassium carbonate (3.5 g) and hydroxylamine hydrochloride (743.6 mg) and the mixture was stirred by heating under reflux for 19 hr. The reaction mixture was concentrated under reduced pressure, acetic acid (20 ml) and acetic anhydride (2.2 ml) were added, and the mixture was stirred with heating under reflux for 1.5 hr. Water (50 ml) was added to the reaction mixture, and the mixture was extracted with ethyl acetate (50 ml×3). The organic layer was washed with saturated brine (50 ml), dried over anhydrous magnesium sulfate, and concentrated under reduced pressure. The residue was purified by silica gel column chromatography (elution solvent: chloroform/methanol) to give 3-(1-t-butoxycarbonylpiperidin-4-yl)-5-methyloxadiazole (661 mg, yield 35%).
To a solution of the obtained 3-(1-t-butoxycarbonylpiperidin-4-yl)-5-methyloxadiazole (661 mg) in methanol (1 ml) was added 4 mol/L hydrochloric acid·ethyl acetate (1.9 ml) and the mixture was stirred at room temperature for 7 hr. The reaction mixture was concentrated under reduced pressure to give the object product (413 mg, yield 100%).

### Example 1: N-[1-(4-chlorobenzoyl)piperidin-4-yl]-N-phenyl-2-(piperidin-1-yl)acetamide

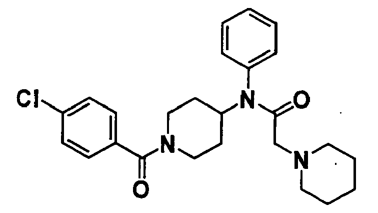

To a solution of N-phenyl-N-(piperidin-4-yl)-2-(piperidin-1-yl)acetamide (200 mg) synthesized in Reference Example 2 (1) and triethylamine (0.14 ml) in dichloromethane (10 ml) was added 4-chlorobenzoylchloride (151 mg) and the mixture was stirred at room temperature for 3 hr. Saturated aqueous sodium hydrogen carbonate solution (10 ml) was added to the reaction mixture and the mixture was extracted with chloroform (10 ml×3). The organic layer was washed with saturated brine (10 ml), dried over anhydrous magnesium sulfate, and concentrated under reduced pressure. The residue was purified by basic silica gel column chromatography (elution solvent: hexane/ethyl acetate) to give the object product (253 mg, yield 87%).

### Example 2: N-[1-(4-chloro-3-fluorobenzoyl)piperidin-4-yl]-N-phenyl-2-(piperidin-1-yl)acetamide

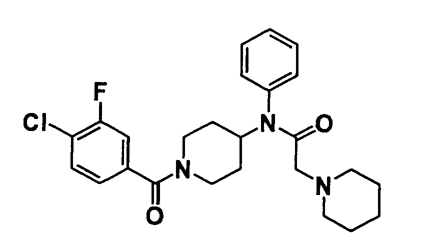

To a solution of 4-chloro-3-fluorobenzoic acid (166 mg), 1-ethyl-3-(3-dimethylaminopropyl)carbodiimide hydrochloride (WSC·HCl) (191 mg) and 1-hydroxybenzotriazole (1-HOBt) (134 mg) in dichloromethane (5 ml) was added a solution of N-phenyl-N-(piperidin-4-yl)-2-(piperidin-1-yl)acetamide (200 mg) synthesized in Reference Example 2 (1) in dichloromethane (5 ml) and the mixture was stirred at room temperature for 12 hr. Saturated aqueous sodium hydrogen carbonate solution (10 ml) was added to the reaction mixture and the mixture was extracted with chloroform (10 mlx3). The organic layer was washed with saturated brine (30 ml), dried over anhydrous magnesium sulfate, and concentrated under reduced pressure. The residue was purified by basic silica gel column chromatography (elution solvent: hexane/ethyl acetate) to give the object product (230 mg, yield 76%) as an oil. This product and fumaric acid (58.5 mg) were dissolved in isopropanol and concentrated under reduced pressure to give an amorphous form (229 mg).

### Example 3: N-phenyl-N-[1-(4-trifluoromethylbenzoyl)piperidin-4-yl]-2-[(4-ethoxycarbonyl)piperidin-1-yl]acetamide

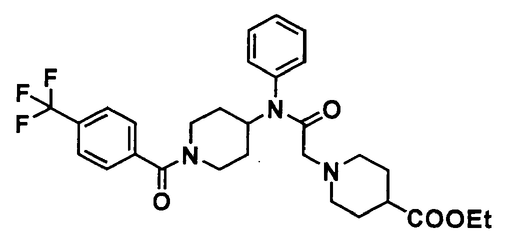

To a solution of 2-chloro-N-phenyl-N-[1-(4-trifluoromethylbenzoyl)piperidin-4-yl]acetamide (200 mg) synthesized in Reference Example 3 (1) in acetonitrile (5 ml) was added ethyl isonipecotate (222 mg) and the mixture was stirred at 70°C for 4.5 hr. The reaction mixture was concentrated under reduced pressure and the residue was purified by basic silica gel column chromatography (elution solvent: hexane/ethyl acetate) to give the object product (273 mg, yield 100%).

### Example 4: 3-methyl-2-(morpholin-4-yl)-N-phenyl-N-[1-(4-trifluoromethylbenzoyl)piperidin-4-yl]butylamide

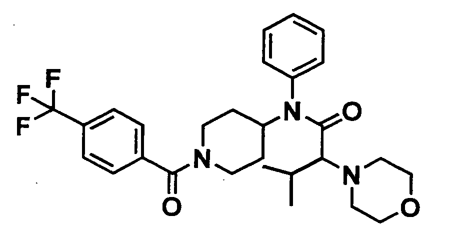

### [step 1]

(4-Phenylaminopiperidin-1-yl)-(4-trifluoromethylphenyl)methanone (0.70 g) obtained in Reference Example 3 (1), step 2, N-phthaloyl-DL-valine (0.59 g), diphenylphosphate chloride (0.95 g) and pyridine (0.32 g) were dissolved in acetonitrile (20 ml), and the mixture was heated under reflux overnight. After cooling to room temperature, saturated aqueous potassium carbonate solution was added and the mixture was extracted with ethyl acetate (30 ml×2). The organic layer was washed with 10 wt% aqueous citric acid solution, and then saturated brine, dried over anhydrous sodium sulfate, and concentrated under reduced pressure. The obtained oil was purified by silica gel column chromatography (elution solvent: hexane/ethyl acetate) to give 3-methyl-N-phenyl-2-(phthalimido-2-yl)-N-[1-(4-trifluoromethylbenzoyl)piperidin-4-yl]butylamide (1.15 g) as a colorless oil.

### [step 2]

The oil obtained in step 1, 3-methyl-N-phenyl-2-(phthalimido-2-yl)-N-[1-(4-trifluoromethylbenzoyl)piperidin-4-yl]butylamide (1.15 g), was dissolved in ethanol (10 ml) and the mixture was heated under reflux. Hydrazine monohydrate (0.20 g) was added dropwise and the mixture was stirred for 30 min. After cooling to room temperature, saturated aqueous potassium carbonate solution was added to dissolve a white solid, and the mixture was extracted with chloroform (30 ml×2). The organic layer was washed with saturated brine, dried over anhydrous sodium sulfate, and concentrated under reduced pressure to give 2-amino-3-methyl-N-phenyl-N-[1-(4-trifluoromethylbenzoyl)piperidin-4-yl]butylamide as an oil.

### [step 3]

The oil obtained in step 2, 2-amino-3-methyl-N-phenyl-N-[1-(4-trifluoromethylbenzoyl)piperidin-4-yl]butylamide, was dissolved in acetonitrile (20 ml), bis(2-bromoethyl)ether (0.70 g) and triethylamine (0.61 g) were added and the mixture was heated under reflux overnight. After cooling to room temperature, the mixture was concentrated under reduced pressure, and the obtained oil was purified by basic silica gel chromatography (elution solvent: hexane/ethyl acetate) to give a colorless oil (0.62 g). The oil was dissolved in ethanol (10 ml), 2 mol/L hydrochloric acid solution in ethanol (1 ml) was added and the mixture was concentrated under reduced pressure. Diisopropyl ether was added to the obtained residue and the mixture was filtered to give the object product as a white solid (0.55 g, yield of three steps from step 1 to step 3, 60%).

Unless particularly described, using the corresponding starting material compounds and by reaction and treatment in the same manner as in Example 1, Example 2 or Example 3, the compounds of Examples 5-224 were obtained. They are shown in Tables 1-11.

In the compounds of Examples 5-224 described in the Tables, those produced by a method similar to that of Example 1 are indicated as "production method I", those produced by a method similar to that of Example 2 are indicated as "production method II" and those produced by a method similar to that of Example 3 are indicated as "production method III" in the following Tables.
The compounds free of particular melting points mean that they were amorphous. Moreover, for example, "3,4-Cl₂-Ph" means a 3,4-dichloro-phenyl group, and "2,6,4-Cl₃-Ph" means a 2,6,4-trichloro-phenyl group.

**Table 1:**

| | | | |
|---|---|---|---|
| 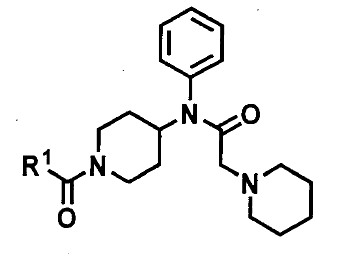 | | or a salt thereof | |

| Ex. No. | R¹ | salt | production method |
|---|---|---|---|
| 5 | 3,4-Cl₂-Ph | (free base) | I |
| 6 | 2,4,6-Cl₃-Ph | fumarate | I |
| 7 | 2-MeO-4-Cl-Ph | fumarate | II |
| 8 | 2,5-F₂-4-Cl-Ph | fumarate | I |
| 9 | 4-CF₃-Ph | fumarate | I |
| 10 | 4-CF₃-Ph | oxalate | I |
| 11 | 2-F-4-CF₃-Ph | fumarate | II |
| 12 | 3-F-4-CF₃-Ph | fumarate | II |
| 13 | 4-CF₃O-Ph | fumarate | II |
| 14 | 4-CN-Ph | (free base) | I |
| 15 | 4-F-Ph | (free base) | I |
| 16 | 3-Cl-4-F-Ph | fumarate | I |
| 17 | 4-Ph-Ph | fumarate | II |
| 18 | 4-Me-Ph | fumarate | I |
| 19 | 4-cHex-Ph | fumarate | II |
| 20 | 2-CF₃-Ph | fumarate | I |
| 21 | 2-Cl-Ph | fumarate | I |
| 22 | 2-MeO-Ph | fumarate | I |
| 23 | 3-NO₂-4-Cl-Ph | (free base) | I |
| 24 | Ph | fumarate | I |

**Table 2:**

| | | | | |
|---|---|---|---|---|
| 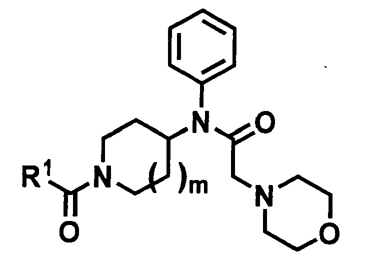 | | or a salt thereof | | |

| Ex. No. | R¹ | m | salt/melting point (°C) | production method |
|---|---|---|---|---|
| 25 | 4-Cl-Ph | 1 | fumarate | I |
| 26 | 3,4-Cl₂-Ph | 1 | fumarate | I |
| 27 | 3-F-4-Cl-Ph | 1 | fumarate | II |
| 28 | 2,5-F₂-4-Cl-Ph | 1 | fumarate | II |
| 29 | 4-CF₃-Ph | 1 | (free base) 148-150 | I |
| 30 | 4-CF₃-Ph | 1 | hydrochloride ·1/2 H₂O 203-205 | I |
| 31 | 2-F-4-CF₃-Ph | 1 | fumarate | II |
| 32 | 3-F-4-CF₃-Ph | 1 | fumarate | II |
| 33 | 3-F-4-CF₃-Ph | 1 | hydrochloride | II |
| 34 | 2,3-F₂-4-CF₃-Ph | 1 | fumarate | II |
| 35 | 4-CF₃O-Ph | 1 | fumarate | II |
| 36 | 4-CF₃O-Ph | 1 | hydrochloride 179-183 | II |
| 37 | 4-(CF₃)₂C(OH)-Ph | 1 | fumarate | II |
| 38 | 4-CN-Ph | 1 | fumarate | I |
| 39 | 4-Me-Ph | 1 | fumarate | I |
| 40 | 4-CF₃CO-Ph | 1 | 1.5 fumarate | II |
| 41 | 4-MeOCH₂-Ph | 1 | 2 fumarate | II |
| 42 | 4-CH₂F-Ph | 1 | 2 fumarate | II |
| 43 | 4-CF₃CH(Cl)-Ph | 1 | 1.2 fumarate | II |
| 44 | 4-Br-Ph | 1 | fumarate | I |
| 45 | 4-MeS-Ph | 1 | fumarate | II |
| 46 | 4-MeSO₂-Ph | 1 | (free base) | II |
| 47 | 4-MeOCO-Ph | 1 | fumarate | I |
| 48 | 4-Cl-Ph | 0 | fumarate | I |
| 49 | 4-CF₃-Ph | 0 | fumarate | I |
| 50 | 2,4-(CF₃)₂-Ph | 1 | fumarate | I |
| 51 | 4-CF₃-Ph | 2 | fumarate | I |

**Table 3:**

| | | | | | |
|---|---|---|---|---|---|
| 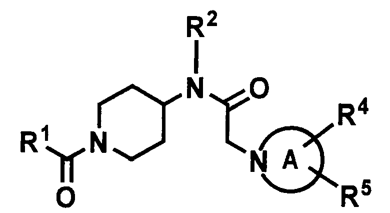 | | | or a salt thereof | | |

| Ex. No. | R¹ | R² | 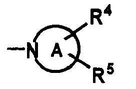 | salt | production method |
|---|---|---|---|---|---|
| 52 | 4-Cl-Ph | 4-F-Ph | 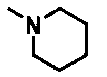 | fumarate | I |
| 53 | 4-Cl-Ph | 3-Cl-Ph | 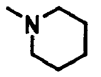 | fumarate | I |
| 54 | 4-Cl-Ph | 3-Cl-Ph | 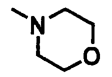 | fumarate | I |
| 55 | 4-CF₃-Ph | 4-MeO-Ph | 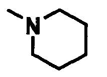 | (free base) | I |
| 56 | 4-CF₃-Ph | 4-F-Ph | 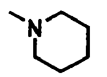 | (free base) | I |
| 57 | 4-CF₃-Ph | 3-Cl-Ph | 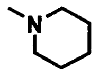 | fumarate | I |
| 58 | 4-CF₃-Ph | 4-Cl-Ph | 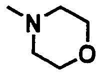 | (free base) | I |
| 59 | 4-CF₃-Ph | 3-Cl-Ph | 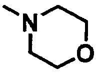 | fumarate | I |
| 60 | 4-CF₃-Ph | 4-Me-Ph | 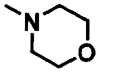 | fumarate | I |
| 61 | 4-Cl-Ph | 4-CF₃-Ph | 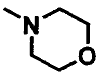 | fumarate | I |
| 62 | 4-CF₃-Ph | 2-Cl-Ph | 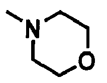 | fumarate | I |
| 63 | 4-CF₃-Ph | 2-Cl-Ph | 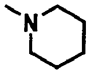 | fumarate | I |
| 64 | 4-CF₃-Ph | 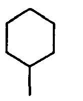 | 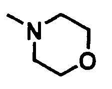 | fumarate | I |
| 65 | 4-Cl-Ph | 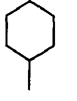 | 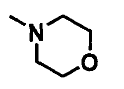 | fumarate | I |
| 66 | 4-CF₃-Ph | 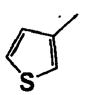 | 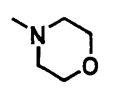 | fumarate | I |
| 67 | 4-CF₃-Ph | 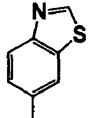 | 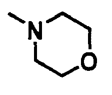 | fumarate | III |
| 68 | 4-CF₃-Ph | 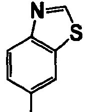 | 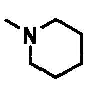 | fumarate | III |
| 69 | 4-CF₃-Ph | 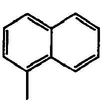 | 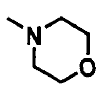 | fumarate | III |
| 70 | 4-CF₃-Ph | 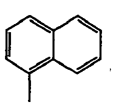 | 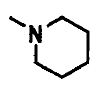 | fumarate | III |
| 71 | 4-CF₃-Ph | 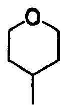 | 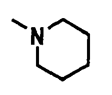 | fumarate | III |
| 72 | 4-CF₃-Ph | 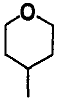 | 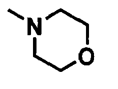 | fumarate | III |
| 73 | 4-CF₃-Ph | 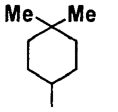 | 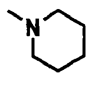 | 2.5 fumarate | III |
| 74 | 4-CF₃-Ph | 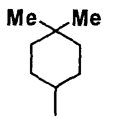 | 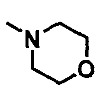 | 2 fumarate | III |
| 75 | 4-CF₃-Ph | 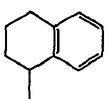 | 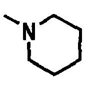 | fumarate | III |
| 76 | 4-CF₃-Ph | 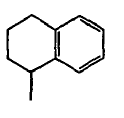 | 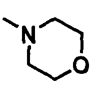 | fumarate | III |
| 77 | 4-CF₃-Ph | 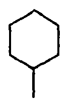 | 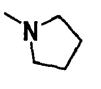 | fumarate | II |
| 78 | 4-Cl-Ph | 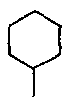 | 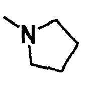 | 1.2 fumarate | I |
| 79 | 4-CF₃-Ph | 3-Me₂N-Ph | 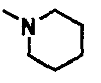 | fumarate | III |
| 80 | 4-CF₃-Ph | 3-MeO-Ph | 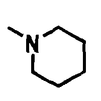 | fumarate | III |
| 81 | 4-CF₃-Ph | 3-Cl-Ph | 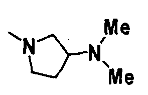 | 1.2 fumarate | III |
| 82 | 4-CF₃-Ph | 3-COOEt-Ph | 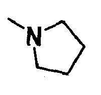 | (free base) | III |

**Table 4:**

| | | | | |
|---|---|---|---|---|
| 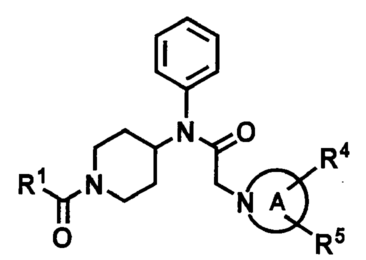 | | | or a salt thereof | |

| Ex. No. | R¹ | 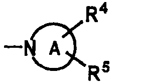 | salt | production method |
|---|---|---|---|---|
| 83 | 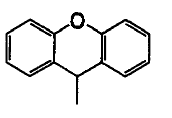 | 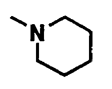 | (free base) | II |
| 84 | 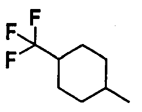 | 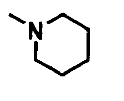 | fumarate | II |
| 85 | 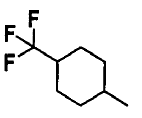 | 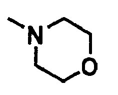 | fumarate | II |
| 86 | 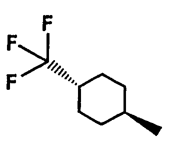 | 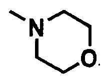 | 1.5 fumarate | II |
| 87 | 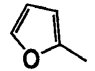 | 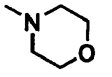 | (free base) | II |
| 88 | 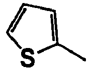 | 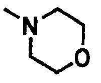 | (free base) | II |
| 89 | 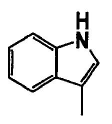 | 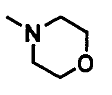 | fumarate | II |
| 90 | 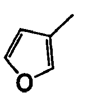 | 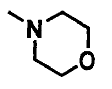 | (free base) | II |
| 91 | 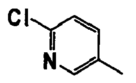 | 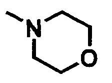 | (free base) | II |
| 92 | 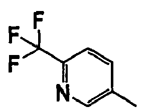 | 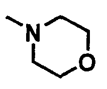 | (free base) | II |
| 93 | 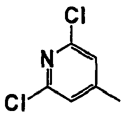 | 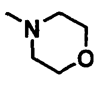 | fumarate | II |
| 94 | 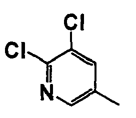 | 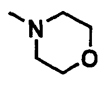 | fumarate | II |
| 95 | 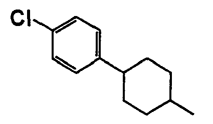 | 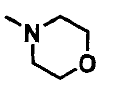 | (free base) | II |
| 96 | 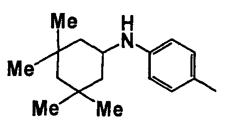 | 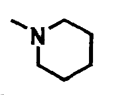 | (free base) | II |
| 97 | 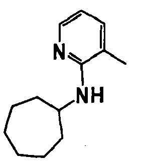 | 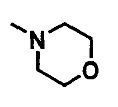 | oxalate | II |
| 98 | 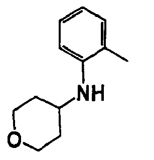 | 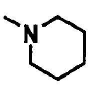 | fumarate | II |
| 99 | 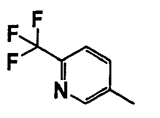 | 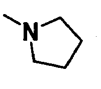 | fumarate | II |
| 100 | 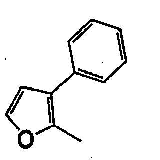 | 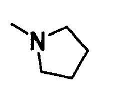 | (free base) | II |
| 101 | 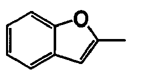 | 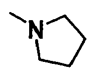 | fumarate | II |

**Table 5:**

| | | | | |
|---|---|---|---|---|
| 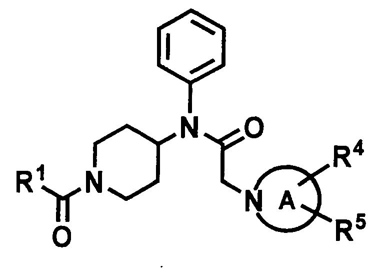 | | | or a salt thereof | |

| Ex. No. | R¹ | 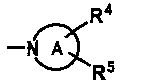 | salt | production method |
|---|---|---|---|---|
| 102 | 4-Cl-Ph | 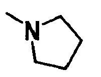 | fumarate | I |
| 103 | 4-CF₃-Ph | 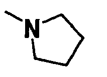 | fumarate | I |
| 104 | 3,4-Cl₂-Ph | 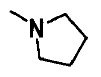 | fumarate | I |
| 105 | 3-F-4-Cl-Ph | 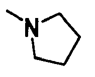 | fumarate | II |
| 106 | 4-CF₃-Ph | 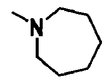 | (free base) | I |
| 107 | 4-Cl-Ph | 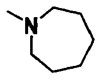 | (free base) | I |
| 108 | 4-CF₃-Ph | 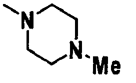 | fumarate | I |
| 109 | 3-CF₃-Ph | 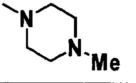 | fumarate | I |
| 110 | 2-CF₃-Ph | 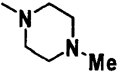 | fumarate | I |
| 111 | 4-CF₃-Ph | 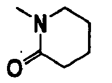 | (free base) | I |
| 112 | 4-Cl-Ph | 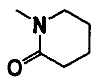 | (free base) | I |
| 113 | 4-CF₃-Ph | 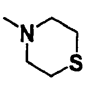 | fumarate | III |
| 114 | 4-CF₃-Ph | 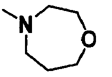 | fumarate | III |
| 115 | 4-CF₃-Ph | 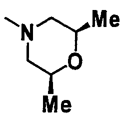 | fumarate | III |
| 116 | 4-CF₃-Ph | 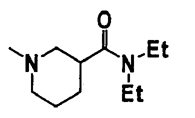 | fumarate | III |
| 117 | 4-CF₃-Ph | 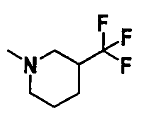 | (free base) | III |
| 118 | 4-CF₃-Ph | 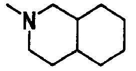 | 1.3 fumarate | III |
| 119 | 4-CF₃-Ph | 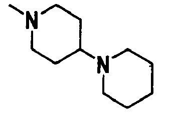 | 2 fumarate | III |
| 120 | 4-CF₃-Ph | 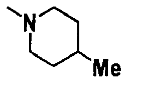 | fumarate | III |
| 121 | 4-CF₃-Ph | 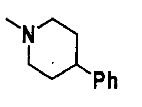 | fumarate | III |
| 122 | 4-CF₃-Ph | 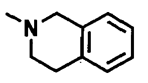 | fumarate | III |
| 123 | 4-CF₃-Ph | 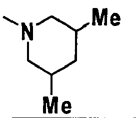 | 1.3 fumarate | III |
| 124 | 4-CF₃-Ph | 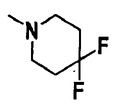 | (free base) | III |
| 125 | 4-CF₃-Ph | 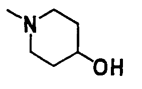 | fumarate | III |
| 126 | 4-CF₃-Ph | 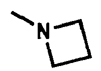 | fumarate | III |
| 127 | 4-CF₃-Ph | 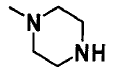 | fumarate | III |
| 128 | 4-CF₃-Ph | 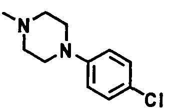 | fumarate | III |
| 129 | 4-CF₃-Ph | 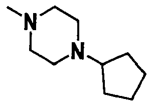 | fumarate | III |
| 130 | 4-CF₃-Ph | 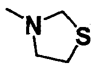 | fumarate | III |
| 131 | 4-CF₃-Ph | 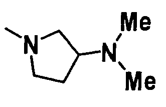 | fumarate | III |
| 132 | 4-CF₃-Ph | 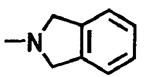 | fumarate | III |
| 133 | 4-CF₃-Ph | 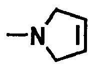 | fumarate | III |
| 134 | 4-CF₃-Ph | 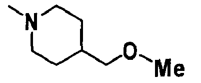 | fumarate | III |
| 135 | 4-CF₃-Ph | 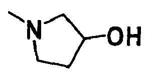 | fumarate | III |
| 136 | 4-CF₃-Ph | 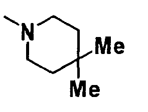 | fumarate | III |
| 137 | 4-CF₃-Ph | 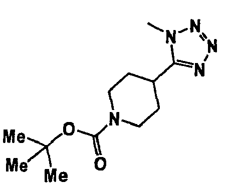 | 1.4 fumarate | III |
| 138 | 4-CF₃-Ph | 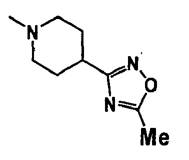 | fumarate | III |
| 139 | 4-CF₃-Ph | 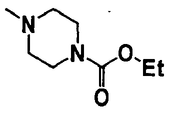 | fumarate | III |
| 140 | 4-CF₃-Ph | 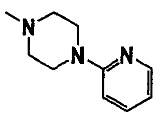 | fumarate | III |
| 141 | 4-CF₃-Ph | 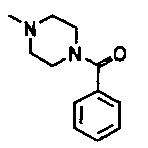 | fumarate | III |
| 142 | 4-CF₃-Ph | 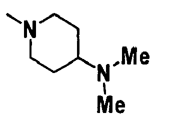 | fumarate | III |
| 143 | 4-CF₃-Ph | 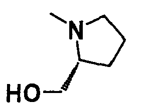 | fumarate | III |
| 144 | 4-CF₃-Ph | 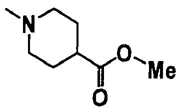 | (free base) | III |
| 145 | 4-CF₃-Ph | 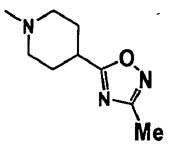 | fumarate | III |
| 146 | 4-CF₃-Ph | 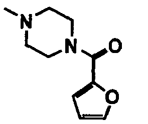 | fumarate | III |
| 147 | 4-CF₃-Ph | 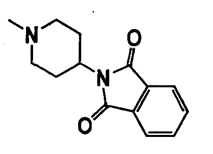 | 2 hydrochlor ide | III |

**Table 6:**

| | | | | | |
|---|---|---|---|---|---|
| 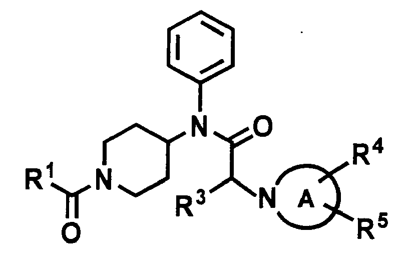 | | | | or a salt thereof | |

| Ex. No. | R¹ | R³ | 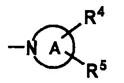 | salt | production method |
|---|---|---|---|---|---|
| 148 | 4-CF₃-Ph | Ph | 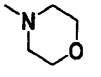 | 2 fumarate | III |
| 149 | 4-CF₃-Ph | Me | 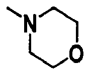 | fumarate | III |
| 150 | 4-CF₃-Ph | Ph | 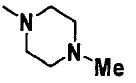 | 2 hydrochloride | III |
| 151 | 4-CF₃-Ph | i-Pr | 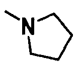 | 2 hydrochloride | II |
| 152 | 4-CF₃-Ph | i-Pr | 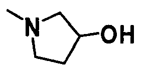 | hydrochloride | II |
| 153 | 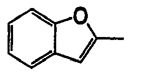 | i-Pr | 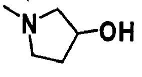 | hydrochloride | II |

**Table 7:**

| | | | | |
|---|---|---|---|---|
| 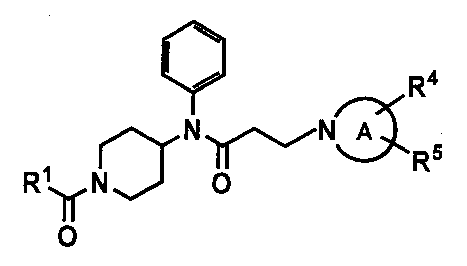 | | | or a salt thereof | |

| Ex. No. | R¹ | 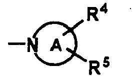 | salt | production method |
|---|---|---|---|---|
| 154 | 4-CF₃-Ph | 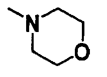 | fumarate | I |
| 155 | 4-CF₃-Ph | 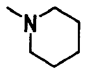 | (free base) | I |
| 156 | 4-Cl-Ph | 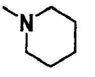 | (free base) | I |
| 157 | 4-CF₃-Ph | 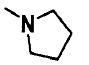 | 1.2 fumarate | I |
| 158 | 4-Cl-Ph | 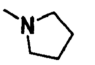 | 1.3 fumarate | I |
| 159 | 4-CF₃-Ph | 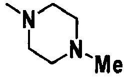 | 1.2 fumarate | III |
| 160 | 3,4-Cl₂-Ph | 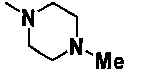 | fumarate | I |
| 161 | 4-CF₃-Ph | 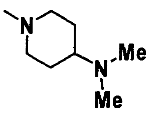 | 1.1 fumarate | III |
| 162 | 4-CF₃-2,3-F₂-Ph | 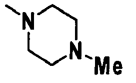 | 2 fumarate | II |
| 163 | 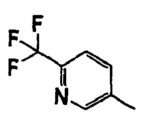 | 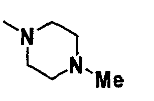 | 1.25 fumarate | II |
| 164 | 4-MeO-Ph | 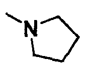 | fumarate | II |
| 165 | 4-CF₃-Ph | 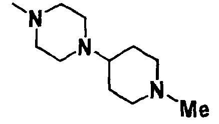 | 2.2 fumarate | II |
| 166 | 4-CF₃-Ph | 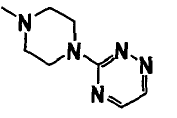 | fumarate | III |
| 167 | 4-CF₃-Ph | 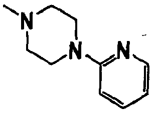 | fumarate | III |
| 168 | 4-CF₃-Ph | 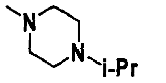 | fumarate | III |
| 169 | 4-CF₃-Ph | 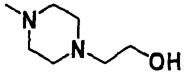 | fumarate | III |
| 170 | 4-CF₃-Ph | 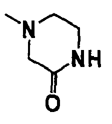 | 1.1 fumarate | III |
| 171 | 4-CF₃-Ph | 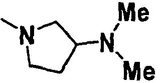 | 2.3 fumarate | II |
| 172 | 3-CF₃-Ph | 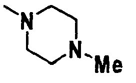 | 2 hydrochloride | II |
| 173 | 2-CF₃-Ph | 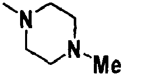 | 2 hydrochloride | II |
| 174 | 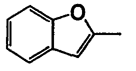 | 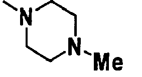 | 2 hydrochloride | II |
| 175 | 4-CF₃-Ph | 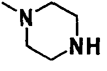 | 2 hydrochloride | III |
| 176 | 4-CF₃-3-F-Ph | 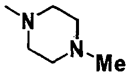 | 2 hydrochloride | II |
| 177 | 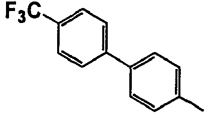 | 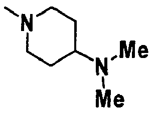 | 1.4 fumarate | II |
| 178 | 3,4-Cl₂-Ph | 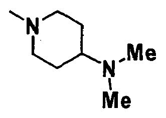 | 1.3 fumarate | I |
| 179 | 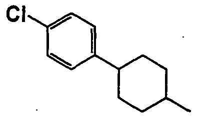 | 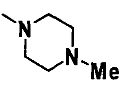 | 2 hydrochloride | II |
| 180 | 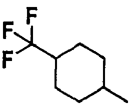 | 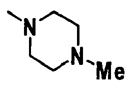 | 2 hydrochloride | II |
| 181 | 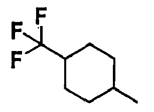 | 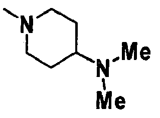 | fumarate | II |

**Table 8:**

| | | | | | | |
|---|---|---|---|---|---|---|
| 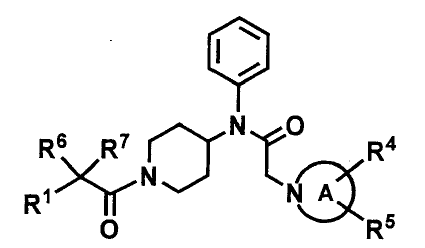 | | | | or a salt thereof | | |

| Ex. No. | R¹ | R⁶ | R⁷ | 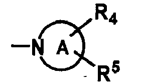 | salt | production method |
|---|---|---|---|---|---|---|
| 182 | 4-Cl-Ph | H | H | 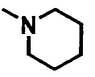 | (free base) | II |
| 183 | 4-Cl-Ph | H | H | 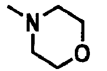 | fumarate | II |
| 184 | 4-CF₃-Ph | H | H | 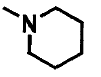 | fumarate | II |
| 185 | 4-CF₃-Ph | H | H | 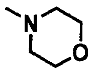 | fumarate | II |
| 186 | 3,4-Cl₂-Ph | H | H | 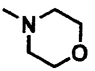 | fumarate | II |
| 187 | 4-Me₂N-Ph | H | H | 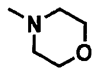 | fumarate | II |
| 188 | 4-CF₃O-Ph | H | H | 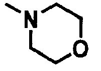 | fumarate | II |
| 189 | 4-Ph-Ph | H | H | 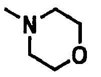 | fumarate | II |
| 190 | 4-Cl-Ph | Me | Me | 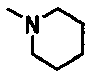 | (free base) | II |
| 191 | 2-F-Ph | Me | Me | 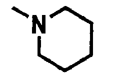 | (free base) | II |
| 192 | Ph | Ph | H | 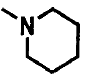 | (free base) | II |
| 193 | Ph | Ph | OH | 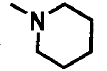 | (free base) | II |
| 194 | cHex | Ph | H | 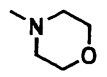 | 2 fumarate | II |
| 195 | 4-Cl-Ph | Me | Me | 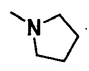 | (free base) | II |
| 196 | cHex | Ph | H | 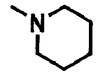 | 1.5 fumarate | II |
| 197 | 4-Cl-Ph | i-Pr | H | 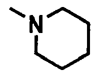 | (free base) | I |

**Table 9:**

| | | | | |
|---|---|---|---|---|
| 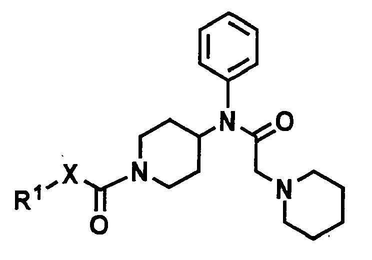 | | | or a salt thereof | |

| Ex. No. | R¹ | X | salt | production method |
|---|---|---|---|---|
| 198 | 4-Cl-Ph | 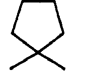 | (free base) | II |
| 199 | 3-CF₃-Ph | 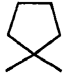 | (free base) | II |
| 200 | 4-F-Ph | 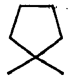 | fumarate | II |
| 201 | 3-F-Ph | 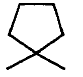 | (free base) | II |
| 202 | 2-F-Ph | 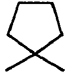 | (free base) | II |
| 203 | Ph | 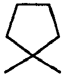 | (free base) | II |
| 204 | 4-MeO-Ph | 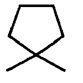 | fumarate | II |
| 205 | Ph | 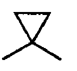 | (free base) | II |
| 206 | 4-Cl-Ph | 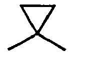 | (free base) | II |
| 207 | 4-MeO-Ph | 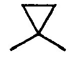 | fumarate | II |
| 208 | 4-Me-Ph | 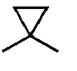 | (free base) | II |
| 209 | 4-Cl-Ph | 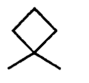 | (free base) | II |
| 210 | 4-Cl-Ph | 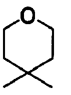 | (free base) | II |
| 211 | 4-CF₃-Ph | 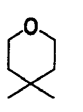 | (free base) | II |

**Table 10:**

| | | | | | |
|---|---|---|---|---|---|
| 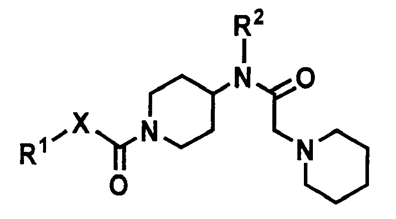 | | | or a salt thereof | | |

| Ex. No. | R¹ | X | R² | salt | production method |
|---|---|---|---|---|---|
| 212 | 4-Cl-Ph | 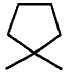 | 4-F-Ph | (free base) | II |
| 213 | 2-F-Ph | 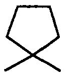 | 4-F-Ph | (free base) | II |
| 214 | 4-Cl-Ph | 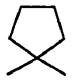 | 4-MeO-Ph | fumarate | II |
| 215 | 3-CF₃-Ph | 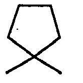 | 4-MeO-Ph | 2.5 fumarate | II |
| 216 | 3-CF₃-Ph | 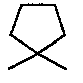 | 4-F-Ph | 2.5 fumarate | II |
| 217 | 4-CF₃-Ph | 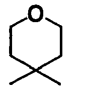 | 4-MeO-Ph | (free base) | II |
| 218 | 4-CF₃-Ph | 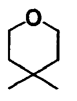 | 4-F-Ph | (free base) | II |
| 219 | 2-F-Ph | 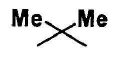 | 4-MeO-Ph | (free base) | II |
| 220 | 4-Cl-Ph | 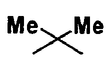 | 4-MeO-Ph | (free base) | II |

**Table 11:**

| | | | | | | |
|---|---|---|---|---|---|---|
| 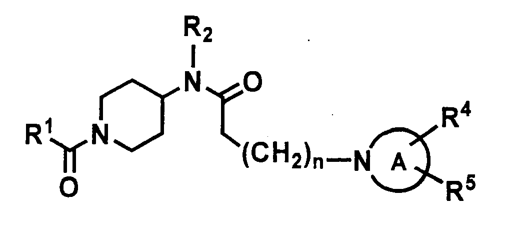 | | | | | | |

| Ex. No. | R¹ | R² | n | 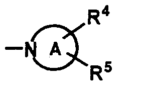 | salt | production method |
|---|---|---|---|---|---|---|
| 221 | 4-CF₃-Ph | 3-MeO-Ph | 1 | 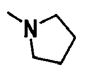 | fumarate | III |
| 222 | 4-CF₃-Ph | 3-F-Ph | 1 | 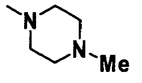 | 1.1 fumarate | III |
| 223 | 4-CF₃-Ph | 3-Cl-Ph | 1 | 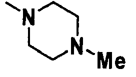 | 2 hydrochloride | III |
| 224 | 4-CF₃-Ph | Ph | 2 | 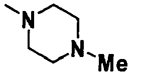 | 2 hydrochloride | III |

### Example 225: N-phenyl-2-[4-(pyrrolidin-1-ylcarbonyl)piperazin-1-yl]-N-[1-(4-trifluoromethylbenzoyl)piperidin-4-yl]acetamide

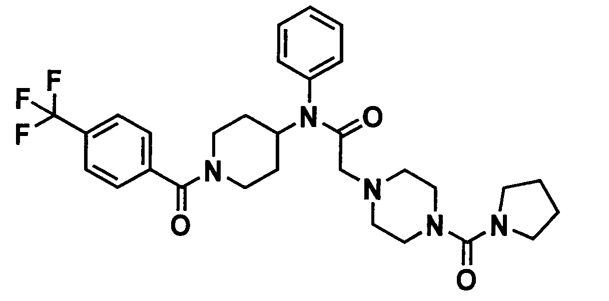

To a solution of 2-(piperazin-1-yl)-N-phenyl-N-[1-(4-trifluoromethylbenzoyl)piperidin-4-yl]acetamide (compound of Example 127) (100 mg) in acetonitrile (5 ml) were added triethylamine (59 µL) and 1-pyrrolidinecarbonyl chloride (36.6 mg) and the mixture was stirred at 40°C for 3 hr. Water (10 ml) was added to the reaction mixture, and the mixture was extracted with ethyl acetate (10 ml×3). The organic layer was washed with saturated brine (30 ml), dried over anhydrous magnesium sulfate, and concentrated under reduced pressure. The residue was purified by basic silica gel column chromatography (elution solvent: chloroform/methanol) to give the object product (117.9 mg, yield 98%) as a white solid.

### Example 226: 2-(4-dimethylaminocarbonylpiperazin-1-yl)-N-phenyl-N-[1-(4-trifluoromethylbenzoyl)piperidin-4-yl]acetamide

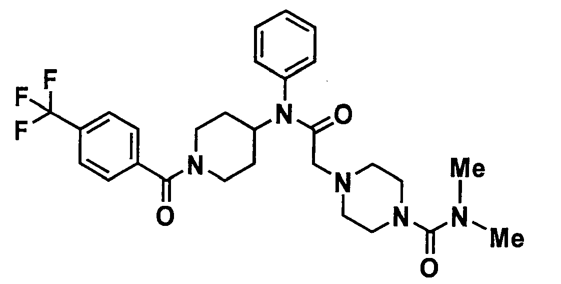

Using the corresponding starting material and by the treatment in the same manner as in Example 225, the object product (105 mg, yield 85%) was obtained.

### Example 227: N-(3-carboxylphenyl)-2-(pyrrolidin-1-yl)-N-[1-(4-trifluoromethylbenzoyl)piperidin-4-yl]acetamide

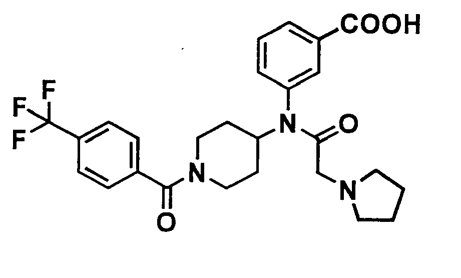

To a solution of N-(3-ethoxycarbonylphenyl)-2-(pyrrolidin-1-yl)-N-[1-(4-trifluoromethylbenzoyl)piperidin-4-yl]acetamide (compound of Example 82) (158 mg) in tetrahydrofuran (10 ml) was added 1 mol/L aqueous sodium hydroxide solution (0.44 ml) and the mixture was stirred at room temperature for 2.5 hr. The reaction mixture was acidified with 1 mol/L aqueous hydrochloric acid solution, and the mixture was extracted with chloroform (50 ml×3). The organic layer was dried over anhydrous sodium sulfate, and concentrated under reduced pressure to give colorless crystals (45 mg). A solution of 4 mol/L hydrochloric acid in ethyl acetate (5 ml) was added thereto and the mixture was concentrated under reduced pressure to give the object product as colorless crystals (30 mg, yield 19%).

### Example 228: 2-(4-aminopiperidin-1-yl)-N-phenyl-N-[1-(4-trifluoromethylbenzoyl)piperidin-4-yl]acetamide

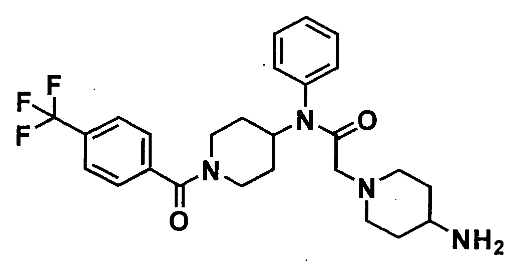

A solution of 2-(4-phthalimidopiperidin-1-yl)-N-phenyl-N-[1-(4-trifluoromethylbenzoyl)piperidin-4-yl]acetamide (compound of Example 147) (200 mg) in ethanol (5 ml) was heated to 70°C, hydrazine monohydrate (47 µL) was added dropwise and the mixture was stirred for 4 hr. After cooling to room temperature, aqueous sodium hydroxide solution was added to dissolve a white solid, and the mixture was extracted with chloroform. After washing with saturated brine, the mixture was dried over anhydrous sodium sulfate, and concentrated under reduced pressure. The residue was purified by basic silica gel column chromatography (elution solvent: chloroform/methanol) to give the object product (171.7 mg, yield 100%) as an oil. The oil was dissolved in ethyl acetate, a solution of 4 mol/L hydrochloric acid in ethyl acetate was added and the mixture was concentrated under reduced pressure to give an amorphous form of the object product (171.7 mg).

### Example 229: N-phenyl-2-[5-(piperidin-4-yl)tetrazol-1-yl]-N-[1-(4-trifluoromethylbenzoyl)piperidin-4-yl]acetamide

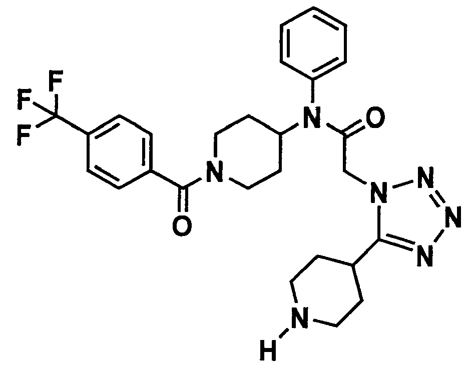

To a solution of 2-[5-(1-t-butoxycarbonylpiperidin-4-yl)tetrazol-1-yl]-N-phenyl-N-[1-(4-trifluoromethylbenzoyl)piperidin-4-yl]acetamide (compound of Example 137) (110 mg) in methanol (1 ml) was added 4 mol/L hydrochloric acid in 1,4-dioxane (0.13 ml) and the mixture was stirred for 12 hr. Aqueous sodium hydroxide solution was added, and the mixture was extracted with chloroform, washed with saturated brine, dried over anhydrous sodium sulfate, and concentrated under reduced pressure to give the object product (85.6 mg, yield 92%) as an oil. This product and fumaric acid (18.3 mg) were dissolved in isopropanol and the mixture was concentrated under reduced pressure to give an amorphous form of the object product (80.0 mg).

### Example 230: 3-methyl-2-(piperazin-1-yl)-N-phenyl-N-[1-(4-trifluoromethylbenzoyl)piperidin-4-yl]butylamide

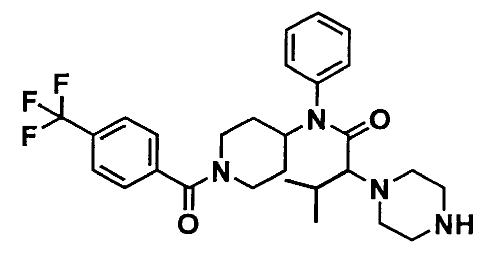

To a solution of 3-methyl-2-[1-benzyloxycarbonylpiperazin-4-yl)-N-phenyl-N-[1-(4-trifluoromethylbenzoyl)piperidin-4-yl]butylamide (402 mg), obtained by reacting 2-amino-3-methyl-N-phenyl-N-[1-(4-trifluoromethylbenzoyl)piperidin-4-yl]butylamide obtained in Example 4, step 2, with the corresponding dibrominated form, in methanol (10 ml) was added palladium hydroxide (50 mg), and the mixture was stirred under a hydrogen atmosphere for 15 hr. The reaction mixture was filtered, and the filtrate was concentrated under reduced pressure. The residue was purified by basic silica gel column chromatography (elution solvent: chloroform/methanol), a solution of 4 mol/L hydrochloric acid in ethyl acetate was added and the mixture was concentrated under reduced pressure to give the object product (57.4 mg, yield 21%).

The NMR data of the above-mentioned Example compounds are shown in the following Table 12.

Table 12:
conditions: 1.300MHz, DMSO-d₆
2.300MHz, CDCl₃
3.400MHz, DMSO-d₆
4.400MHz, CDCl₃
5.300MHz, CD₃OD
6.400MHz, CD₃OD

| Ex. | conditions | ¹H-NMR data |
|---|---|---|
| 1 | 2 | 1.20-1.63 (m, 8H), 1.80-1.90 (m, 2H), 2.30-2.40 (m, 4H), 2.72-3.11 (m, 4H), 3.70-3.80 (m, 1H), 4.80-4.90 (m, 2H), 7.00-7.18 (m, 2H), 7.22-7.43 (m, 7H) |
| 2 | 1 | 1.01-1.42 (m, 8H), 1.72-1.90 (m, 2H), 2.32-2.40 (m, 4H), 2.75-2.85 (m, 3H), 3.10-3.20 (m, 1H), 3.45-3.55 (m, 1H), 4.40-4.50 (m, 1H), 4.61-4.67 (m, 1H), 6.58 (s, 2H), 7.12-7.22 (m, 3H), 7.37-7.47 (m, 4H), 7.59-7.63 (m, 1H) |
| 3 | 2 | 1.23 (t, 3H, J=7.1Hz), 1.30-1.50 (m, 2H), 1.65-2.23 (m, 7H), 2.76-2.90 (m, 5H), 3.10-3.20 (m, 1H), 3.60-3.70 (m, 1H), 4.10 (q, 2H, J=7.1Hz), 4.81-4.91 (m, 2H), 7.00-7.20 (m, 2H), 7.39-7.44 (m, 5H), 7.63 (brd, 2H, J=7.9Hz) |
| 5 | 2 | 1.20-1.56 (m, 8H), 1.80-1.90 (m, 2H), 2.32 (brs, 4H), 2.73-2.85 (m, 3H), 3.10-3.20 (m, 1H), 3.60-3.70 (m, 1H), 4.65-4.89 (m, 2H), 7.05-7.14 (m, 3H), 7.41-7.45 (m, 5H) |
| 6 | 2 | 1.15-1.48 (m, 8H), 1.65-1.70 (m, 1H), 1.86-1.90 (m, 1H), 2.37 (brs, 4H), 2.84-2.91 (m, 3H), 3.12-3.20 (m, 2H), 4.47-4.52 (m, 1H), 4.66-4.74 (m, 1H), 6.58 (s, 2H), 7.19 (brs, 2H), 7.43 (brs, 3H), 7.69-7.75 (m, 2H) |
| 7 | 1 | 0.98-1.43 (m, 8H), 1.60-1.89 (m, 2H), 2.35 (brs, 4H), 2.74-2.82 (m, 3H), 2.97-3.33 (m, 2H), 3.43 (s, 1.5H), 3.78 (s, 1.5H), 4.47-4.69 (m, 2H), 6.57 (s, 2H), 6.98-7.50 (m, 8H) |
| 8 | 1 | 1.08-1.43 (m, 8H), 1.69-1.74 (m, 1H), 1.84-1.89 (m, 1H), 2.34 (brs, 4H), 2.79-2.88 (m, 3H), 3.11-3.20 (m, 1H), 3.37-3.42 (m, 1H), 4.47-4.52 (m, 1H), 4.62-4.70 (m, 1H), 6.58 (s, 2H), 7.20 (brs, 2H), 7.44-7.52 (m, 4H), 7.69-7.74 (m, 1H) |
| 9 | 1 | 1.08-1.46 (m, 8H), 1.70-1.91 (m, 2H), 2.33 (brs, 4H), 2.78 (brs, 2H), 2.85-2.95 (m, 1H), 3.10-3.25 (m, 1H), 3.35-3.56 (m, 1H), 4.50-4.67 (m, 2H), 6.60 (s, 2H), 7.22 (brs, 2H), 7.40-7.51 (m, 5H), 7.77 (brd, 2H, J=7.9Hz) |
| 11 | 1 | 1.08-1.42 (m, 8H), 1.72 (brd, 1H, J=11.7Hz), 1.88 (brd, 1H, J=12.3Hz), 2.35 (brs, 4H), 2.79-2.91 (m, 3H), 3.13-3.35 (m, 2H), 4.53 (brd, 1H, J=13.6Hz), 4.63-4.71 (m, 1H), 6.58 (s, 2H), 7.22 (brs, 2H), 7.44-7.64 (m, 5H), 7.77 (d, 1H, J=9.5Hz) |
| 12 | 1 | 1.14-1.44 (m, 6H), 1.69-1.90 (m, 2H), 2.41 (brs, 4H), 2.80-2.86 (m, 3H), 3.12-3.21 (m, 1H), 3.43-3.48 (m, 1H), 4.47-4.69 (m, 2H), 6.58 (s, 2H), 7.21-7.31 (m, 3H), 7.44-7.52 (m, 4H), 7.81 (t, 1H, J=7.7Hz) |
| 13 | 3 | 1.10-1.42 (m, 8H), 1.70-1.90 (m, 2H), 2.33 (brs, 4H), 2.75-2.90 (m, 3H), 3.09-3.20 (m 1H), 3.40-3.60 (m, 1H), 4.40-4.60 (m, 1H), 4.62-4.66 (m, 1H), 6.58 (s, 2H), 7.22 (brs, 2H), 7.36-7.46 (m, 7H) |
| 14 | 2 | 1.14-1.93 (m, 10H), 2.32 (brs, 4H), 2.73 (s, 2H), 2.80-2.90 (m, 1H), 3.10-3.25 (m, 1H), 3.55-3.65 (m, 1H), 4.72-4.91 (m, 2H), 7.08 (brs, 2H), 7.38-7.43 (m, 5H), 7.65-7.68 (m, 2H) |
| 15 | 2 | 1.20-1.39 (m, 4H), 1.49-1.57 (m, 4H), 1.80-1.90 (m, 2H), 2.30-2.34 (m, 4H), 2.73 (s, 2H), 2.80-3.20 (m, 2H), 2.85-3.20 (m, 2H), 3.70-3.90 (m, 1H), 4.70-4.91 (m, 2H), 7.00-7.08 (m, 4H), 7.27-7.34 (m, 2H), 7.41-7.44 (m, 3H) |
| 16 | 1 | 1.06-1.46 (m, 8H), 1.70-1.80 (m, 2H), 2.25-2.35 (m, 4H), 2.70-2.85 (m, 3H), 3.10-3.20 (m, 1H), 3.50-3.60 (m, 1H), 4.40-4.64 (m, 2H), 6.58 (s, 2H), 7.19-7.33 (m, 3H), 7.39-7.56 (m, 6H) |
| 17 | 1 | 1.05-1.42 (m, 8H), 1.80-1.90 (m, 2H), 2.25-2.35 (m, 4H), 2.76 (m, 2H), 3.10-3.50 (m, 4H), 4.60-4.76 (m, 1H), 6.60 (s, 2H), 7.22-7.25 (m, 2H), 7.35-7.50 (m, 8H), 7.66-7.70 (m, 4H) |
| 18 | 1 | 1.03-1.44 (m, 8H), 1.65-1.80 (m, 2H), 2.29 (s, 3H), 2.38-2.40 (m, 4H), 2.80-2.86 (m, 3H), 3.00-3.08 (m, 1H), 3.50-3.65 (m, 1H), 4.40-4.68 (m, 2H), 6.58 (s, 3H), 7.12-7.22 (m, 6H), 7.44-7.49 (m, 3H) |
| 19 | 1 | 1.01-1.42 (m, 14H), 1.66-1.77 (m, 7H), 2.25-2.38 (m, 4H), 2.48-2.49 (m, 2H), 2.78 (s, 2H), 3.60-3.70 (m, 1H), 4.44-4.65 (m, 2H), 6.58 (s, 2H), 7.14-7.24 (m, 6H), 7.43-7.46 (m, 3H) |
| 20 | 1 | 1.04-1.42 (m, 8H), 1.60-1.86 (m, 2H), 2.25-2.30 (m, 4H), 2.76-3.18 (m, 5H), 4.50-4.68 (m, 2H), 6.60 (s, 2H), 7.15-7.80 (m, 9H) |
| 21 | 1 | 1.03-1.42 (m, 8H), 1.60-1.70 (m, 1H), 1.83-1.85 (m, 1H), 2.32 (brs, 4H), 2.77-2.86 (m, 3H), 3.10-3.18 (m, 2H), 4.50-4.69 (m, 2H), 6.58 (s, 2H), 7.17-7.49 (m, 9H) |
| 22 | 1 | 1.03-1.63 (m, 9H), 1.80-1.84 (m, 1H), 2.30-2.35 (m, 4H), 2.76-2.82 (m, 3H), 2.88-3.24 (m, 2H), 3.76 (s, 3H), 4.49-4.69 (m, 2H), 6.58 (s, 2H), 6.90-7.07 (m, 3H), 7.21-7.47 (m, 6H) |
| 23 | 2 | 1.20-1.57 (m, 8H), 1.90-2.00 (m, 2H), 2.30-2.34 (m, 4H), 2.74 (s, 2H), 2.85-3.00 (m, 1H), 3.20-3.30 (m, 1H), 3.60-3.70 (m, 1H), 4.70--4.93 (m, 2H), 7.08 (brs, 2H), 7.42-7.48 (m, 4H), 7.57 (d, 1H, J=8.3Hz), 7.83 (d, 1H, J=1.8Hz) |
| 24 | 1 | 1.03-1.45 (m, 8H), 1.70-1.80 (m, 2H), 2.38 (brs, 4H), 2.80-2.90 (m, 3H), 3.10-3.20 (m, 1H), 3.50-3.60 (m, 1H), 4.50-4.71 (m, 2H), 6.59 (s, 2H), 7.24-7.48 (m, 10H) |
| 25 | 1 | 1.03-1.20 (m, 2H), 1.60-1.85 (m, 2H), 2.26 (brs, 4H), 2.69-2.80 (m, 3H), 3.05-3.60 (m, 6H), 4.25-4.70 (m, 2H), 6.61 (s, 2H), 7.21-7.30 (m, 4H), 7.43-7.46 (m, 5H) |
| 26 | 1 | 1.03-1.16 (m, 2H), 1.70-1.90 (m, 2H), 2.25-2.28 (m, 4H), 2.69-2.90 (m, 3H), 3.17-3.50 (m, 6H), 4.35-4.70 (m, 2H), 6.60 (s, 2H), 7.20-7.28 (m, 3H), 7.43-7.48 (m, 3H), 7.57 (d, 1H, J=2.0Hz), 7.65 (d, 1H, J=8.3Hz) |
| 27 | 3 | 1.32-1.22 (m, 2H), 1.62-1.85 (m, 2H), 2.27 (brs, 4H), 2.69-2.85 (m, 3H), 3.10-3.60 (m, 6H), 4.40-4.50 (m, 1H), 4.60-4.70 (m, 1H), 6.61 (s, 2H), 7.13 (d, 1H, J=8.3Hz), 7.23 (brs, 2H), 7.37-7.46 (m, 4H), 7.61 (t, 1H, J=7.9Hz) |
| 28 | 1 | 1.03-1.23 (m, 2H), 1.71 (brd, 1H, J=12.5Hz), 1.86 (brd, 1H, J=11.7Hz), 2.20-2.40 (m, 4H), 2.72 (s, 2H), 2.84 (brt, 1H, J=11.9Hz), 3.15 (brt, 1H, J=12.5Hz), 3.36-3.47 (m, 5H), 4.49 (brd, 1H, J=12.8Hz), 4.62-4.71 (m, 1H), 6.61 (s, 4H), 7.22 (brs, 2H), 7.43-7.53 (m, 4H), 7.69-7.74 (m, 1H) |
| 29 | 2 | 1.12-1.20 (m, 2H), 1.70-1.91 (m, 2H), 2.28 (brs, 4H), 2.71 (brs, 2H), 2.79-2.89 (m, 1H), 3.17-3.22 (m, 1H), 3.47-3.62 (m, 5H), 4.49-4.54 (m, 1H), 4.63-4.72 (m, 1H), 6.64 (brs, 2H), 7.24 (brs, 2H), 7.41-7.51 (m, 5H), 7.77 (brd, 2H, J=8.3Hz) |
| 31 | 1 | 1.03-1.22 (m, 2H), 1.72 (brd, 1H, J=11.7Hz), 1.88 (brd, 1H, J=12.3Hz), 2.27 (brs, 4H), 2.70 (s, 2H), 2.87 (brt, 1H, J=11.9Hz), 3.17-3.48 (m, 6H), 4.52 (brd, 1H, J=13.0Hz), 4.63-4.72 (m, 1H), 6.61 (s, 2H), 7.22 (brs, 2H), 7.44-7.64 (m, 5H), 7.77 (d, 1H, J=9.4Hz) |
| 33 | 1 | 1.13-1.24 (m, 2H), 1.72-1.97 (m, 2H), 2.82-2.91 (m, 1H), 3.00-3.55 (m, 7H), 3.62-3.84 (m, 5H), 4.49-4.69 (m, 2H), 7.29-7.32 (m, 3H), 7.49-7.53 (m, 4H), 7.82 (t, 1H, J=7.8Hz) |
| 34 | 1 | 1.03-1.18 (m, 2H), 1.71 (brd, 1H, J=12.0Hz), 1.89 (brd, 1H, J=12.0Hz), 2.26 (brs, 4H), 2.69 (s, 2H), 2.88 (brt, 1H, J=12.0Hz), 3.10-3.48 (m, 6H), 4.51 (brd, 1H, J=13.2Hz), 4.60-4.75 (m, 1H), 6.61 (s, 2H), 7.22-7.46 (m, 6H), 7.61-7.62 (m, 1H) |
| 35 | 3 | 1.04-1.26 (m, 2H), 1.65-1.90 (m, 2H), 2.27 (brs, 4H), 2.69 (s, 2H), 2.80-2.85 (m 1H), 3.10-3.20 (m, 1H), 3.45-3.57 (m, 5H), 4.40-4.55 (m, 1H), 4.60-4.70 (m, 1H), 6.61 (s, 2H), 7.23 (brs, 2H), 7.36-7.46 (m, 7H) |
| 37 | 1 | 1.05-1.20 (m, 2H), 1.60-1.90 (m, 2H), 2.20-2.30 (m, 4H), 2.68 (s, 2H), 2.75-2.90 (m, 1H), 3.10-3.60 (m, 6H), 4.40-4.75 (m, 2H), 6.59 (s, 2H), 7.20-7.35 (m, 2H), 7.40-7.51 (m, 5H), 7.69 (brd, 2H, J=8.2Hz) |
| 38 | 3 | 1.03-1.20 (m, 2H), 1.65-1.85 (m, 2H), 2.27 (brs, 4H), 2.69 (s, 2H), 2.80-2.90 (m, 1H), 3.15-3.3.46 (m, 6H), 4.47-4.68 (m, 2H), 6.60 (s, 2H), 7.22 (brs, 2H), 7.35-7.50 (m, 5H), 7.86 (d, 2H, J=4.3Hz) |
| 39 | 3 | 1.09-1.17 (m, 2H), 1.70-1.85 (m, 2H), 2.20-2.32 (m, 7H), 2.66 (s, 2H), 2.70-3.20 (m, 2H), 3.40-3.62 (m, 5H), 4.40-4.65 (m, 2H), 6.60 (s, 2H), 7.12-7.23 (m, 5H), 7.86 (d, 2H, J=4.3H), 7.12-7.23 (m, 6H), 7.42-7.47 (m, 3H) |
| 40 | 1 | 1.05-1.20 (m, 2H), 1.60-1.90 (m, 2H), 2.25-2.78 (m, 4H), 2.71 (s, 2H), 2.70-2.90 (m, 1H), 3.15-3.47 (m, 6H), 4.48-4.67 (m, 2H), 6.61 (s, 2H), 7.21-7.31 (m, 2H), 7.44-7.61 (m, 5H), 8.05 (d, 2H, J=8.1Hz) |
| 41 | 1 | 1.03-1.22 (m, 2H), 1.60-1.90 (m, 2H), 2.34 (brs, 4H), 2.80-2.90 (m, 3H), 3.19-3.49 (m, 8H), 4.40-4.49 (m, 4H), 4.60-4.70 (m, 1H), 6.61 (s, 4H), 7.21-7.47 (m, 9H) |
| 42 | 1 | 1.03-1.23 (m, 2H), 1.60-1.90 (m, 2H), 2.28 (brs, 4H), 2.71 (s, 2H), 2.80-2.85 (m, 1H), 3.10-3.48 (m, 6H), 4.49-4.69 (m, 2H), 5.34 (s, 1H), 5.50 (s, 1H), 6.61 (s, 4H), 7.21-7.31 (m, 4H), 7.40-7.49 (m, 5H) |
| 43 | 1 | 1.10-1.20 (m, 2H), 1.65-1.90 (m, 2H), 2.26-2.30 (m, 4H), 2.69 (s, 2H), 2.75-2.90 (m, 1H), 3.20-3.54 (m, 7H), 4.45-4.70 (m, 2H), 6.61 (s, 2H), 7.23-7.60 (m, 9H) |
| 44 | 1 | 1.03-1.22 (m, 2H), 1.60-1.90 (m, 2H), 2.27-2.29 (m, 4H), 2.69-2.81 (m, 3H), 3.10-3.20 (m, 1H), 3.40-3.60 (m, 5H), 4.40-4.70 (m, 2H), 6.61 (s, 2H), 7.20-7.24 (m, 4H), 7.43-7.48 (m, 3H), 7.59 (d, 2H, J=8.4Hz) |
| 45 | 3 | 1.03-1.22 (m, 2H), 1.70-1.90 (m, 2H), 2.27 (brs, 4H), 2.47 (s, 3H), 2.69 (s, 2H), 2.80-3.20 (m, 2H), 3.45-3.66 (m, 5H), 4.40-4.68 (m, 2H), 6.61 (s, 2H), 7.19-7.29 (m, 6H), 7.42-7.47 (m, |
| 46 | 4 | 3H) 1.16-1.40 (m, 2H), 1.75-2.00 (m, 2H), 2.41 (brs, 4H), 2.76 (s, 2H), 2.80-2.95 (m, 1H), 3.07 (s, 3H), 3.10-3.20 (m, 1H), 3.62-3.69 (m, 5H), 4.78-4.89 (m, 2H), 7.06-7.12 (m, 2H), 7.45-7.49 (m, 5H), 7.97 (d, 2H, J=7.8Hz) |
| 47 | 3 | 1.03-1.20 (m, 2H), 1.65-1.90 (m, 2H), 2.26 (brs, 4H), 2.69 (s, 2H), 2.75-2.90 (m, 1H), 3.10-3.46 (m, 6H), 3.85 (s, 3H), 4.40-4.68 (m, 2H), 6.61 (s, 2H), 7.22 (brs, 2H), 7.38-7.46 (m, 5H), 7.95 (d, 2H, J=7.6Hz) |
| 48 | 1 | 1.64-1.74 (m, 1H), 2.00-2.15 (m, 1H), 2.20-2.30 (m, 4H), 2.60-2.75 (m, 2H), 2.90-3.00 (m, 0.5H), 3.10-3.50 (m, 6.5H), 3.60-3.80 (m, 1H), 4.80-4.95 (m, 0.5H), 5.05-5.20 (m, 0.5H), 6.61 (s, 2H), 7.20-7.60 (m, 9H) |
| 49 | 1 | 1.60-1.80 (m, 1H), 2.00-2.15 (m, 1H), 2.20-2.30 (m, 4H), 2.65-2.74 (m, 2H), 2.90-3.00 (m, 0.5H), 3.20-3.50 (m, 6.5H), 3.60-3.80 (m, 1H), 4.90-5.00 (m, 0.5H), 5.05-5.20 (m, 0.5H), 6.61 (s, 2H), 7.20-7.60 (m, 7H), 7.73-7.80 (m, 2H) |
| 52 | 3 | 1.03-1.14 (m, 2H), 1.29-1.53 (m, 6H), 1.65-1.90 (m, 2H), 2.32 (brs, 4H), 2.75-2.90 (m, 3H), 3.10-3.20 (m, 1H), 3.45-3.60 (m, 1H), 4.40-4.70 (m, 2H), 6.58 (s, 2H), 7.27-7.32 (m, 6H), 7.44-7.46 (m, 2H) |
| 53 | 3 | 1.10-1.40 (m, 8H), 1.65-1.90 (m, 2H), 2.27 (brs, 4H), 2.79 (brs, 3H), 3.10-3.20 (m, 1H), 3.50-3.60 (m, 1H), 4.40-4.65 (m, 2H), 6.59 (s, 2H), 7.20-7.52 (m, 8H) |
| 54 | 3 | 1.15-1.20 (m, 2H), 1.65-1.90 (m, 2H), 2.25 (brs, 4H), 2.70-2.90 (m, 3H), 3.10-3.20 (m, 1H), 3.45-3.60 (m, 5H), 4.40-4.61 (m, 2H), 6.61 (s, 2H), 7.23-7.49 (m, 5H) |
| 55 | 2 | 1.10-1.38 (m, 4H), 1.51-1.59 (m, 4H), 1.68-1.90 (m, 2H), 2.33 (brs, 4H), 2.73 (s, 2H), 2.80-2.90 (m, 1H), 3.10-3.20 (m, 1H), 3.60-3.70 (m, 1H), 3.86 (s, 3H), 4.79-4.89 (m, 2H), 6.92-6.99 (m, 4H), 7.42 (d, 2H, J=8.4Hz), 7.64 (d, 2H, J=8.4Hz) |
| 56 | 2 | 1.20-1.58 (m, 8H), 1.70-1.90 (m, 2H), 2.34 (brs, 4H), 2.73-2.90 (m, 3H), 3.00-3.20 (m, 1H), 3.70-3.80 (m, 1H), 3.86 (s, 3H), 4.72-4.88 (m, 2H), 6.90-6.96 (m, 4H), 7.23-7.35 (m, 4H) |
| 57 | 3 | 1.10-1.50 (m, 8H), 1.65-1.90 (m, 2H), 2.27 (brs, 4H), 2.70-2.90 (m 3H), 3.10-3.20 (m, 1H), 3.40-3.50 (m, 1H), 4.45-4.70 (m, 2H), 6.59 (s, 2H), 7.23 (brs, 1H), 7.38-7.52 (m, 5H), 7.76 (d, 2H, J=8.3Hz) |
| 58 | 2 | 1.15-1.45 (m, 2H), 1.75-2.00 (m, 2H), 2.40-2.42 (m, 4H), 2.75-2.95 (m, 3H), 3.10-3.20 (m, 1H), 3.66-3.69 (m, 5H), 4.80-4.89 (m, 2H), 7.05 (brs, 2H), 7.41-7.44 (m, 4H), 7.65 (d, 2H, J=8.1Hz) |
| 59 | 3 | 1.15-1.25 (m, 2H), 1.70-1.90 (m, 2H), 2.24 (brs, 4H), 2.70-2.90 (m, 3H), 3.15-3.60 (m, 6H), 4.50-4.65 (m, 2H), 6.61 (s, 2H), 7.24 (brs, 1H), 7.40-7.52 (m, 5H), 7.76 (d, 1H, J=8.0Hz) |
| 60 | 1 | 1.03-1.56 (m, 2H), 1.60-1.90 (m, 2H), 2.25-2.28 (m, 4H), 2.34 (s, 3H), 2.68 (s, 2H), 2.75-2.85 (m, 1H), 3.10-3.49 (m, 6H), 4.45-4.64 (m, 2H), 6.61 (s, 2H), 7.09 (brs, 2H), 7.25 (d, 2H, J=7.9Hz), 7.49 (d, 2H, J=8.3Hz), 7.76 (d, 2H, J=8.3Hz) |
| 61 | 3 | 1.12-1.22 (m, 2H), 1.70-1.90 (m, 2H), 2.23 (brs, 4H), 2.73-2.90 (m, 3H), 3.10-3.59 (m, 6H), 4.40-4.70 (m, 2H), 6.61 (s, 2H), 7.31 (d, 2H, J=7.7Hz), 7.44-7.48 (m, 4H), 7.82 (d, 2H, J=7.7Hz) |
| 62 | 5 | 1.2-1.4 (m, 1H), 1.5-1.7 (m, 1H), 1.75-2.25 (m, 2H), 2.5-2.8 (m, 4H), 2.8-3.1 (m, 3H), 3.70 (m, 4H),4.6-4.8 (m, 2H), 6.73 (s, 2H), 7.4-7.6 (m, 5H), 7.65-7.8 (m, 3H) |
| 63 | 5 | 1.2-1.4 (m, 2H), 1.5-1.7 (m, 3H), 1.75-1.95 (m, 5H), 2.0-2.25 (m, 2H), 2.9-3.2 (m, 5H), 3.3-3.4 (m, 1H), 3.5-3.75 (m, 2H),4.6-4.8 (m, 2H), 6.69 (s, 2H), 7.4-7.6 (m, 5H), 7.65-7.80 (m, 3H) |
| 64 | 1 | 1.01-1.80 (m, 14H), 2.36 (brs, 4H), 2.71-3.54 (m, 10H), 3.71-3.76 (m, 0.5H), 3.95-4.05 (m, 0.5H), 4.46-4.64 (m, 2H), 6.61 (s, 2H), 7.54 (brd, 1H, J=7.9Hz), 7.66 (brd, 1H, J=8.1Hz), 7.82 (brd, 2H, J=7.5Hz) |
| 65 | 1 | 1.01-1.80 (m, 14H), 2.37 (brs, 4H), 2.71-3.54 (m, 10H), 3.71-3.79 (m, 0.5H), 3.95-4.05 (m, 0.5H), 4.43-4.60 (m, 2H), 6.61 (s, 2H), 7.37 (brd, 1H, J=9.2Hz), 7.45-7.52 (m, 3H) |
| 66 | 2 | 1.10-1.25 (m, 2H), 1.60-1.85 (m, 2H), 2.31 (brs, 4H), 2.78-2.90 (m, 3H), 3.10-3.50 (m, 6H), 4.49-4.64 (m, 2H), 6.61 (s, 2H), 6.98 (brd, 1H, J=5.0Hz), 7.50 (brd, 3H, J=7.9Hz), 7.61-7.64 (m, 1H), 7.77 (brd, 2H, J=8.3Hz) |
| 67 | 5 | 1.3-1.6 (m, 2H), 1.8-2.2 (m, 2H), 2.6-2.8 (m, 5H), 2.9-3.2 (m, 3H), 3.6-3.8 (m, 5H), 4.7-4.9 (m, 2H), 6.73 (s, 2H), 7.4-7.5 (m, 3H), 7.65-7.80 (m, 2H), 8.09 (m, 1H), 8.19 (m, 1H), 9.39 (s, 1H) |
| 68 | 5 | 1.0-1.2 (m, 1H), 1.5-2.0 (m, 2H), 2.1-2.4 (m, 2H), 2.5-2.7 (m, 4H), 2.8-3.1 (m, 2H), 3.1-3.3 (m, 1H), 3.4-3.6 (m, 1H), 3.6-3.8 (m, 5H), 4.5-4.8 (m, 1H), 6.72 (s, 2H), 7.4-8.1 (m, 11H) |
| 69 | 5 | 1.3-1.6 (m, 4H), 1.8-2.2 (m, 6H), 2.9-3.2 (m, 5H), 3.5-3.7 (m, 3H), 4.7-4.9 (m, 2H), 6.69 (s, 2H), 7.4-7.5 (m, 3H), 7.65-7.80 (m, 2H), 8.09 (m, 1H), 8.21 (m, 1H), 9.41 (s, 1H) |
| 70 | 5 | 1.0-1.4 (m, 1H), 1.5-2.0 (m, 10H), 2.8-3.3 (m, 6H), 3.45-3.60 (m, 1H), 3.65-3.8 (m, 2H), 4.5-4.8 (m, 1H), 6.72 (s, 2H), 7.4-8.1 (m, 11H) |
| 71 | 1 | 1.15-1.90 (m, 12H), 2.50-2.90 (m, 3H), 3.05-3.60 (m, 7H), 3.73-3.94 (m, 3H), 4.46-4.68 (m, 1H), 6.60 (s, 2H), 7.53-7.68 (m, 2H), 7.79-7.83 (m, 2H) |
| 72 | 1 | 1.20-1.95 (m, 6H), 2.36 (brs, 4H), 2.50-2.90 (m, 3H), 3.02-3.53 (m, 12H), 3.70-4.10 (m, 2.5H), 4.49-4.70 (m, 0.5H), 6.59 (s, 2H), 7.53-7.67 (m, 2H), 7.80-7.82 (m, 2H) |
| 73 | 1 | 0.84-1.98 (m, 24H), 2.40 (brs, 4H), 2.70-3.60 (m, 6.5H), 3.85-3.95 (m, 0.5H), 4.40-4.60 (m, 0.5H), 4.93-4.97 (m, 0.5H), 6.59 (s, 5H), 7.52-7.69 (m, 2H), 7.78-7.83 (m, 2H) |
| 74 | 1 | 0.87-1.97 (m, 18H), 2.37 (brs, 4H), 3.00-3.80 (m, 10.5H), 3.80-4.05 (m, 0.5H), 4.45-4.60 (m, 0.5H), 4.95-4.98 (m, 0.5H), 6.61 (s, 4H), 7.53-7.68 (m, 2H), 7.79-7.83 (m, 2H) |
| 75 | 1 | 1.22-2.05 (m, 14H), 2.41 (brs, 4H), 2.60-2.90 (m, 3H), 3.05-3.60 (m, 5H), 4.10-4.25 (m, 0.5H), 4.30-4.50 (m, 0.5H), 4.60-4.70 (m, 0.5H), 5.30-5.40 (m, 0.5H), 6.58 (s, 2H), 6.90-7.20 (m, 4H), 7.45-7.55 (m, 1H), 7.63-7.67 (m, 1H), 7.78-7.80 (m, 2H) |
| 76 | 1 | 1.22-2.00 (m, 8H), 2.39 (brs, 4H), 2.60-2.90 (m, 3H), 3.13-3.56 (m, 9H), 4.20-4.30 (m, 0.5H), 4.40-4.50 (m, 0.5H), 4.60-4.70 (m, 0.5H), 5.25-5.40 (m, 0.5H), 6.61 (s, 2H), 6.95-7.20 (m, 4H), 7.45-7.55 (m, 1H), 7.63-7.67 (m, 1H), 7.75-7.85 (m, 2H) |
| 83 | 2 | 1.10-1.78 (m, 10H), 2.18-2.30 (m, 4H), 2.52-2.71 (m, 4H), 3.71-3.76 (m, 1H), 4.63-4.72 (m, 2H), 5.34 (s, 1H), 6.68-6.76 (m, 2H), 6.96-7.26 (m, 8H), 7.35-7.41 (m, 3H) |
| 84 | 3 | 0.95-1.76 (m, 18H), 2.15-2.40 (m, 5H), 2.75-2.80 (m, 3H), 3.00-3.15 (m 1H), 3.40-3.60 (m, 2H), 3.84-4.00 (m, 1H), 4.38-4.45 (m, 1H), 4.55-4.65 (m, 1H), 6.59 (s, 2H), 7.20 (d, 2H, J=6.6Hz), 7.40-7.45 (m, 3H) |
| 85 | 3 | 0.95-1.09 (m, 2H), 1.20-1.78 (m, 10H), 2.20-2.48 (m, 5H), 2.72-2.90 (m, 3H), 3.00-3.15 (m, 1H), 3.20-3.40 (m, 2H), 3.51 (brs, 4H), 3.84-3.99 (m, 1H), 4.42-4.45 (m, 1H), 4.58-4.64 (m, 1H), 6.62 (s, 2H), 7.22 (d, 2H, J=6.8Hz), 7.41-7.47 (m, 3H) |
| 86 | 1 | 0.94-1.07 (m, 2H), 1.29-1.79 (m, 10H), 2.10-2.27 (m, 5H), 2.67-2.72 (m, 3H), 2.96-3.09 (m, 1H), 3.20-3.48 (m, 5H), 3.95-3.99 (m, 1H), 4.38-4.43 (m, 1H), 4.57-4.66 (m, 1H), 6.60 (s, 2H), 7.20 (d, 2H, J=6.2Hz), 7.39-7.46 (m, 3H) |
| 87 | 2 | 1.26-1.39 (m, 2H), 1.91 (brd, 2H, J=12.1Hz), 2.42 (brs, 4H), 2.76 (s, 2H), 2.80-3.20 (m, 2H), 3.67-3.70 (m, 4H), 4.50-4.70 (m, 2H), 4.86-4.94 (m, 1H), 6.43 (s, 1H), 6.91 (d, 1H, J=3.3Hz), 7.05-7.08 (m, 2H), 7.40-7.43 (m, 4H) |
| 88 | 2 | 1.26-1.38 (m, 2H), 1.89 (brd, 2H, J=13.9Hz), 2.40-2.43 (m, 4H), 2.76 (s, 2H), 3.00-3.10 (m, 2H), 3.67-3.70 (m, 4H), 4.40-4.55 (m, 2H), 4.84-4.93 (m, 1H), 6.98-7.01 (m, 1H), 7.05-7.15 (m, 2H), 7.20 (dd, 1H, J=3.7,1.1Hz), 7.39-7.45 (m, 4H) |
| 89 | 1 | 1.05-1.19 (m, 2H), 1.75-1.80 (m, 2H), 2.25-2.27 (m, 4H), 2.68 (s, 2H), 2.97-3.06 (m, 2H), 3.44-3.47 (m, 4H), 4.29 (brt, 1H, J=12.9Hz), 4.68-4.72 (m, 1H), 6.59 (s, 2H), 6.99-7.13 (m, 2H), 7.20-7.23 (m, 2H), 7.37-7.58 (m, 6H) |
| 90 | 2 | 1.23-1.28 (m, 2H), 1.88 (brd, 2H, J=11.9Hz), 2.41 (t, 4H, J=4.5Hz), 2.76 (s, 2H), 2.80-3.20 (m, 2H), 3.69 (t, 4H, J=4.5Hz), 4.10-4.20 (m, 1H), 4.60-4.92 (m, 2H), 6.45 (s, 1H), 7.07-7.09 (m, 2H), 7.37-7.45 (m, 4H), 7.61 (s, 1H), 7.21-7.47 (m, 9H) |
| 91 | 2 | 1.20-1.40 (m, 2H), 1.80-2.00 (m, 2H), 2.39-2.43 (m, 4H), 2.76-2.86 (m, 3H), 3.10-3.30 (m, 1H), 3.66-3.70 (m, 5H), 4.76-4.93 (m, 2H), 7.09 (brs, 2H), 7.34-7.46 (m, 4H), 7.61-7.66 (m, 1H), 8.32-8.33 (m, 1H) |
| 92 | 2 | 1.22-1.42 (m, 2H), 1.83-2.00 (m, 2H), 2.41 (t, 4H, J=4.3Hz), 2.77 (s, 2H), 2.85-2.93 (m, 1H), 3.18-3.27 (m, 1H), 3.61-3.70 (m, 5H), 4.47-4.94 (m, 2H), 7.06-7.12 (m, 2H), 7.42-7.50 (m, 3H), 7.72 (d, 1H, J=8.1Hz), 7.83-7.87 (m, 1H), 8.64 (s, 1H) |
| 93 | 1 | 1.11-1.22 (m, 2H), 1.67-1.85 (m, 2H), 2.26 (brs, 4H), 2.67 (s, 2H), 2.77-2.86 (m, 1H), 3.11-3.47 (m, 6H), 4.43-4.64 (m, 2H), 6.61 (s, 2H), 7.21-7.23 (m, 2H), 7.43-7.55 (m, 5H) |
| 94 | 1 | 1.15-1.22 (m, 2H), 1.68-1.83 (m, 2H), 2.27 (brs, 4H), 2.69 (s, 2H), 2.75-2.90 (m, 1H), 3.20-3.52 (m, 6H), 4.46-4.69 (m, 2H), 6.61 (s, 2H), 7.22 (brs, 2H), 7.43-7.46 (m, 3H), 8.15 (s, 1H), 8.33 (s, 1H) |
| 95 | 2 | 1.19-1.93 (m, 13H), 2.41-2.63 (m, 6H), 2.76 (s, 2H), 3.15 (brt, 1H, J=12.5Hz), 3.69 (brt, 4H, J=4.6Hz), 3.95 (brd, 1H, J=12.7Hz), 4.68-4.88 (m, 2H), 7.08-7.12 (m, 4H), 7.23-7.26 (m, 2H), 7.41-7.43 (m, 3H) |
| 98 | 1 | 1.03-1.41 (m, 10H), 1.61-1.79 (m, 4H), 2.30-2.35 (m, 4H), 2.76 (s, 2H), 2.90-3.00 (m, 1H), 3.27-3.35 (m, 4H), 3.73-3.78 (m, 2H), 4.62-4.67 (m, 2H), 6.53-6.59 (m, 3H), 6.69 (d, 1H, J=8.6Hz), 6.87-6.91 (m, 1H), 7.11-7.21 (m, 3H), 7.42-7.45 (m, 3H) |
| 102 | 1 | 1.03-1.18 (m, 2H), 1.60-1.84 (m, 6H), 2.64 (brs, 4H), 2.80-2.90 (m, 1H), 3.09-3.20 (m, 3H), 3.45-3.60 (m, 1H), 4.40-4.70 (m, 2H), 6.56 (s, 2H), 7.22-7.48 (m, 9H) |
| 103 | 1 | 1.06-1.22 (m, 2H), 1.66-1.84 (m, 6H), 2.64 (brs, 4H), 2.80-2.90 (m, 1H), 3.10-3.25 (m, 3H), 3.40-3.50 (m, 1H), 4.44-4.56 (m, 1H), 4.60-4.72 (m, 1H), 6.56 (s, 2H), 7.23 (brs, 2H), 7.46-7.49 (m, 5H), 7.75 (d, 2H, J=8.0Hz) |
| 104 | 1 | 1.03-1.22 (m, 2H), 1.65-1.91 (m, 6H), 2.62 (brs, 4H), 2.80-2.90 (m, 1H), 3.07-3.25 (m, 3H), .3.45-3.55 (m, 1H), 4.40-4.75 (m, 2H), 6.55 (s, 2H), 7.24-7.28 (m, 3H), 7.45-7.48 (m, 3H), 7.57 (d, 1H, J=1.4Hz), 7.65 (d, 1H, J=8.1Hz) |
| 105 | 3 | 1.12-1.23 (m, 2H), 1.66-1.83 (m, 6H), 2.63 (brs, 4H), 2.80-2.90 (m, 1H), 3.08-3.17 (m, 3H), 3.51-3.60 (m, 1H), 4.46-4.68 (m, 2H), 6.55 (s, 2H), 7.13 (dd, 1H, J=8.2, 1.8Hz), 7.24 (d, 2H, J=5.9Hz), 7.37-7.49 (m, 4H), 7.61 (t, 1H, J=7.9Hz) |
| 106 | 3 | 1.03-1.20 (m, 2H), 1.40-1.60 (m, 8H), 1.65-1.85 (m, 2H), 2.60-2.62 (m 4H), 2.80-2.90 (m, 1H), 3.01 (s, 2H), 3.10-3.20 (m, 1H), 3.40-3.50 (m, 1H), 4.40- 4.70 (m, 2H), 6.58 (s, 2H), 7.23 (brs, 2H), 7.44-7.50 (m, 5H), 7.76 (d, 2H, J=8.3Hz) |
| 107 | 1 | 1.14-1.40 (m, 2H), 1.53-1.64 (m, 8H), 1.75-1.95 (m, 2H), 2.58-2.60 (m 4H), 2.80-3.20 (m, 4H), 3.60-3.80 (m, 1H), 4.72-4.92 (m, 2H), 7.07 (brs, 2H), 7.22-7.43 (m, 7H) |
| 108 | 3 | 1.03-1.20 (m, 2H), 1.75-1.89 (m, 2H), 2.18 (s, 3H), 2.31-2.49 (m, 8H), 2.68 (s, 2H), 2.80-2.90 (m, 1H), 3.16-3.50 (m, 2H), 4.45-4.70 (m, 2H), 6.56 (s, 2H), 7.22 (brs, 2H), 7.43-7.49 (m, 5H), 7.75 (d, 2H, J=8.0Hz ) |
| 109 | 1 | 1.10-1.20 (m, 2H), 1.60-1.90 (m, 2H), 2.24 (brs, 4H), 2.34 (brs, 4H), 2.44 (s, 3H), 2.70 (s, 2H), 2.80-2.90 (m, 1H), 3.10-3.25 (m, 1H), 3.40-3.50 (m, 1H), 4.40-4.70 (m, 2H), 6.55 (s, 2H), 7.20 (brs, 2H), 7.43-7.46 (m, 3H), 7.56-7.66 (m, 3H), 7.78 (d, 1H, J=7.5Hz) |
| 110 | 1 | 0.98-1.22 (m, 2H), 1.57-1.62 (m, 1H), 1.80-1.90 (m, 41H), 2.30 (brs, 4H), 2.38 (brs, 4H), 2.53 (s, 3H), 2.71-2.87 (m, 3H), 3.00-3.17 (m, 2H), 4.50-4.67 (m, 2H), 6.55 (s, 2H), 7.10-7.25 (m, 2H), 7.35-7.45 (m, 4H), 7.56-7.78 (m, 3H) |
| 111 | 2 | 1.20-1.50 (m, 2H), 1.80-1.96 (m, 6H), 2.37 (s, 2H), 2.80-2.90 (m, 1H), 3.05-3.20 (m, 1H), 3.32 (brs, 2H), 3.50-3.80 (m, 3H), 4.81-4.89 (m, 2H), 7.19 (brs, 2H), 7.39-7.47 (m, 5H), 7.63 (d, 2H, J=8.1Hz) |
| 112 | 2 | 1.20-1.40 (m, 2H), 1.80-1.97 (m, 6H), 2.37 (s, 2H), 2.80-2.90 (m, 1H), 3.05-3.20 (m, 1H), 3.32 (brs, 2H), 3.60-3.80 (m, 3H), 4.68-4.89 (m, 2H), 7.13-7.50 (m, 9H) |
| 113 | 5 | 1.20-1.50 (m, 2H), 1.80-2.10 (m, 2H), 2.70-2.80 (m, 4H), 2.95-3.05 (m, 5H), 3.20 (s, 2H), 3.20-3.30 (m, 1H), 4.60-4.90 (m, 2H), 6.72 (s, 2H), 7.26 (m, 2H), 7.51 (m, 5H), 7.74 (m, 2H) |
| 114 | 5 | 1.20-1.50 (m, 2H), 1.80-2.10 (m, 4H), 2.90-3.00 (m, 1H), 3.05-3.15 (m, 5H), 3.56 (s, 2H), 3.60-3.70 (m, 1H), 3.75-3.85 (m, 2H), 4.60-4.90 (m, 2H), 6.70(s, 2H), 7.29(m, 2H), 7.53 (m, 5H), 7.74 (m, 2H) |
| 115 | 5 | 1.09-1.10 (m, 6H), 1.20-1.50 (m, 2H), 1.80-2.10 (m, 4H), 2.90-3.00 (m, 3H), 3.26 (s, 2H), 3.15-3.25 (m, 1H), 3.60-3.70 (m, 1H), 3.75-3.85 (m, 2H), 4.60-4.90 (m, 2H), 6.70(s, 2H), 7.29(m, 2H), 7.53 (m, 5H), 7.74 (m, 2H) |
| 116 | 1 | 0.91-1.98 (m, 14H), 2.49-2.60 (m, 4H), 2.70 (s, 2H), 2.80-3.00 (m, 2H), 3.09-3.43 (m, 6H), 4.48-4.66 (m, 2H), 6.54 (s, 2H), 7.20 (brs, 2H), 7.30-7.50 (m, 5H), 7.76 (brd, 2H, J=8.3Hz) |
| 117 | 2 | 1.10-1.40 (m, 2H), 1.75-2.00 (m, 6H), 2.20-2.40 (m, 1H), 2.81-3.00 (m, 7H), 3.05-3.25 (m, 1H), 3.60- 3.70 (m, 1H), 4.70-4.91 (m, 2H), 7.00-7.15 (m, 2H), 7.39-7.45 (m, 5H), 7.64 (brd, 2H, J=8.3Hz) |
| 118 | 1 | 1.05-2.20 (m, 16H), 2.47-2.50 (m, 4H), 2.70-2.85 (m, 2H), 3.10-3.50 (m, 3H), 4.45-4.70 (m, 2H), 6.59 (s, 2.6H), 7.21 (brs, 2H), 7.43-7.50 (m, 5H), 7.76 (brd, 2H, J=8.3Hz) |
| 119 | 1 | 1.05-1.20 (m, 2H), 1.35-2.00 (m, 13H), 2.60-2.85 (m, 11H), 3.10-3.20 (m, 1H), 3.40-3.50 (m, 1H), 4.20-4.70 (m, 2H), 6.55 (s, 4H), 7.20 (brs, 2H), 7.43-7.50 (m, 5H), 7.76 (brd, 2H, J=8.4Hz) |
| 120 | 1 | 0.83 (d, 3H, J=6.2Hz), 1.01-1.30 (m, 5H), 1.46 (brd, 2H, J=11.0Hz), 1.65-2.01 (m, 4H), 2.62-2.84 (m, 5H), 3.05-3.20 (m, 1H), 3.40-3.50 (m, 1H), 4.48-4.70 (m, 2H), 6.59 (s, 2H), 7.20 (brs, 2H), 7.44-7.50 (m, 5H), 7.76 (brd, 2H, J=8.4Hz) |
| 121 | 1 | 1.05-1.25 (m, 2H), 1.53-1.90 (m, 5H), 2.00-2.15 (m, 2H), 2.70-2.90 (m, 5H), 3.10-3.50 (m, 2H), 4.40-4.80 (m, 2H), 6.60 (s, 2H), 7.13-7.29 (m, 7H), 7.45-7.50 (m, 5H), 7.76 (brd, 2H, J=8.4Hz) |
| 122 | 1 | 1.05-1.25 (m, 2H), 1.65-1.90 (m, 2H), 2.57-2.69 (m, 4H), 2.87-2.96 (m, 3H), 3.30 (brt, 1H, J=6.2Hz), 3.40-3.55 (m, 2H), 4.19 (s, 1H), 4.40-4.80 (m, 2H), 6.54 (s, 2H), 6.94-7.08 (m, 4H), 7.16-7.24 (m, 3H), 7.44-7.50 (m, 4H), 7.76 (brd, 2H, J=8.3Hz) |
| 123 | 1 | 0.70-0.87 (m, 6H), 1.05-1.25 (m, 2H), 1.50-1.95 (m, 8H), 2.57-2.62 (m, 2H), 2.73-2.85 (m, 3H), 3.05-3.55 (m, 2H), 4.20-4.75 (m, 2H), 6.59 (s, 2.6H), 7.19 (brs, 2H), 7.39-7.49 (m, 5H), 7.75 (brd, 2H, J=8.3Hz) |
| 124 | 2 | 1.20-1.50 (m, 2H), 1.70-2.05 (m, 6H), 2.52 (brt, 4H, J=5.5Hz), 2.82-2.90 (m, 3H), 3.10-3.25 (m, 1H), 3.60-3.80 (m, 1H), 4.70-4.95 (m, 2H), 7.00-7.20 (m, 2H), 7.39-7.46 (m, 5H), 7.73 (brd, 2H, J=8.1Hz) |
| 125 | 1 | 1.05-1.35 (m, 4H), 1.45-1.90 (m, 4H), 1.90-2.05 (m, 2H), 2.69 (brs, 2H), 2.80-2.90 (m, 2H), 3.05-3.50 (m, 4H), 3.69-3.78 (m, 1H), 4.40-4.70 (m, 2H), 6.53 (s, 2H), 7.19 (brs, 2H), 7.43-7.50 (m, 5H), 7.76 (brd, 2H, J=8.4Hz) |
| 126 | 1 | 1.00-1.25 (m, 2H), 1.50-1.90 (m, 2H), 2.10-2.30 (m, 2H), 2.60-2.90 (m, 3H), 3.05-3.50 (m, 6H), 4.40-4.70 (m, 2H), 6.58 (s, 2H), 7.05-7.50 (m, 7H), 7.68-7.77 (m, 2H) |
| 127 | 1 | 1.03-1.30 (m, 2H), 1.65-1.90 (m, 2H), 2.15-2.25 (m, 1H), 2.40-2.50 (m, 4H), 2.75-2.95 (m, 7H), 3.05-3.20 (m, 1H), 3.40-3.50 (m, 1H), 4.40-4.70 (m, 2H), 6.47 (s, 2H), 7.15-7.30 (m, 2H), 7.44-7.50 (m, 5H), 7.76 (d, 2H, J=8.3Hz) |
| 128 | 1 | 1.05-1.25 (m, 2H), 1.60-1.90 (m, 2H), 2.42 (brs, 4H), 2.77-2.90 (m, 3H), 3.02-3.50 (m, 6H), 4.45-4.70 (m, 2H), 6.61 (s, 2H), 6.88 (d, 2H, J=9.0Hz), 7.17-7.22 (m, 4H), 7.44-7.50 (m, 5H), 7.76 (d, 2H, J=8.1Hz) |
| 129 | 1 | 1.05-1.90 (m, 12H), 2.32 (brs, 4H), 2.50-2.60 (m, 5H), 2.68 (s, 2H), 2.75-2.90 (m, 1H), 3.10-3.20 (m, 1H), 3.40-3.55 (m, 1H), 4.45-4.70 (m, 2H), 6.60 (s, 2H), 7.15-7.25 (m, 2H), 7.43-7.50 (m, 5H), 7.76 (d, 2H, J=8.4Hz) |
| 130 | 2 | 1.20-1.40 (m, 2H), 1.70-2.00 (m, 2H), 2.71 (t, 2H, J=6.3Hz), 2.79-2.90 (m, 3H), 3.02 (t, 2H, J=6.3Hz), 3.10-3.20 (m, 1H), 3.60-3.70 (m, 1H), 4.09 (s, 2H), 4.80-4.94 (m, 2H), 7.00-7.20 (m, 2H), 7.39-7.45 (m, 5H), 7.64 (d, 2H, J=8.3Hz) |
| 131 | 1 | 1.03-1.25 (m, 2H), 1.60-1.90 (m, 4H), 2.31 (s, 6H), 2.41-2.57 (m, 4H), 2.70-2.92 (m, 3H), 3.10-3.20 (m, 2H), 3.40-3.50 (m, 1H), 4.45-4.70 (m, 2H), 6.52 (s, 2H), 7.15-7.30 (m, 2H), 7.44-7.50 (m, 5H), 7.76 (d, 2H, J=8.4Hz) |
| 132 | 1 | 1.05-1.30 (m, 2H), 1.65-1.95 (m, 2H), 2.80-2.95 (m, 1H), 3.11-3.20 (m, 3H), 3.40-3.55 (m, 1H), 3.89 (brs, 4H), 4.45-4.75 (m, 2H), 6.60 (s, 2H), 7.15-7.30 (m, 6H), 7.46-7.50 (m, 5H), 7.76 (d, 2H, J=8.3Hz) |
| 133 | 1 | 1.03-1.25 (m, 2H), 1.80-1.95 (m, 2H), 2.72 (brs, 2H), 2.75-2.90 (m, 1H), 3.03 (brs, 1H), 3.19 (brs, 1H), 3.44 (brs, 4H), 4.48-4.70 (m, 2H), 5.70 (s, 2H), 6.58 (s, 2H), 7.20-7.30 (m, 2H), 7.45-7.50 (m, 5H), 7.76 (d, 2H, J=8.1Hz) |
| 148 | 1 | 1.10-1.25 (m, 2H), 1.50-2.00 (m, 2H), 2.26 (brs, 4H), 2.75-2.90 (m, 1H), 3.10-3.66 (m, 7H), 4.40-4.60 (m, 1H), 4.64-4.80 (m, 1H), 6.55-6.75(m, 5H), 7.10 (brs, 2H), 7.26-7.56 (m, 9H), 7.74 (brd, 2H, J=7.3Hz) |
| 149 | 1 | 0.96 (d, 3H, J=6.6Hz), 1.03-1.30 (m, 2H), 1.60-1.97 (m, 2H), 2.10-2.25 (m, 2H), 2.38-2.45 (m, 2H), 2.84 (q, 1H, J=6.7Hz), 3.10-3.20 (m, 1H), 3.40-3.60 (m, 6H), 4.40-4.75 (m, 2H), 6.60 (s, 2H), 7.10-7.30 (m, 2H), 7.43-7.50 (m, 5H), 7.76 (d, 2H, J=7.9Hz) |
| 154 | 3 | 1.04-1.30 (m, 2H), 1.70-1.91 (m, 2H), 2.00 (t, 2H, J=7.3Hz), 2.19 (brs, 4H), 2.47-2.51 (m, 2H), 2.80-2.90 (m, 1H), 3.20-3.80 (m, 6H), 4.45-4.60 (m, 1H), 4.67-4.73 (m, 1H), 6.61 (s, 2H), 7.23 (brs, 2H), 7.45-7.51 (m, 5H), 7.77 (d, 2H, J=8.0Hz) |
| 155 | 2 | 1.13-1.52 (m, 8H), 1.76-1.95 (m, 2H), 2.10 (t, 2H, J=7.6Hz), 2.22 (brs, 4H), 2.59 (t, 2H, J=7.6Hz), 2.80-2.90 (m, 1H), 3.12-3.19 (m, 1H), 3.62-3.66 (m, 1H), 4.72-4.95 (m, 2H), 7.07 (brs, 2H), 7.39-7.42 (m, 5H), 7.63 (d, 2H, J=8.1Hz) |
| 156 | 2 | 1.28-1.82 (m, 10H), 2.31 (t, 2H, J=7.6Hz), 2.20-2.22 (m, 4H), 2.59 (t, 2H, J=7.6Hz), 2.84-3.13 (m, 2H), 3.60-3.80 (m, 1H), 4.74-4.93 (m, 2H), 7.06 (brs, 2H), 7.22-7.43 (m, 7H) |
| 157 | 1 | 1.03-1.20 (m, 2H), 1.67-1.91 (m, 6H), 2.11 (t, 2H, J=7.3Hz), 2.59 (brs, 4H), 2.81-2.86 (m, 3H), 3.16-3.43 (m, 2H), 4.40-4.55 (m, 1H), 4.64-4.72 (m, 1H), 6.53 (s, 2H), 7.22 (brs, 2H), 7.46-7.50 (m, 5H), 7.76 (t, 2H, J=8.1Hz) |
| 158 | 1 | 1.10-1.20 (m, 2H), 1.68-1.91 (m, 6H), 2.12 (t, 2H, J=7.4Hz), 2.59-2.64 (m, 4H), 2.84-2.89 (m, 3H), 3.10-3.20 (m, 1H), 3.40-3.60 (m, 1H), 4.40-4.55 (m, 1H), 4.63-4.71 (m, 1H), 6.53 (s, 2H), 7.20-7.30 (m, 4H), 7.43-7.50 (m, 5H) |
| 182 | 2 | 0.72-0.88 (m, 1H), 1.07-1.79 (m, 9H), 2.30-2.32 (m, 4H), 2.55-2.65 (m, 1H), 2.70 (s, 2H), 3.02-3.12 (m, 1H), 3.60 (s, 2H), 3.75-3.80 (m, 1H), 4.64-4.68 (m, 1H), 4.76-4.86 (m, 1H), 6.89 (brs, 1H), 7.03-7.06 (m, 3H), 7.15-7.19 (m, 2H), 7.37-7.43 (m, 3H) |
| 183 | 3 | 0.80-1.03 (m, 2H), 1.61-1.75 (m, 2H), 2.24-2.27 (m, 4H), 2.53-2.61 (m, 1H), 2.67 (s, 2H), 3.04 (t, 1H, J=12.2Hz), 3.44-3.47 (m, 4H), 3.59 (d, 1H, J=25.0Hz), 3.63 (d, 1H, J=25.0Hz), 3.91 (brd, 1H, J=13.9Hz), 4.39 (brd, 1H, J=12.7Hz), 4.57-4.64 (m, 1H), 6.60 (s, 2H), 7.10-7.12 (m, 4H), 7.24 (dd, 2H, J=6.5, 1.8Hz), 7.42 (brs, 3H) |
| 184 | 3 | 0.80-1.03 (m, 3H), 1.12-1.43 (m, 5H), 1.64-1.77 (m, 2H), 2.35 (brs, 4H), 2.53-2.62 (m, 1H), 2.80 (s, 2H), 3.07 (brt, 1H, J=12.6Hz), 3.69-3.79 (m, 2H), 3.94 (brd, 1H, J=13.7Hz), 4.41 (brd, 1H, J=12.6Hz), 4.60-4.70 (m, 1H), 6.58 (s, 2H), 7.12 (brs, 2H), 7.32 (d, 2H, J=7.9Hz), 7.41 (brs, 2H), 7.56 (d, 2H, J=7.9Hz) |
| 185 | 3 | 0.85-1.03 (m, 2H), 1.64-1.76 (m, 2H), 2.26-2.27 (m, 4H), 2.59 (t, 1H, J=13.7Hz), 2.68 (s, 2H), 3.06 (t, 2H, J=12.2Hz), 3.44-3.47 (m, 4H), 3.68-3.78 (m, 2H), 3.93 (d, 1H, J=13.7Hz), 4.40 (d, 1H, J=13.2Hz), 4.55-4.65 (m, 1H), 6.61 (s, 2H), 7.10-7.20 (m, 2H), 7.33 (d, 2H, J=8.0Hz), 7.40 (brs, 3H), 7.56 (d, 2H, J=8.0Hz) |
| 186 | 1 | 0.80-1.00 (m, 2H), 1.60-1.80 (m, 2H), 2.25 (brs, 4H), 2.50-2.58 (m, 1H), 2.66 (s, 2H), 3.00-3.10 (m, 1H), 3.44 (brs, 4H), 3.58-3.66 (m, 2H), 3.90-4.00 (m, 1H), 4.30-4.40 (m, 1H), 4.55-4.65 (m, 1H), 6.59 (s, 2H), 7.07-7.14 (m, 3H), 7.37-7.46 (m, 5H) |
| 187 | 1 | 0.80-1.00 (m, 2H), 1.55-1.70 (m, 2H), 2.25 (brs, 4H), 2.50-2.57 (m, 1H), 2.66 (s, 2H), 2.83 (s, 6H), 2.93-3.02 (m, 1H), 3.40-3.50 (m, 6H), 3.90 (brd, 1H, J=13.2Hz), 4.40 (brd, 1H, J=12.5Hz), 4.50-4.65 (m, 1H), 6.53-6.61 (m, 4H), 6.90 (d, 2H, J=8.4Hz), 7.10-7.20 (m, 2H), 7.41 (brs, 3H) |
| 188 | 1 | 0.84-1.03 (m, 2H), 1.65-1.80 (m, 2H), 2.25-2.27 (m, 4H), 2.53-2.62 (m, 1H), 2.67 (s, 2H), 3.06 (brt, 1H, J=12.2Hz), 3.44-3.47 (m, 4H), 3.66 (s, 2H), 3.94 (brd, 1H, J=13.2Hz), 4.40 (brd, 1H, J=13.2Hz), 4.45-4.66 (m, 1H), 6.60 (s, 2H), 7.10-7.24 (m, 6H), 7.41 (brs, 3H) |
| 189 | 1 | 0.84-1.03 (m, 2H), 1.60-1.80 (m, 2H), 2.20-2.30 (m, 4H), 2.52-2.58 (m, 1H), 2.65 (s, 2H), 3.05 (brt, 1H, J=12.2Hz), 3.43-3.46 (m, 4H), 3.65 (s, 2H), 3.95 (brd, 1H, J=13.2Hz), 4.42 (brd, 1H, J=11.4Hz), 4.55-4.65 (m, 1H), 6.61 (s, 2H), 7.10-7.51 (m, 12H), 7.59 (d, 1H, J=7.7Hz) |
| 191 | 2 | 1.27-1.71 (m, 18H), 2.29 (brs, 4H), 2.50-2.80 (m, 4H), 3.41-3.50 (m, 1H), 4.65-4.73 (m, 2H), 6.68-7.24 (m, 6H), 7.41-7.42 (m, 3H) |
| 192 | 2 | 0.64-0.70 (m, 1H), 1.13-1.79 (m, 9H), 2.29 (brs, 4H), 2.58-2.69 (m, 3H), 2.98-3.07 (m, 1H), 3.88-3.93 (m, 1H), 3.88-3.93 (m, 1H), 4.72-4.83 (m, 2H), 5.12 (s, 1H), 6.84 (brs, 1H), 7.04-7.42 (m, 14H) |
| 193 | 2 | 1.09-1.80 (m, 10H), 2.27 (brs, 4H), 2.63-2.80 (m, 4H), 3.60-3.65 (m, 1H), 4.65-4.75 (m, 2H), 5.97 (s, 1H), 7.16-7.42 (m, 15H) |
| 194 | 1 | 0.55-1.90 (m, 15H), 2.25-2.32 (m, 4H), 2.62-2.75 (m, 2H), 2.85-3.10 (m, 2H), 3.30-3.60 (m, 5H), 4.05-4.25 (m, 1H), 4.39-4.43 (m, 1H), 4.50-4.75 (m, 1H), 6.65 (s, 4H), 7.10-7.49 (m, 10H) |
| 198 | 2 | 1.12-1.68 (m, 16H), 2.15-2.66 (m, 10H), 3.48-3.50 (m, 1H), 4.67-4.75 (m, 2H), 6.78-7.03 (m, 6H), 7.44 (brs, 3H). |
| 199 | 2 | 1.34-1.68 (m, 18H), 2.20-2.32 (m, 5H), 2.55-2.69 (m, 3H), 3.50-3.60 (m, 1H), 4.63-4.71 (m, 2H), 6.78-6.83 (m, 5H), 7.01-7.09 (m, 1H), 7.38-7.40 (m, 3H) |
| 200 | 1 | 1.12-1.70 (m, 16H), 2.28-2.66 (m, 10H), 3.40-3.50 (m, 1H), 4.61-4.69 (m, 2H), 7.18-7.39 (m, 9H) |
| 201 | 2 | 1.34-1.68 (m, 18H), 2.20-2.32 (m, 5H), 2.55-2.69 (m, 3H), 3.50-3.60 (m, 1H), 4.63-4.71 (m, 2H), 6.78-6.83 (m, 5H), 7.01-7.09 (m, 1H), 7.38-7.40 (m, 3H) |
| 202 | 2 | 1.05-1.68 (m, 18H), 2.17-2.29 (m, 5H), 2.55-2.67 (m, 3H), 3.50-3.60 (m, 1H), 4.64-4.73 (m, 2H), 6.67-7.18 (m, 6H), 7.41 (brs, 3H) |
| 203 | 2 | 1.27-1.69 (m, 18H), 2.17-2.29 (m, 5H), 2.50-2.66 (m, 3H), 3.50-3.55 (m, 1H), 4.62-4.71 (m, 1H), 7.03-7.13 (m, 6H), 7.39-7.40 (m, 3H) |
| 204 | 1 | 1.25-1.60 (m, 18H), 1.95-2.05 (m, 1H), 2.25 (brs, 4H), 2.55-2.68 (m, 3H), 3.00-3.04 (m, 1H), 3.28-3.33 (m, 1H), 3.72 (s, 3H), 4.40-4.50 (m, 1H), 6.57 (s, 2H), 6.67 (d, 2H, J=8.8Hz), 6.87-7.15 (m, 4H), 7.43 (brs, 3H) |
| 205 | 2 | 0.69-1.74 (m, 14H), 2.30 (brs, 4H), 2.68-2.85 (m, 4H), 4.10-4.10 (m, 1H), 4.60-4.79 (m, 2H), 6.86-7.15 (m, 7H), 7.40 (brs, 3H) |
| 206 | 2 | 1.25-1.57 (m, 14H), 2.25-2.30 (m, 4H), 2.60-2.90 (m, 4H), 3.95-4.10 (m, 1H), 4.60-4.81 (m, 2H), 6.94-6.98 (m, 2H), 7.07-7.11 (m, 2H), 7.24-7.28 (m, 2H), 7.39-7.43 (m, 1H) |
| 207 | 1 | 0.57-0.91 (m, 14H), 1.82 (brs, 4H), 2.10-2.26 (m, 4H), 3.21 (s, 3H), 3.45-3.55 (m, 1H), 3.80-4.20 (m, 2H), 6.09 (s,2H), 6.19 (d, 2H, J=8.8Hz), 6.42 (d, 2H, J=8.8Hz), 6.50-6.70 (m, 2H), 6.91 (brs, 3H) |
| 208 | 1 | 1.09-1.39 (m, 14H), 2.22 (s, 3H), 2.28 (brs, 4H), 2.60-2.90 (m, 4H), 3.80-4.00 (m, 1H), 4.35-4.60 (m, 2H), 6.58 (s, 2H), 6.83-7.10 (m, 6H), 7.44 (brs, 3H) |
| 209 | 2 | 1.00-1.10 (m, 1H), 1.30-1.40 (m, 3H), 1.47-1.53 (m, 4H), 1.69-2.05 (m, 4H), 2.25-2.30 (m, 4H), 2.50-2.60 (m, 2H), 2.65 (s, 2H), 2.70-2.80 (m, 1H), 2.90-3.00 (m, 1H), 3.23-3.29 (m, 1H), 4.62-4.75 (m, 2H), 6.73 (brs, 1H), 6.98 (brs, 1H), 7.13 (s, 4H), 7.41 (brs, 3H) |
| 211 | 2 | 1.27-1.62 (m, 12H), 2.16 (brd, 2H, J=13.0Hz), 2.27 (brs, 4H), 2.60-2.75 (m, 4H), 3.60-3.95 (m, 6H), 4.65-4.74 (m, 1H), 7.21-7.26 (m, 4H), 7.40-7.43 (m, 5H) |
| 212 | 2 | 1.26-1.69 (m, 18H), 2.15-2.28 (m, 5H), 2.40-2.64 (m, 3H), 3.40-3.60 (m, 1H), 4.63-4.74 (m, 2H), 6.79-7.16 (m, 8H) |
| 213 | 2 | 1.42-1.73 (m, 18H), 2.18-2.26 (m, 5H), 2.55-2.65 (m, 3H), 3.50-3.60 (m, 1H), 4.61-4.72 (m, 2H), 6.69-6.80 (m, 2H), 6.91-7.20 (m, 6H) |
| 214 | 1 | 1.23-1.60 (m, 18H), 2.29 (brs, 4H), 2.45-2.50 (m, 2H), 2.71 (s, 2H), 3.20-3.60 (m, 1H), 3.83 (s, 3H), 4.40-4.50 (m, 2H), 6.58 (s, 2H), 6.90-7.04 (m, 6H), 7.14 (brd, 2H, J=8.4Hz) |
| 215 | 1 | 1.23-1.77 (m, 18H), 2.42 (brs, 4H), 2.49-2.60 (m, 2H), 2.84 (s, 2H), 3.30-3.40 (m, 1H), 3.81 (s, 3H), 4.40-4.50 (m, 2H), 6.59 (s, 5H), 6.68-6.93 (m, 3H), 7.19-7.65 (m, 5H) |
| 216 | 1 | 1.30-1.77 (m, 18H), 2.39-2.60 (m, 6H), 2.84 (s, 2H), 3.40-3.50 (m, 1H), 4.40-4.50 (m, 2H), 6.60 (s, 5H), 6.89 (s, 1H), 7.20-7.65 (m, 7H) |
| 217 | 2 | 1.33-1.62 (m, 12H), 2.15-2.29 (m, 6H), 2.60-2.73 (m, 4H), 3.70-3.88 (m, 9H), 4.62-4.70 (m, 1H), 6.77-6.90 (m, 3H), 7.23-7.26 (m, 3H), 7.45 (d, 2H, J=8.4Hz) |
| 218 | 1 | 1.26-1.71 (m, 12H), 2.16-2.26 (m, 6H), 2.57-2.68 (m, 4H), 3.68-3.89 (m, 6H), 4.61-4.71 (m, 1H), 7.06-7.12 (m, 3H), 7.24-7.30 (m, 2H), 7.39-7.46 (m, 3H) |
| 219 | 2 | 1.26-1.62 (m, 16H), 2.29 (brs, 4H), 2.50-2.68 (m, 4H), 3.30-3.50 (m, 1H), 3.88 (s, 3H), 4.61-4.69 (m, 2H), 6.71-7.25 (m, 8H) |
| 220 | 2 | 1.26-1.70 (m, 16H), 2.27-2.31 (m, 4H), 2.60-2.67 (m, 4H), 3.30-3.50 (m, 1H), 3.90 (s, 3H), 4.62-4.73 (m, 2H), 6.70-6.94 (m, 4H), 6.99-7.12 (m, 4H) |
| 4 | 6 | 1.06(m,6H), 1.1-1.6(m,4H), 1.8-2.2(m,2H), 2.35(m,1H), 2.96(m,1H), 3.1-3.8(m,3H), 3.8-4.2(m,4H), 4.6-5.0(m,2H), 7.3-7.4(m,2H), 7.4-7.6(m,5H), 7.6-7.8(m,2H) |
| 50 | 2 | 0.90-1.30 (m, 1H), 1.10-1.55 (m, 7H), 1.71 (s, 6H), 2.46-2.70 (m, 6H), 2.84 (s, 2H), 3.30-3.50 (m, 1H), 4.65-4.80 (m, 2H), 6.80-7.15 (m, 6H), 7.40-7.55 (m, 3H) |
| 51 | 1 | 1.20-2.10 (m, 6H), 2.20-2.50 (m, 4H), 2.69 (brd, 2H, J=8.6Hz), 3.00-3.85 (m, 8H), 4.40-4.60 (m, 1H), 6.61 (s, 2H), 7.18-7.30 (m, 2H), 7.35-7.50 (m, 5H), 7.72-7.85 (m, 2H) |
| 77 | 3 | 1.00-1.80 (m, 18H), 2.28-2.40 (m, 1H), 2.65-2.85 (m, 5H), 2.95-3.90 (m, 5H), 4.40-4.65 (m, 1H), 6.54 (s, 2H), 7.54 (d, 1H, J=8.0Hz), 7.68 (d, 1H, J=8.0Hz), 7.82 (d, 1H, J=8.0Hz) |
| 78 | 3 | 1.04-1.85 (m, 18H), 2.25-2.40 (m, 1H), 2.80 (brs, 4H), 3.00-3.20 (m, 1H), 3.30-3.85 (m, 6H), 4.40-4.60 (m, 2H), 6.57 (s, 2H), 7.20-7.55 (m, 4H) |
| 79 | 5 | 1.3-1.7 (m, 4H), 1.8-1.9 (m, 5H), 1.9-2.0 (m, 1H), 2.99 (m, 7H), 3.1-3.25 (m, 4H), 3.66 (m, 3H), 4.6-4.8 (m, 4H), 6.51 (m, 2H), 6.68 (s, 2H), 6.88 (m, 1H), 7.33 (t, 1H, J=8.0Hz), 7.51 (d, 2H, J=8.0Hz), 7.74 (d, 2H, J=8.0Hz) |
| 80 | 5 | 1.3-1.7 (m, 4H), 1.8-1.9 (m, 5H), 1.9-2.0 (m, 1H), 2.94 (m, 1H), 3.1-3.25 (m, 4H), 3.63 (m, 3H), 3.85 (s, 3H), 4.6-4.8 (m, 4H), 6.69 (s, 2H), 6.86 (m, 2H), 7.11 (m, 1H), 7.46 (t, 1H, J=8.0Hz), 7.52 (d, 2H, J=8.0Hz), 7.74 (d, 2H, J=8.0Hz) |
| 81 | 5 | 1.23-1.47 (m, 2H), 1.77-1.90 (m, 1H), 1.90-2.10 (m, 2H), 2.10-2.28 (m, 1H), 2.44-2.58 (m, 1H), 2.63-2.76 (m, 1H), 2.77 (s, 6H), 2.84-2.97 (m, 2H), 2.98-3.17 (m, 3H), 3.55-3.76 (m, 3H), 4.65-4.85 (m, 2H), 6.69 (s, 2.4H), 7.15-7.27 (m, 1H), 7.32-7.35 (m, 1H), 7.45-7.60 (m, 4H) , 7.74 (d, 2H, J=8.1Hz) |
| 82 | 4 | 1.06-1.23 (m, 1H), 1.32-1.50 (m, 4H),1.66-1.76 (m, 4H),1.78-2.05 (m, 3H), 2.39-2.55 (m, 4H), 2.80-2.96 (m, 3H), 3.09-3.20 (m, 1H), 3.59-3.74 (m, 1H), 4.35-4.54 (m, 2H), 4.68-5.00 (m, 2H), 7.20-7.37 (m, 1H), 7.42 (d, 2H, J=8.1Hz), 7.49-7.58 (m, 1H), 7.64 (d, 2H, J=8.1Hz), 7.70-7.85 (m, 1H) , 8.12 (d, 1H, J=8.1Hz) |
| 99 | 3 | 1.10-1.25 (m, 2H), 1.58-1.95 (m, 6H), 2.55 (brs, 4H), 2.80-2.90 (m, 1H), 2.95-3.05 (s, 2H), 3.15-3.25 (m, 1H), 3.40-3.50 (m, 1H), 4.50-4.60 (m, 1H), 4.62-4.75 (m, 1H), 6.54 (s, 2H), 7.23 (brs, 2H), 7.40-7.52 (m, 3H), 7.93 (d, 1H, J=8.0Hz ), 8.02 (d, 1H, J=8.0Hz ), 8.69 (s, 1H) |
| 100 | 3 | 1.55-1.70 (m, 1H), 1.00-1.15 (m, 1H), 1.50-1.60 (m, 5H), 1.75-1.90 (m, 1H), 2.32 (brs, 4H), 2.74-3.05 (m, 4H), 3.35-3.50 (m, 1H), 4.35-4.50 (m, 1H), 4.55-4.70 (m, 1H), 6.86 (s, 1H), 7.15-7.31(m, 7H), 7.45 (brs, 3H), 7.78 (s, 1H) |
| 101 | 3 | 1.10-1.25 (m, 2H), 1.64 (brs, 4H), 1.80-1.95 (m, 2H), 2.40-2.60 (m, 4H), 2.95-3.05 (m, 4H), 4.20-4.50 (m, 2H), 4.68-4.80 (m, 1H), 6.55 (s, 2H), 7.20-7.31 (m, 4H), 7.39-7.47 (m, 4H), 7.61 (d, 1H, J=8.0Hz ), 7.69 (d, 1H, J=8.0Hz ) |
| 134 | 1 | 1.05-2.00 (m, 11H), 2.60-2.90 (m, 6H), 3.05-3.55 (m, 6H), 4.40-4.70 (m, 2H), 6.59 (s, 2H), 7.15-7.25 (m, 2H), 7.38-7.52 (m, 5H), 7.76 (d, 2H, J=8.0Hz) |
| 135 | 5 | 1.3-1.5 (m, 2H), 1.8-2.1 (m, 3H), 2.95 (m, 1H), 3.2-3.5 (m, 5H), 3.6-3.7 (m, 1H), 3.79 (s, 2H), 4.46 (m, 1H), 4.65-4.85 (m, 2H), 6.69 (s, 2H), 7.31 (m, 2H), 7.4-7.6 (m, 5H), 7.74 (d, 2H, J=8.0Hz) |
| 136 | 1 | 0.82 (s, 6H), 1.10-1.30 (m, 6H), 1.65-1.90 (m, 2H), 2.25-2.40 (m, 4H), 2.82 (s, 2H), 3.10-3.50 (m, 3H), 4.40-4.75 (m, 2H), 6.59 (s, 2H), 7.15-7.25 (m, 2H), 7.40-7.50 (m, 5H), 7.76 (d, 2H, J=8.0Hz) |
| 137 | 3 | 1.15-1.25 (m, 2H), 1.39 (s, 9H), 1.50-1.60 (m, 2H), 1.70-1.95 (m, 4H), 2.80-3.05 (m, 3H), 3.10-3.20 (m, 1H), 3.40-3.50 (m, 1H), 3.85-3.95 (m, 2H), 4.45-4.65 (m, 2H), 5.10 (s, 2H), 6.62 (s, 2.8H), 7.35-7.60 (m, 7H), 7.75 (d, 2H, J=8.1Hz) |
| 138 | 3 | 1.10-1.25 (m, 2H), 1.50-1.90 (m, 6H), 2.05-2.25 (m, 2H), 2.52 (s, 3H), 2.65-2.90 (m, 5H), 3.10-3.50 (m, 3H), 4.40-4.58 (m, 1H), 4.60-4.72 (m, 1H), 6.61 (s, 2H), 7.22 (brs, 2H), 7.38-7.52 (m, 5H), 7.75 (d, 2H, J=8.0Hz ) |
| 139 | 6 | 1.24 (t, 3H, J=7.1Hz), 1.2-1.4 (m, 2H), 1.8-2.1 (m, 2H), 2.94 (m, 1H), 3.09 (s, 2H), 3.5-3.7 (m, 6H), 4.10 (q, 2H, J=7.1Hz), 4.6-4.8 (m, 2H), 6.74 (s, 2H), 7.25 (m, 2H), 7.50 (m, 5H), 7.74 (d, 2H, J=8.0Hz) |
| 140 | 6 | 1.2-1.4 (m, 2H), 1.8-2.1 (m, 2H), 2.7-3.1 (m, 5H), 3.25 (s, 2H), 3.5-3.7 (m, 6H), 4.6-4.8 (m, 2H), 6.69 (m, 3H), 6.83 (d, 1H, J=8.0Hz), 7.28 (m, 2H), 7.4-7.6 (m, 6H), 7.74 (d, 2H, J=8.0Hz), 8.07 (m, 1H) |
| 141 | 6 | 1.2-1.4 (m, 2H), 1.8-2.1 (m, 2H), 2.5-2.7 (m, 4H), 2.9-3.0 (m, 1H), 3.04 (s, 2H), 3.4-3.9 (m, 6H), 4.6-4.8 (m, 2H), 6.74 (s, 2H), 7.2-7.3 (m, 2H), 7.3-7.6 (m, 10H), 7.74 (d, 2H, J=8.0Hz) |
| 142 | 3 | 1.10-1.42 (m, 4H), 1.65-1.72 (m, 2H), 1.80-1.95 (m, 3H), 2.33 (s, 6H), 2.60-2.70 (m, 4H), 2.80-2.90 (m, 1H), 3.10-3.20 (m, 1H), 3.40-3.50 (m, 1H), 4.45-4.55 (m, 1H), 4.60-4.70 (m, 1H), 6.50 (s, 2H), 7.15-7.25 (brs, 2H), 7.43-7.49 (m, 5H), 7.75 (d, 2H, J=8.3Hz ) |
| 143 | 6 | 1.2-1.4 (m, 2H), 1.7-1.9 (m, 2H), 1.95-2.2 (m, 3H), 2.9-3.0 (m, 1H), 3.1-3.2 (m, 1H), 3.30 (s, 2H), 3.49 (m, 1H), 3.6-3.9 (m, 5H), 3.99 (d, 1H, J=16Hz), 4.6-4.8 (m, 2H), 6.70 (s, 2H), 7.2-7.4 (m, 2H), 7.4-7.6 (m, 5H), 7.74 (d, 2H, J=8.0Hz) |
| 144 | 4 | 1.10-1.45 (m, 2H), 1.70-2.30 (m, 9H), 2.70-2.90 (m, 5H), 3.10-3.20 (m, 1H), 3.60-3.75 (m, 4H), 4.70-4.90 (m, 2H), 7.00-7.15 (m, 2H), 7.40-7.50 (m, 5H), 7.63 (d, 2H, J=7.9Hz) |
| 145 | 3 | 1.10-1.25 (m, 2H), 1.55-1.95 (m, 4H), 2.10-2.20 (m, 2H), 2.29 (s, 3H), 2.60-2.90 (m, 6H), 3.10-3.50 (m, 4H), 4.45-4.75 (m, 2H), 6.61 (s, 2H), 7.22 (brs, 2H), 7.40-7.55 (m, 5H), 7.75 (d, 2H, J=8.0Hz) |
| 146 | 3 | 1.05-1.25 (m, 2H) 1.65-1.78 (m, 1H), 1.80-1.92 (m, 1H), 2.30-2.40 (m, 4H), 2.73-2.95 (m, 1H),2.76 (s, 2H), 3.05-3.75 (m, 6H), 4.42-4.59 (m, 1H), 4.60-4.73 (m, 1H), 6.56-6.60 (m, 1H), 6.61 (s, 2.3H), 6.93 (d, 1H, J=3.4Hz), 7.15-7.32 (m, 2H), 7.38-7.56 (m, 5H) , 7.73-7.85 (m, 3H) |
| 147 | 4 | 1.20-1.45 (m, 2H), 1.50-1.65 (m, 4H), 1.80-2.00 (m, 2H), 2.10-2.25 (m, 2H), 2.50-2.60 (m, 1H), 2.80-3.00 (m, 5H), 3.10-3.20 (m, 1H), 3.60-3.70 (m, 1H), 4.00-4.10 (m, 1H) , 4.75-4.95 (m, 2H), 7.10-7.20 (m, 2H), 7.40-7.50 (m, 5H), 7.60-7.70 (m, 4H), 7.80-7.85 (m, 2H) |
| 150 | 6 | 1.20-1.40 (m, 2H), 1.70-2.05 (m, 2H), 2.80-3.20 (m, 8H), 3.45-3.65 (m, 5H), 3.98-4.02 (m, 1H), 4.20-4.75 (m, 2H), 4.80-4.95 (m, 1H), 6.40-6.50 (m, 1H), 7.05-7.80 (m, 13H) |
| 151 | 3 | 0.45-1.00 (m, 8H), 1.40-1.70 (m, 6H), 1.70-1.80 (m, 1H), 2.40-2.60 (m, 1H), 2.60-3.20 (m, 7H), 4.10-4.20 (m, 1H), 4.25-4.40 (m, 1H), 6.85-7.00 (m, 2H), 7.05-7.25 (m, 5H), 7.35-7.45 (m, 2H) |
| 152 | 3 | 0.87-1.40 (m, 8H), 1.70-2.20 (m, 5H), 2.80-2.90 (m, 1H), 2.95-3.05 (m, 1H), 3.10-3.65 (m, 6H), 4.30-4.40 (m, 1H), 4.45-4.55 (m, 1H), 4.65-4.80 (m, 1H), 7.25-7.40 (m, 2H), 7.45-7.60 (m, 5H), 7.70-7.85 (m, 2H) |
| 153 | 3 | 0.87-0.1.40 (m, 8H), 1.80-2.20 (m, 5H), 2.90-2.70 (m, 8H), 4.25-4.50 (m, 2H), 4.70-4.85 (m, 1H), 7.25-7.75 (m, 10H) |
| 159 | 3 | 1.05-1.25 (m, 2H), 1.60-1.90 (m, 2H), 1.97 (t, 2H, J=7.3Hz ), 2.10-2.49 (m, 13H), 2.75-2.90 (m, 1H), 3.10-3.20 (m, 1H), 3.35-3.50 (m, 1H), 4.42-4.55 (m, 1H), 4.60-4.75 (m, 1H), 6.56 (s, 2H), 7.10-7.30 (m, 2H), 7.40-7.52 (m, 5H), 7.75 (d, 2H, J=8.0Hz ) |
| 160 | 3 | 1.05-1.20 (m, 2H), 1.65-1.90 (m, 2H), 1.98 (t, 2H, J=7.3Hz ), 2.22-2.51 (m, 13H), 2.75-2.90 (m, 1H), 3.10-3.25 (m, 1H), 3.45-3.60 (m, 1H), 4.40-4.55 (m, 1H), 4.65-4.75 (m, 1H), 6.57 (s, 2H), 7.15-7.32 (m, 3H), 7.45-7.50 (m, 3H), 7.58 (s, 1H), 7.66 (d, 1H, J=8.3Hz) |
| 161 | 3 | 1.10-1.48 (m, 5H), 1.68-2.00 (m, 8H), 2.38 (s, 6H), 2.80-2.92 (m, 2H), 2.80-2.95 (m, 1H), 3.10-3.25 (m, 1H), 3.40-3.55 (m, 1H), 4.45-4.60 (m, 1H), 4.65-4.75 (m, 1H), 6.52 (s, 2H), 7.23 (brs, 2H), 7.48-7.51 (m, 5H), 7.76 (d, 2H, J=7.3Hz) |
| 162 | 3 | 1.00-1.20 (m, 2H), 1.65-1.75 (m, 1H), 1.85-1.90 (m, 1H), 1.97 (t, 2H, J=7.3Hz), 2.20-2.50 (m, 5H), 2.80-2.95 (m, 1H), 3.10-3.50 (m, 10H), 4.45-4.55 (m, 1H), 4.65-4.75 (m, 1H), 6.58 (s, 4H), 7.15-7.28(m, 2H), 7.30-7.38 (m, 1H), 7.40-7.55 (m, 3H), 7.60-7.70 (m, 1H) |
| 163 | 3 | 1.10-1.25 (m, 2H), 1.60-2.00 (m, 4H), 2.10-2.51 (m, 13H), 2.80-2.90 (m, 1H), 3.15-3.25 (m, 1H), 3.40-3.50 (m, 1H), 4.45-4.55 (m, 1H), 4.60-4.75 (m, 1H), 6.57 (s, 2H), 7.15-7.30 (m, 2H), 7.40-7.55 (m, 3H), 7.92 (d, 1H, J=8.2Hz), 8.02 (d, 1H, J=8.2Hz), 8.69 (s, 1H) |
| 164 | 3 | 1.05-1.20 (m, 2H), 1.65-1.85 (m, 6H), 2.13 (t, 2H, J=7.3Hz), 2.64 (brs, 4H), 2.80-3.20 (m, 5H), 3.65-3.76 (m, 4H), 4.30-4.75 (m, 2H), 6.51(m, 2H), 6.59 (s, 2H), 6.93 (d, 2H, J=8.5Hz), 7.22-7.24 (brd, 4H, J=7.8Hz), 7.46-7.49 (m, 3H) |
| 165 | 3 | 1.05-1.25 (m, 2H), 1.40-1.55 (m, 2H), 1.68-1.88 (m, 3H), 1.98 (t, 2H, J=7.3Hz), 2.20-2.60 (m, 17H), 2.80-2.90 (m, 1H), 2.95-3.05 (m, 1H), 3.10-3.20 (m, 1H), 3.40-3.50 (m, 1H), 3.45-3.60 (m, 1H), 3.65-3.75 (m, 1H), 6.57 (s, 4H), 7.22 (brs, 2H), 7.48-7.50 (m, 5H), 7.76 (d, 2H, J=8.3Hz) |
| 166 | 6 | 1.2-1.4 (m, 2H), 1.7-2.0 (m, 2H), 2.40 (d, 2H, J=7.0Hz), 2.83 (t, 4H, J=5.0Hz), 3.07 (d, 2H, J=7.0Hz), 3.63 (m, 1H), 4.00 (m, 4H), 4.6-4.8 (m, 2H), 6.71 (s, 2H), 7.2-7.3 (m, 2H), 7.4-7.6 (m, 5H), 7.74 (d, 2H, J=8.0Hz), 8.32 (d, 2H, J=2.4Hz), 8.56 (d, 2H, J=2.4Hz) |
| 167 | 6 | 1.2-1.4 (m, 2H), 1.7-2.0 (m, 2H), 2.3-2.5 (m, 2H), 2.9-3.1 (m, 5H), 3.10 (m, 2H), 3.65 (m, 6H), 4.6-4.8 (m, 2H), 6.69 (m, 3H), 6.73 (m, 1H), 7.2-7.3 (m, 2H), 7.4-7.6 (m, 6H), 7.74 (d, 2H, J=8.0Hz), 8.10 (m, 1H) |
| 168 | 6 | 1.2-1.4 (m, 9H), 1.7-2.0 (m, 2H), 2.20 (d, 2H, J=7.0Hz), 2.5-2.8 (m, 5H), 2.93 (m, 1H), 3.3-3.5 (m, 1H), 3.62 (m, 2H), 4.6-4.8 (m, 2H), 6.71 (s, 2H), 7.2-7.3 (m, 2H), 7.4-7.6 (m, 5H), 7.74 (d, 2H, J=8.0Hz) |
| 169 | 6 | 1.2-1.4 (m, 9H), 1.7-2.0 (m, 2H), 2.24 (d, 2H, J=7.0Hz), 2.7-3.1 (m, 13H), 2.93 (m, 1H), 3.77 (m, 2H), 4.6-4.8 (m, 2H), 6.69 (s, 2H), 7.2-7.3 (m, 2H), 7.4-7.6 (m, 5H), 7.74 (d, 2H, J=8.0Hz) |
| 170 | 3 | 1.10-1.20 (m, 2H), 1.60-2.05 (m, 4H), 2.30-2.40 (m, 2H), 2.60-2.70 (m, 2H), 2.80-2.95 (m, 1H), 3.03-3.45 (m, 6H), 4.40-4.55 (m, 1H), 6.62 (s, 2H), 7.18-7.30 (m, 2H), 7.40-7.55 (m, 5H) , 7.76 (brd, 2H, J=8.0Hz) |
| 171 | 3 | 1.05-1.30 (m, 2H), 1.55-2.05 (m, 6H), 2.26 (s, 6H), 2.35-2.70 (m, 6H), 2.75-3.65 (m, 4H), 4.40-4.55 (m, 1H), 4.60-4.75 (m, 1H), 6.57 (s, 2H), 7.15-7.30 (m, 2H), 7.40-7.50 (m, 5H), 8.3 (brd, 2H, J=7.3Hz) |
| 172 | 6 | 1.20-1.50 (m, 2H), 1.80-2.10 (m, 2H), 2.40-2.55 (m, 2H), 2.80-3.00 (m, 4H), 3.20-3.85 (m, 12H), 4.60-4.90 (m, 2H), 7.20-7.35 (m, 2H), 7.45-7.65 (m, 6H), 7.70-7.80 (m, 1H) |
| 173 | 6 | 1.20-1.50 (m, 2H), 1.70-1.88 (m, 1H), 1.95-2.10 (m, 1Hz), 2.45-2.55 (m, 2H), 2.80-3.00 (m, 4H), 3.10-3.80 (m, 12H), 4.65-4.90 (m, 2H), 7.20-7.40 (m, 3H), 7.45-7.80 (m, 6H) |
| 174 | 6 | 1.10-1.30 (m, 2H), 1.85-1.95 (m, 2H), 2.30-2.45 (m, 2H), 2.49 (s, 3H), 2.70-2.85 (m, 2H), 3.15-3.65 (m, 11H), 4.25-4.50 (m, 1H), 4.70-4.85 (m, 1H), 7.28-7.32 (m, 4H), 7.39-7.50 (m, 4H), 7.61 (brd, 1H, J=8.3Hz), 7.69 (brd, 1H, J=7.8Hz) |
| 175 | 6 | 1.20-1.45 (m, 2H), 1.80-2.05 (m, 2H), 2.40-2.55 (m, 2H), 2.80-3.00 (m, 1H), 3.20-3.70 (m, 12H), 4.60-4.95 (m, 2H), 7.30 (brs, 2H), 7.40-7.60 (m, 5H), 7.73 (d, 2H, J=7.8Hz) |
| 176 | 6 | 1.20-1.40 (m, 2H), 1.80-2.05 (m, 2H), 2.45-2.55 (m, 2H), 2.80-3.00 (m, 4H), 3.20-3.80 (m, 12H), 4.60-4.95 (m, 2H), 7.20-7.40 (m, 4H), 7.45-7.60 (m, 3H), 7.70-7.80 (m, 1H) |
| 177 | 6 | 1.20-1.44 (m, 2H), 1.60-1.75 (m, 2H), 1.90 (m, 1H), 1.92-2.07 (m, 3H), 2.10-2.27 (m, 4H), 2.56-2.82 (m, 10H), 2.85-3.25 (m, 4H), 4.60-4.95 (m, 2H), 6.69 (s, 2.7H), 7.18-7.35 (m, 2H), 7.37-7.46 (m, 2H), 7.46-7.57 (m, 3H), 7.69-7.78 (m, 4H) , 7.80-7.86 (m, 2H) |
| 178 | 5 | 1.18-1.45 (m, 2H), 1.78-2.11 (m, 4H), 2.10-2.30 (m, 4H), 2.70-2.82 (m, 8H), 2.84-3.11 (m, 4H), 3.59-3.78 (m, 1H), 4.53-4.85 (m, 2H), 6.69 (s, 2.7H), 7.15-7.32 (m, 2H), 7.18-7.32 (m, 3H) , 7.43-7.62 (m, 5H) |
| 179 | 6 | 1.10-1.35 (m, 2H), 1.40-1.70 (m, 5H), 1.75-2.05 (m, 6H), 2.40-2.55 (m, 3H), 2.58-2.70 (m, 2H), 2.90-3.00 (m, 4H), 3.10-3.80 (m, 9H), 4.05-4.18 (m, 1H), 4.55-4.65 (m, 1H), 4.75-4.90 (m, 1H), 7.10-7.35 (m, 6H), 7.45-7.58 (m, 3H) |
| 180 | 3 | 0.90-1.10 (m, 2H), 1.20-1.80 (m, 10H), 2.10-2.55 (m, 8H), 2.95-3.60 (m, 11H), 3.80-4.05 (m, 1H), 4.35-4.45 (m, 1H), 4.55-4.70 (m, 1H), 7.20-7.30 (m, 2H), 7.35-7.50 (m, 3H) |
| 181 | 3 | 0.87-1.12 (m, 2H), 1.20-1.90 (m, 17H), 1.98 (t, 2H, J=7.3Hz), 2.10-2.32 (m, 1H), 2.35-2.63 (m, 8H), 2.63-2.70 (m, 2H), 2.72-2.80 (m, 1H), 2.98-3.12 (m, 1H), 3.78-3.90 (m, 1H), 4.34-4.47 (m, 1H), 4.58-4.70 (m, 1H), 6.51 (s, 3H), 7.15-7.25 (m, 2H) , 7.38-7.52 (m, 3H) |
| 195 | 4 | 0.30-0.55 (m, 1H), 1.10-1.55 (m, 7H), 1.71 (s, 6H), 2.46-2.70 (m, 6H), 2.84 (s, 2H), 3.30-3.50 (m, 1H), 4.65-4.80 (m, 2H), 6.80-7.15 (m, 6H), 7.40-7.55 (m, 3H) |
| 196 | 1 | 0.55-1.98 (m, 21H), 2.25-2.50 (m, 6H), 2.70-3.15 (m, 2H), 3.55-3.62 (m, 1H), 4.05-4.80 (m, 3H), 6.65 (s, 3H),7.05-7.55 (m, 10H) |
| 197 | 4 | 0.05-0.18 (m, 0.5H), 0.59-0.62 (m, 3H), 0.77-0.96 (m, 3.5H), 1.15-1.95 (m, 10H), 2.20-2.40 (m, 4H), 2.45-2.73 (m, 3H), 2.85-3.12 (m, 1H), 3.17-3.22 (m, 1H), 3.88-4.03 (m, 1H), 4.63-4.78 (m, 2H), 6.59-7.41 (m, 9H) |
| 221 | 6 | 1.20-1.50 (m, 2H), 1.78-1.90 (m, 1H), 1.91-2.10 (m, 6H), 2.39-2.55 (m, 2H), 2.85-3.00 (m, 1H), 3.25-3.40 (m, 3H), 3.57-3.71 (m, 1H), 3.88-3.98 (m, 3H), 4.63-4.83 (m, 2H), 6.67 (s, 2H), 6.73-6.90 (m, 2H), 7.02-7.10 (m, 1H), 7.37-7.46 (m, 1H), 7.51 (d, 2H, J=8.0Hz) , 7.74 (d, 2H, J=8.0Hz) |
| 222 | 6 | 1.25-1.48 (m, 2H), 1.78-1.90 (m, 1H), 1.95-2.08 (m, 1H), 2.22 (t, 2H, J=7.1Hz), 2.50-2.80 (m, 10H), 2.85-3.15 (m, 6H), 3.57-3.72 (m, 1H), 4.65-4.83 (m, 2H), 6.70 (s, 2.2H), 7.05-7.18 (m, 2H), 7.23-7.31 (m, 1H), 7..48-7.60 (m, 3H), 7.51 (d, 2H, J=8.0Hz) , 7.74 (d, 2H, J=8.0Hz) |
| 223 | 5 | 1.21-1.47 (m, 2H), 1.80-1.94 (m, 1H), 1.95-2.11 (m, 1H), 2.56 (t, 2H, J=6.8Hz), 2.81-3.04 (m, 4H), 3.15-3.30 (m, 1H), 3.38-4.00 (m, 11H), 4.63-4.82 (m, 2H), 7.22-7.35 (m, 1H), 7.42-7.46 (m, 1H), 7.51 (m, 4H) , 7.74 (d, 2H, J=8.1Hz) |
| 224 | 5 | 1.18-1.45 (m, 2H), 1.78-2.11 (m, 4H), 2.19 (t, 2H, J=6.4Hz), 2.78-3.04 (m, 4H), 3.11-3.27 (m, 3H), 3.35-4.08 (m, 9H), 4.60-4.87 (m, 2H), 7.15-7.32 (m, 2H), 7.40-7.60 (m, 5H) , 7.74 (d, 2H, J=8.1Hz) |
| 225 | 4 | 1.15-1.45 (m, 2H), 1.75-1.95 (m, 6H), 2.41 (brs, 4H), 2.79-2.90 (m, 3H), 3.10-3.20 (m, 1H), 3.27-3.33 (m, 8H), 3.60-3.70 (m, 1H), 4.70-4.90 (m, 2H), 7.00-7.15 (m, 2H), 7.40-7.50 (m, 5H), 7.63 (d, 2H, J=8.5Hz) |
| 226 | 3 | 1.10-1.30 (m, 2H), 1.70-1.95 (m, 2H), 2.71 (s, 6H), 2.80-2.90 (m, 1H), 2.95-3.05 (m, 2H), 3.10-3.25 (m, 3H), 3.40-3.55 (m, 3H), 3.73 (brs, 2H), 4.48-4.55 (m, 1H), 4.60-4.70 (m, 1H), 7.20-7.35 (m, 2H), 7.48-7.53 (m, 5H), 7.76 (d, 2H, J=8.3Hz) |
| 227 | 3 | 1.05-1.30 (m, 2H), 1.68-2.00 (m, 6H), 2.40-2.57 (m, 4H), 3.11-3.30 (m, 3H), 3.38-3.55 (m, 1H), 3.60-3.82 (m, 1H), 4.49-4.60 (m, 1H), 4.64-4.75 (m, 1H), 7.50 (d, 2H, J=8.1Hz), 7.53-7.61 (m, 1H), 7.62-7.70 (m, 1H), 7.72-7.87 (m, 3H), 8.06 (d, 1H, J=7.8Hz) |
| 228 | 3 | 1.10-1.30 (m, 2H), 1.70-2.10 (m, 6H), 2.80-3.20 (m, 4H), 3.40-3.50 (m, 4H), 3.61 (brs, 2H), 4.50-4.70 (m, 2H), 7.20-7.35 (m, 2H), 7.45-7.60 (m, 5H), 7.76 (d, 2H, J=8.3Hz) |
| 229 | 3 | 1.15-1.30 (m, 2H), 1.55-1.95 (m, 5H), 2.05-2.20 (m, 1H), 2.75-3.50 (m, 8H), 4.48-4.68 (m, 2H), 5.12 (s, 2H), 6.51 (s, 2H), 7.35-7.60(m, 7H), 7.76 (d, 2H, J=8.3Hz) |
| 230 | 3 | 0.20-0.40 (m, 6H), 0.60-0.80 (m, 2H), 1.15-1.60 (m, 3H), 2.10-2.50 (m, 6H), 2.60-2.70 (m, 1H), 2.90-3.00 (m, 1H), 3.40-3.70 (m, 4H), 3.90-4.05 (m, 1H), 4.15-4.30 (m, 1H), 6.55-6.80 (m, 2H), 6.90-7.10 (m, 5H),7.26 (d, 2H, J=8.3Hz) |

The pharmacological test and the results thereof of the representative compounds of the present invention are explained in the following.

### Experimental Example 1: suppressive action on BN rat DNCB repetitive application dermatitis model

A 8-week-old male rat was sensitized by applying 30 µL of 1.5 wt% solution of dinitrochlorobenzene (DNCB) dissolved in acetone:olive oil=4:1 solution to the right ear on day 1 and 3 days later. Starting from one week thereafter, the same solution was consecutively applied to the right ear every other day in total of 6 times, whereby chronic dermatitis was induced in which the skin thickness increases with time. As for the level of dermatitis, the thickness was measured with a dial gauge thickness before application of the solution, and expressed by the difference from the measured thickness of the right ear immediately before induction on day 1 of the induction. The drug was suspended in 0.5 wt% tragacanth, and orally administered once a day immediately before induction on day 1 of the induction and after 24 hr value measurement the next day. The changes in the ear tumentia with time were monitored, and the area under the curve in a graph showing the values up to 48 hr after the final induction was calculated, from which the percent suppression relative to the area of the solvent control group was determined. As a comparison control compound, prednisolone, which is a steroid, was used.

**Table 13:**

| Example No. | dose (mg/kg) | suppression (%) at 48 hr after final induction |
|---|---|---|
| prednisolone | 2 | 44.0 |
| 1 | 2 | 38.4 |
| 9 | 2 | 38.5 |
| 30 | 2 | 44.2 |
| 33 | 2 | 41.7 |
| 65 | 2 | 20.5 |
| 103 | 2 | 39.6 |
| 155 | 2 | 23.3 |
| 183 | 2 | 37.6 |
| 193 | 2 | 28.8 |
| 198 | 2 | 25.5 |
| | 5 | 45.8 |
| 212 | 2 | 29.2 |
| 217 | 2 | 20.9 |

### Experimental Example 2: suppressive action on TMA (Trimellitic anhydride) repetitive application dermatitis model

Male BALB/cAnNCrlCrlj mice were used. On the previous day, the abdomen of the mice was shaved using Schick Injector (single blade; Schick Japan), and the mice were sensitized by the application of a 10 wt% TMA (acetone:olive oil=4:1) solution (100 µL) to the abdomen. Five days later, a 10 wt% TMA solution (100 µL) was applied to the abdomen again for booster. Starting from 5 days thereafter, the 10 wt% TMA solution (25 µL) was applied to the right ear for 3 consecutive days. At 8 or 9 days from the day of final application, the 10 wt% TMA solution (25 µL) was applied to both ears to allow for induction. The early thickness at 8 hr after the induction was measured using a dial gauge thickness. The level of ear reaction was expressed by the difference between the measured value of ear thickness at 8 hr and that before induction, and the percent suppression was calculated based on the comparison with that of the solvent control group. The test drug was dissolved in DMSO (dimethyl sulfoxide) to the final concentration of 2.5 wt% on the previous day, and prepared with 0.5 wt% methylcellulose (MC) solution. Prednisolone (steroid) used as a comparison control compound was prepared at 1 mg/mL. Each was orally administered twice at 0.5 mL/mouse 30 min before and 4 hr after the induction.

**Table 14:**

| Example No. | dose (mg/kg) | right ear suppression (%) at 8 hr after final induction |
|---|---|---|
| prednisolone | 20 | 67.8 |
| 8 | 20 | 55.7 |
| 9 | 20 | 72.5 |
| 12 | 20 | 58.3 |
| 26 | 20 | 49.1 |
| 29 | 20 | 50.7 |
| 34 | 20 | 60.6 |
| 65 | 20 | 41.0 |
| 86 | 20 | 63.9 |
| 103 | 20 | 52.1 |
| 131 | 80 | 58.1 |
| 133 | 80 | 59.1 |
| 148 | 20 | 41.9 |
| 155 | 20 | 52.4 |
| 158 | 20 | 40.6 |
| 183 | 20 | 48.1 |
| 198 | 20 | 48.7 |
| 78 | 80 | 52.3 |
| 100 | 80 | 66.9 |
| 135 | 80 | 56.0 |
| 138 | 80 | 61.5 |
| 159 | 80 | 62.0 |
| 160 | 80 | 62.0 |
| 221 | 80 | 64.9 |
| 152 | 80 | 76.2 |
| 227 | 80 | 57.2 |
| 177 | 80 | 70.0 |

As is clear from Tables 13 and 14, the compound represented by the above-mentioned formula (I) of the present invention was confirmed to have a suppressive action equal to or greater than that of steroids on skin inflammatory response in atopic dermatitis animal models. Therefore, the compound of the present invention is suggested to have a superior antiallergic action, and further, an anti-inflammatory action.

### Industrial Applicability

As is clear from the above-mentioned Experimental Examples, since the compound of the present invention has a superior antiallergic action, it can be used as an antiallergic agent for the prophylaxis and/or treatment of, for example, allergic diseases such as allergic dermatitis, allergic rhinitis, bronchial asthma, atopic dermatitis, contact dermatitis, urticaria, eczema, allergic ophthalmia and pollinosis, and further, other inflammatory diseases, desirably alone, or in combination with an existing pharmaceutical agent as necessary.
This application is based on a patent application No. 2006-244302 filed in Japan (filing date: September 8, 2006), the contents of which are incorporated in full herein by this reference.

## Claims

1. A compound represented by the following formula (I): 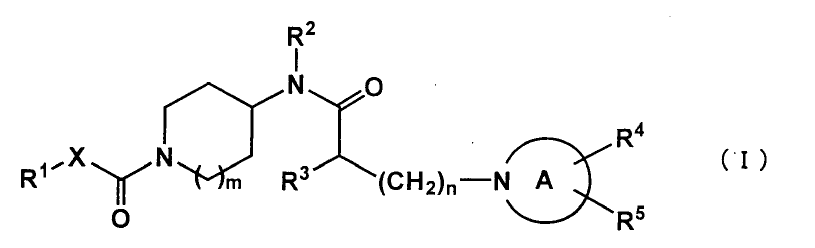 wherein R¹ and R² are the same or different and each is an optionally substituted aryl group, an optionally substituted C₃₋₈ cycloalkyl group, an optionally substituted heterocyclic group or an optionally substituted partially hydrogenated aryl group,
R³ is a hydrogen atom, an optionally substituted C₁₋₆ alkyl group, an optionally substituted aryl group or an optionally substituted benzyl group,
R⁴ and R⁵ are the same or different and each is a hydrogen atom, a halogen atom, a hydroxy group, a carboxyl group, an optionally substituted C₁₋₆ alkyl group, an optionally substituted C₃₋₈ cycloalkyl group, an optionally substituted C₁₋₆ alkoxy group, a C₁₋₆ alkoxycarbonyl group, an optionally substituted amino group, a substituted carbamoyl group, an optionally substituted aryl group, an optionally substituted benzyl group, an optionally substituted benzoyl group, an optionally substituted heterocyclic group or an optionally substituted heterocarbonyl group, or R⁴ and R⁵ in combination may form an oxo group,
X is a single bond or -C(R⁶)(R⁷)-,
wherein R⁶ and R⁷ are the same or different and each is a hydrogen atom, a halogen atom, a hydroxy group, an optionally substituted C₁₋₆ alkyl group or an optionally substituted aryl group, or R⁶ and R⁷ may form, together with the carbon atom bonded thereto, an optionally substituted C₃₋₈ cycloalkane ring,
a substituted piperidine ring or a tetrahydro-2H-pyran ring, ring group A is an azetidin-1-yl group, a pyrrolidin-1-yl group, a piperidino group, a piperazin-1-yl group, a morpholino group, a hexahydroazepin-1-yl group, a hexahydrodiazepin-1-yl group, an aziridin-1-yl group, a perhydroisoquinolin-2-yl group, a perhydroquinolin-1-yl group, a 1,2,3,4-tetrahydroisoquinolin-2-yl group, a 1,2,3,4-tetrahydroquinolin-1-yl group, a 2,3-dihydroindol-1-yl group, a perhydroindol-1-yl group, a 1,3-dihydroisoindol-2-yl group, a perhydroisoindol-2-yl group, a pyrazolidin-1-yl group, a tetrahydroimidazol-1-yl group, a hexahydro-1,4-oxaazepin-4-yl group, an oxazolidin-3-yl group, a thiazolidin-3-yl group, a thiomorpholin-4-yl group, a hexahydropyridazin-1-yl group, a hexahydropyrimidin-1-yl group, a tetrazolyl group, a 2-pyrrolin-1-yl group or a 3-pyrrolin-1-yl group,
m is 0, 1 or 2, and
n is 0, 1 or 2
or a physiologically acceptable salt thereof.

2. The compound of claim 1, wherein the ring group A is an azetidin-1-yl group, a pyrrolidin-1-yl group, a piperidino group, a morpholino group, a piperazin-1-yl group or a perhydroazepin-1-yl group, or a physiologically acceptable salt thereof.

3. The compound of claim 1 or 2, wherein R⁴ and R⁵ are the same or different and each is a hydrogen atom, a halogen atom, a hydroxy group, a C₁₋₆ alkyl group, a C₁₋₆ alkoxy group, a C₁₋₆ dialkylamino group, a C₁₋₆ dialkylcarbamoyl group, a pyrrolidinylcarbonyl group or a C₁₋₆ alkyloxadiazolyl group, or a physiologically acceptable salt thereof.

4. The compound of any one of claims 1 to 3, wherein m is 1, or a physiologically acceptable salt.

5. The compound of any one of claims 1 to 4, wherein R³ is a hydrogen atom or a C₁₋₆ alkyl group, and n is 0 or 1, or a physiologically acceptable salt thereof.

6. The compound of any one of claims 1 to 4, wherein R³ is a hydrogen atom, and n is 0 or 1, or a physiologically acceptable salt thereof.

7. The compound of any one of claims 1 to 6, wherein R¹ and R² are the same or different and each is an aryl group optionally substituted at substitutable position(s) by 1 to 4 atoms or groups selected from the group consisting of a halogen atom, a C₁₋₆ alkyl group, a C₁₋₆ alkoxy group, a trifluoromethyl group, a trifluoromethoxy group, a cyano group, a nitro group, a phenyl group, a carboxyl group, a C₃₋₈ cycloalkyl group, a C₁₋₃ alkoxy-C₁₋₃ alkyl group, a C₁₋₆ alkoxycarbonyl group, a C₁₋₆ alkylcarbonyl group, a trifluoromethylcarbonyl group, a C₁₋₃ alkylthio group, a C₁₋₃ alkylsulfonyl group, a hydroxy group and an amino group (the amino group is optionally substituted by one or two, the same or different, groups selected from the group consisting of a C₁₋₃ alkyl group, a C₃₋₈ cycloalkyl group and a tetrahydro-2H-pyranyl group); a C₃₋₈ cycloalkyl group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group; a hetero ring group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group; or a partially hydrogenated aryl group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group, or a physiologically acceptable salt thereof.

8. The compound of any one of claims 1 to 6, wherein R¹ and R² are the same or different and each is a phenyl group optionally substituted at substitutable position(s) by 1 to 4 atoms or groups selected from the group consisting of a halogen atom, a C₁₋₆ alkyl group, a C₁₋₆ alkoxy group, a trifluoromethyl group, a trifluoromethoxy group, a cyano group, a nitro group, a carboxyl group, a hydroxy group and an amino group (the amino group is optionally substituted by one or the same or different two C₁₋₃ alkyl groups); a cyclohexyl group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group; a pyridyl group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group; or a benzofuranyl group optionally substituted at substitutable position(s) by 1 to 4 atoms or groups selected from the group consisting of a halogen atom, a hydroxy group, a C₁₋₆ alkyl group, a C₁₋₆ alkoxy group, a trifluoromethyl group and a trifluoromethoxy group, or a physiologically acceptable salt thereof.

9. The compound of any one of claims 1 to 8, wherein X is a single bond or -C(R⁶)(R⁷)- wherein R⁶ and R⁷ are the same substituent and is a C₁₋₆ alkyl group, or R⁶ and R⁷ form, together with the carbon atom bonded thereto, a C₃₋₈ cycloalkane ring; a piperidine ring substituted by a C₁₋₆ alkyl group, a phenyl group (the phenyl group is optionally substituted by 1 to 4 atoms or groups selected from the group consisting of a halogen atom, a C₁₋₆ alkyl group, a C₁₋₆ alkoxy group, a trifluoromethyl group and a hydroxy group), a benzyl group (the benzyl group is optionally substituted by 1 to 4 atoms or groups selected from the group consisting of a halogen atom, a C₁₋₆ alkyl group, a C₁₋₆ alkoxy group, a trifluoromethyl group and a hydroxy group) or a C₁₋₃ alkylcarbonyl group; or a tetrahydro-2H-pyran ring, or a physiologically acceptable salt thereof.

10. The compound of any one of claims 1 to 8, wherein X is a single bond, or a physiologically acceptable salt thereof.

11. The compound of claim 1, which is selected from the group consisting of
N-[1-(4-chloro-2,5-difluorobenzoyl)piperidin-4-yl]-N-phenyl-2-(piperidin-1-yl)acetamide
N-phenyl-2-(piperidin-1-yl)-N-[1-(4-trifluoromethylbenzoyl)piperidin-4-yl]acetamide,
N-[1-(3-fluoro-4-trifluoromethylbenzoyl)piperidin-4-yl]-N-phenyl-2-(piperidin-1-yl)acetamide,
N-phenyl-2-(piperidin-1-yl)-N-[1-(4-trifluoromethoxybenzoyl)piperidin-4-yl]acetamide,
2-(morpholin-4-yl)-N-phenyl-N-[1-(4-trifluoromethylbenzoyl)piperidin-4-yl]acetamide,
N-[1-(3-fluoro-4-trifluoromethylbenzoyl)piperidin-4-yl]-2-(morpholin-4-yl)-N-phenylacetamide,
N-[1-(2,3-difluoro-4-trifluoromethylbenzoyl)piperidin-4-yl]-2-(morpholin-4-yl)-N-phenylacetamide,
N-(4-methoxyphenyl)-2-(piperidin-1-yl)-N-[1-(4-trifluoromethylbenzoyl)piperidin-4-yl]acetamide,
N-[1-(4-chlorobenzoyl)piperidin-4-yl]-N-cyclohexyl-2-(morpholin-4-yl)acetamide,
N-[1-(trans-4-trifluoromethylcyclohexylcarbonyl)piperidin-4-yl]-2-(morpholin-4-yl)-N-phenylacetamide,
N-phenyl-2-(pyrrolidin-1-yl)-N-[1-(4-trifluoromethylbenzoyl)piperidin-4-yl]acetamide,
N-[1-(3,4-dichlorobenzoyl)piperidin-4-yl]-N-phenyl-2-(pyrrolidin-1-yl)acetamide,
3-(morpholin-4-yl)-N-phenyl-N-[1-(4-trifluoromethylbenzoyl)piperidin-4-yl]propionamide,
N-[1-(4-chlorobenzoyl)piperidin-4-yl]-N-phenyl-3-(pyrrolidin-1-yl)propionamide,
N-[1-[2-(4-chlorophenyl)-2-methylpropionyl]piperidin-4-yl]-N-phenyl-2-(piperidin-1-yl)acetamide,
N-[1-[1-(4-chlorophenyl)cyclopentane-1-carbonyl]piperidin-4-yl]-N-phenyl-2-(piperidin-1-yl)acetamide,
N-phenyl-2-(piperidin-1-yl)-N-[1-[1-(3-trifluoromethylphenyl)cyclopentane-1-carbonyl]piperidin-4-yl]acetamide,
N-[1-[1-(2-fluorophenyl)cyclopentane-1-carbonyl]piperidin-4-yl]-N-phenyl-2-(piperidin-1-yl)acetamide,
2-(3-dimethylaminopyrrolidin-1-yl)-N-phenyl-N-[1-(4-trifluoromethylbenzoyl)piperidin-4-yl]acetamide,
N-[1-(2,4-ditrifluoromethylbenzoyl)piperidin-4-yl]-2-(morpholin-4-yl)-N-phenylacetamide,
N-cyclohexyl-2-(pyrrolidin-1-yl)-N-[1-(4-trifluoromethylbenzoyl)piperidin-4-yl]acetamide,
2-(3-hydroxypyrrolidin-1-yl)-N-phenyl-N-[1-(4-trifluoromethylbenzoyl)piperidin-4-yl]acetamide,
2-[4-(5-methyloxodiazol-3-yl)]piperidin-1-yl]-N-phenyl-N-[1-(4-trifluoromethylbenzoyl)piperidin-4-yl]acetamide,
3-(4-methylpiperazin-1-yl)-N-phenyl-N-[1-(4-trifluoromethylbenzoyl)piperidin-4-yl]propionamide,
3₋(4-dimethylaminopiperidine-1-yl)-N-phenyl-N-[1-(4-trifluoromethylbenzoyl)piperidin-4-yl]propionamide,
3-(4-methylpiperazin-1-yl)-N-phenyl-N-[1-[(benzofuran-2-yl)carbonyl]piperidin-4-yl]propionamide,
N-(3-carboxylphenyl)-2-(pyrrolidin-1-yl)-N-[1-(4-trifluoromethylbenzoyl)piperidin-4-yl]acetamide, and
N-[1-(3-fluoro-4-trifluoromethylbenzoyl)piperidin-4-yl]-3-(4-methylpiperazin-1-yl)-N-phenylpropionamide, or a physiologically acceptable salt thereof.

12. A pharmaceutical composition comprising the compound of any one of claims 1 to 11 or a physiologically acceptable salt thereof as an active ingredient.

13. The pharmaceutical composition of claim 12, which is used for the prophylaxis and/or treatment of an allergic disease.

14. The pharmaceutical composition of claim 13, wherein the allergic disease is atopic dermatitis or contact dermatitis.

15. Use of the compound of any one of claims 1 to 11 or a physiologically acceptable salt thereof for the production of a pharmaceutical composition to be used for the prophylaxis and/or treatment of an allergic disease.

16. The use of claim 15, wherein the allergic disease is atopic dermatitis or contact dermatitis.

17. A method of preventing and/or treating an allergic disease in a mammal, comprising administering an effective amount of the compound of any one of claims 1 to 11 or a physiologically acceptable salt thereof to the mammal.

18. The method of claim 17, wherein the allergic disease is atopic dermatitis or contact dermatitis.
